# EUROPEAN PATENT APPLICATION

(11) **EP 2 100 941 A2**
(43) Date of publication of application: **16.09.2009**
(21) Application number: 08173052.5
(22) Date of filing: 30.12.2008
(51) Int. Cl.: C09K 11/06, H05B 33/14

(54) **Novel organic electroluminescent compounds and organic electroluminescent device using the same**

(30) Priority: 14.03.2008 KR 20080023831; 29.10.2008 KR 20080106223
(71) Applicant: Gracel Display Inc., Seoul 133-833 (KR)
(72) Inventor: Lee, Soo Young, 472-120, Gyeonggi-do (KR); Shin, Hyo Nim, 136-060, Seoul (KR); Cho, Young Jun, 136-060, Seoul (KR); Kwon, Hyuck Joo, 130-100, Seoul (KR); Kim, Bong Ok, 135-090, Seoul (KR); Kim, Sung Min, 157-886, Seoul (KR); Yoon, Seung Soo, 135-884, Seoul (KR)
(74) Representative: Kent, Venetia Katherine

(57) **Abstract**

The present invention relates to novel organic electroluminescent compounds, and organic light-emitting diodes and organic solar cells comprising the same. More specifically, the organic electroluminescent compounds according to the present invention are represented by Chemical Formula (1) 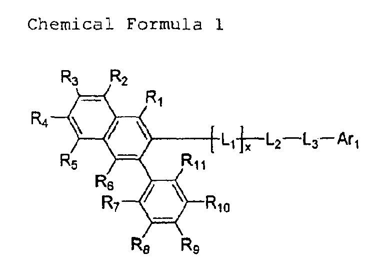 The organic electroluminescent compounds according to the invention exhibit high luminous efficiency and excellent color purity and life property as a material, so that an OLED having very good operation life can be prepared therefrom.

## Description

### FIELD OF THE INVENTION

The present invention relates to novel organic electroluminescent compounds and organic electroluminescent devices comprising the same.

### BACKGROUND OF THE INVENTION

Among display devices, electroluminescence devices (EL devices) are self-luminescent display devices showing the advantage of wide angle of view, excellent contrast and rapid response rate, as compared to LCD's. Eastman Kodak developed in 1987 an organic EL device which employs a low molecular weight aromatic diamine and an aluminum complex as material for forming an EL layer, for the first time [Appl. Phys. Lett. 51, 913, 1987].

An organic EL device is a device wherein, when charge is applied to an organic film formed between an electron injection electrode (cathode) and a hole injection electrode (anode), an electron and a hole form a pair and then diminishes with emitting light. A device can be formed on a transparent flexible substrate such as plastics. The device can be operated at a lower voltage (not more than 10 V) with relatively lower power consumption but excellent color purity, as compared to a plasma display panel or an inorganic EL display.

The most important factor to determine luminous efficiency, lifetime or the like in an organic EL device is electroluminescent material. Several properties required for such electroluminescent materials include that the material should have high fluorescent quantum yield in solid state and high mobility of electrons and holes, is not easily decomposed during vapor-deposition in vacuo, and forms uniform and stable thin film.

Organic electroluminescent materials can be generally classified into high-molecular materials and low-molecular materials. The low-molecular materials include metal complexes and thoroughly organic electroluminescent materials which do not contain metal, from the aspect of molecular structure. Such electroluminescent materials include chelate complexes such as tris(8-quinolinolato)aluminum complexes, coumarin derivatives, tetraphenylbutadiene derivatives, bis(styrylarylene) derivatives, oxadiazole derivatives. From those materials, it is reported that light emission of visible region from blue to red can be obtained.

In order to realize a full-colored OLED display, three EL materials (red, green and blue) are employed, and development of EL materials having high efficiency and long life is a significant subject to enhance the features of the overall organic electroluminescence. EL materials can be functionally classified into host materials and dopant materials. It is generally known that a device structure having the most excellent EL properties can be fabricated with an EL layer prepared by doping a dopant to a host. Recently, development of organic EL devices with high efficiency and long life comes to the fore as an urgent subject, and particularly urgent is development of a material with far better EL properties as compared to conventional EL materials as considering EL properties required for medium to large sized OLED panels.

From this aspect, development of host material is one of the most important factors to be addressed. The desired properties for the host material (serving as a solid state solvent and an energy deliverer) are high purity and appropriate molecular weight to enable vapor-deposition in vacuo. In addition, glass transition temperature and thermal decomposition temperature should be high to ensure thermal stability. Further, the host material should have high electrochemical stability for providing long life. It is to be easy to form an amorphous thin film, with high adhesiveness to other adjacent materials but without interlayer migration.

In the meanwhile, as to conventional blue materials, a number of materials have been developed and commercialized since the development of diphenylvinyl-biphenyl (DPVBi) (Chemical Formula a) by Idemitsu-Kosan. In addition to the blue material system from Idemitsu-Kosan, dinaphthylanthracene (DNA, Chemical Formula b), tetra(t-butyl)perylene (Chemical Formula c) system or the like have been known. However, extensive research and development should be performed with respect to these materials. The distryl compound system of Idemitsu-Kosan, which is known to have highest efficiency up to now, has 6 lm/W of power efficiency and beneficial device lifetime of more than 30,000 hr. However, when it is applied to a full-colored display, the lifetime is merely several thousand hours, owing to the reduction of color purity over operation time. In case of blue electroluminescentce, it becomes advantageous from the aspect of the luminous efficiency, if the electroluminescent wavelength is shifted a little toward longer wavelength. However, it is not easy to apply the material to a display of high quality because of unsatisfactory color purity in blue. In addition, the research and development of such materials are urgently demanded because of the problems in color purity, efficiency and thermal stability.

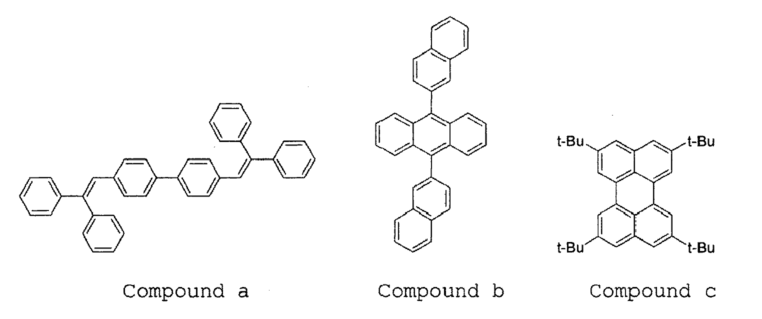

For host materials having high efficiency and long life, various substances with different backbones, such as dispiro-fluorene-anthracene (TBSA), ter-spirofluorene (TSF) and bitriphenylene (BTP), have been developed, but they are not satisfactory in terms of color purity and luminous efficiency.

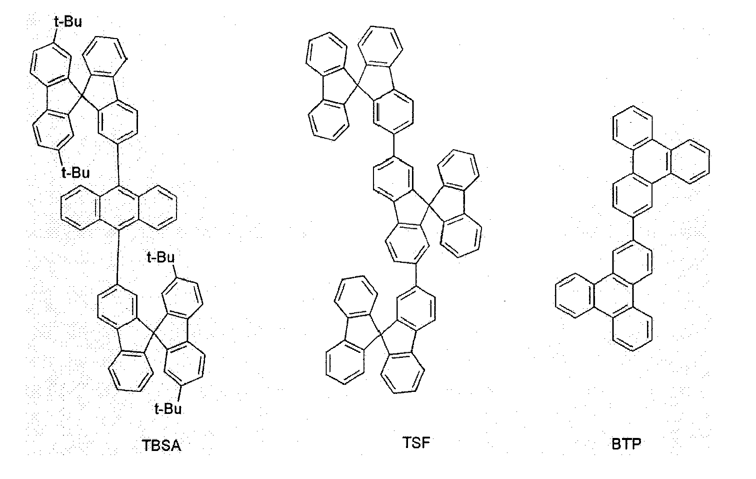

The compound TBSA as reported by Gyeongsang National University and Samsung SDI (Kwon, S. K. et al., Advanced Materials, 2001, 13, 1690; Japanese Patent Laid-Open JP 2002121547), showed luminous efficiency of 3 cd/A at 7.7 V, and relatively good color coordinate of (0.15, 0.11), but it was an example applied as material for a single layer, being inappropriate for practical use.

The compound TSF reported by Taiwan National University (Wu, C. -C. et al., Advanced Materials, 2004, 16, 61; US Patent Publication US 2005040392) showed relatively good external quantum efficiency of 5.3%, but it is still insufficient for practical use.

The compound BTP reported by Chingwha National University of Taiwan (Cheng, C. -H. et al., Advanced Materials, 2002, 14, 1409; US Patent Publication 2004076852) showed luminous efficiency of 2.76 cd/A and relatively good color coordinate of (0.16, 0.14), but this was still insufficient for practical use.

As described above, conventional materials are constituted by a single layer, not forming host-dopant thin layer, and is difficult to be used practically from the aspect of color purity and efficiency. It lacks reliable data with respect to its long life.

In the meanwhile, according to a patent application of Mitsui Chemicals (Japan) (US Patent Publication 7,166,240), the compounds shown below have the absorption spectrum at 390 to 430 nm, with luminous efficiency of 4.6 cd/A. However, on the basis of these data, the compounds with above absorption wavelength range, electroluminescence of greenish blue color is anticipated, and the Patent Publication indicates the color as bluish green color.

Particularly, embodiment of pure blue color is impossible with the symmetrical structure of the Patent Publication, and the material, which cannot provide pure blue luminescence, is inadequate to be practically applied to a full-colored display.

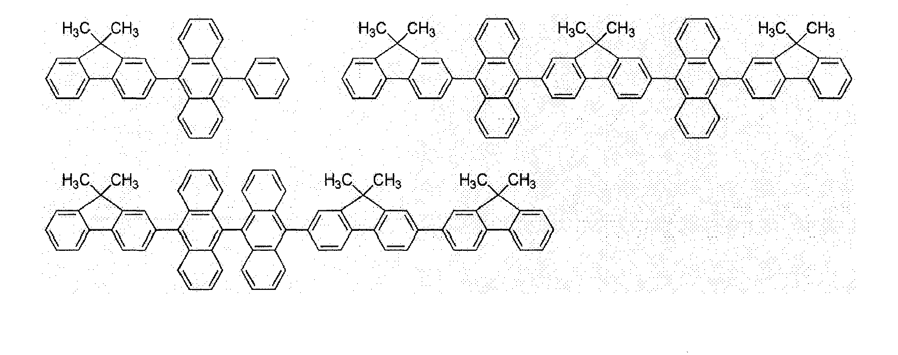

### SUMMARY OF THE INVENTION

Thus, the first object of the invention is to overcome the problems described above, and to provide an organic electroluminescent compound comprising an excellent backbone to obtain better luminous efficiency, device life and appropriate color coordinate, as compared to conventional host material.

The second object of the invention is to provide an organic electroluminescent device of high efficiency and long life by employing the organic electroluminescent compound as EL material.

The third object is to provide an organic EL device employing the organic EL compound in the electroluminescent layer.

The fourth object is to provide a solar cell comprising the organic electroluminescent compound.

Thus, present invention relates to organic electroluminescent compounds represented by Chemical Formula (1), and organic electroluminescent devices comprising the same. The organic electroluminescent compounds according to the invention exhibit high luminous efficiency, and excellent color purity and life property of the material, so that OLED's with very excellent operation life can be manufactured therefrom. 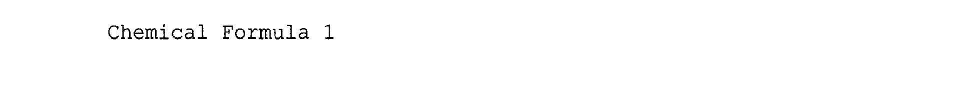 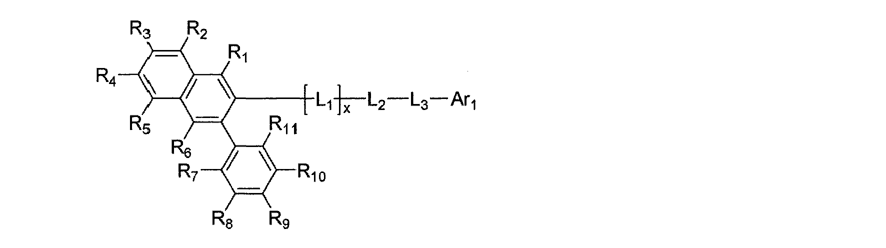 wherein, L₁ represents (C6-C60)arylene or (C3-C60)heteroarylene containing one or more heteroatom(s) selected from N, O and S, or a bivalent group selected from the following structures:

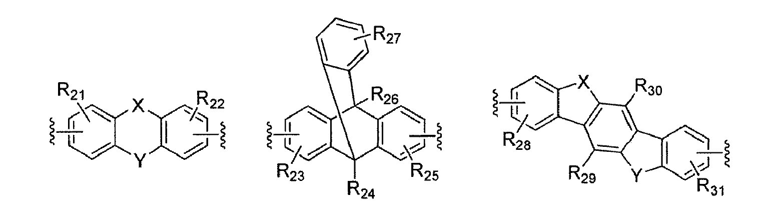

L₂ and L₃ independently represent a chemical bond, or (C1-C60)alkyleneoxy, (C1-C60)alkylenethio, (C6-C60)aryleneoxy, (C6-C60)arylenethio, (C6-C60)arylene or (C3-C60)heteroarylene containing one or more heteroatom(s) selected from N, O and S; Ar₁ represents NR₄₁R₄₂, (C6-C60)aryl, (C3-C60)heteroaryl containing one or more heteroatom(s) selected from N, O and S, a 5-or 6-membered heterocycloalkyl containing one or more heteroatom(s) selected from N, O and S, (C3-C60)cycloalkyl, adamantyl, (C7-C60)bicycloalkyl, or a substituent selected from the following structures:

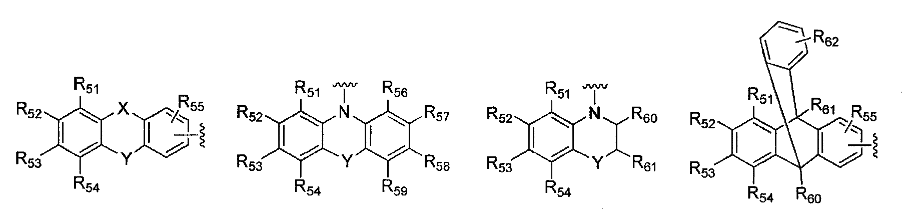

R₁ through R₁₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁ through R₁₁ may be linked to an adjacent substituent via (C3-C60)alkylene or (C3-C60)alkenylene with or without a fused ring to form an alicyclic ring, or a monocyclic or polycyclic aromatic ring;

R₂₁ through R₃₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₂₁ through R₃₁ may be linked to an adjacent substituent via (C3-C60)alkylene or (C3-C60)alkenylene with or without a fused ring to form an alicyclic ring, or a monocyclic or polycyclic aromatic ring;

R₄₁ and R₄₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₄₁ and R₄₂ may be linked via (C3-C60)alkylene or (C3-C60)alkenylene with or without a fused ring to form an alicyclic ring, or a monocyclic or polycyclic aromatic ring;

R₅₁ through R₆₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₅₁ through R₆₂ may be linked to an adjacent substituent via (C3-C60)alkylene or (C3-C60)alkenylene with or without a fused ring to form an alicyclic ring, or a monocyclic or polycyclic aromatic ring;

X and Y independently represent a chemical bond, or -(CR₇₁R₇₂)m-, -N(R₇₃)-, -S-, -O-, -Si(R₇₄) (R₇₅)-, -P(R₇₆)-, -C(=O)-, -B(R₇₇)-, -In(R₇₈)-, -Se-, -Ge(R₇₉) (R₈₀)-, -Sn(R₈₁) (R₈₂)-, -Ga(R₈₃)- or -(R₈₄)C=C(R₈₅)-;

R₇₁ through R₈₅ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₇₁ and R₇₂, R₇₄ and R₇₅, R₇₉ and R₈₀, R₈₁ and R₈₂, or R₈₄ and R₈₅ may be linked via (C3-C60)alkylene or (C3-C60)alkenylene with or without a fused ring to form an alicyclic ring, or a monocyclic or polycyclic aromatic ring; the arylene or heteroarylene of L₁ through L₃, the aryl or heteroaryl of Ar₁, or the alkyl, aryl, heteroaryl, heterocycloalkyl, cycloalkyl, trialkylsilyl, dialkylarylsilyl, triarylsilyl, alkenyl, alkynyl, alkylamino or arylamino of R₁ through R₁₁, R₂₁ through R₃₁, R₄₁, R₄₂, R₅₁ through R₆₂, and R₇₁ through R₈₅ may be further substituted by one or more substituent(s) selected from deuterium, halogen, (C1-C60)alkyl, halo(C1-C60)alkyl, (C6-C60)aryl, (C3-C60)heteroaryl containing one or more heteroatom(s) selected from N, O and S, with or without (C6-C60)aryl substituent(s), morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino,(C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 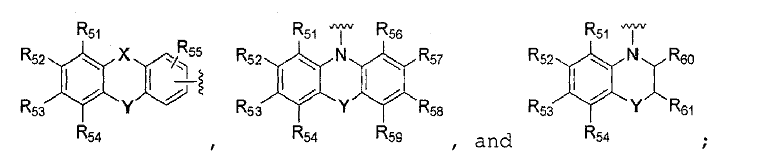 m is an integer from 1 to 4; and x is an integer from 1 to 4.

### BRIEF DESCRIPTION OF THE DRAWINGS

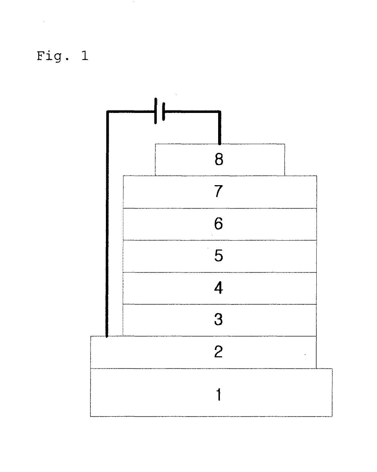

Fig. 1 is a cross-sectional view of an OLED.

### DETAILED DESCRIPTION OF THE INVENTION

Referring now to the Drawings, Fig. 1 illustrates a cross-sectional view of an OLED of the present invention comprising a Glass 1, a Transparent electrode 2, a Hole injection layer 3, a Hole transport layer 4, an Electroluminescent layer 5, an Electron transport layer 6, an Electron injection layer 7 and an Al cathode 8.

The term "alkyl" described herein and any substituents comprising "alkyl" moiety include both linear and branched species.

The term "aryl" described herein means an organic radical derived from aromatic hydrocarbon via elimination of one hydrogen atom. Each ring comprises a monocyclic or fused ring system containing from 4 to 7, preferably from 5 to 6 cyclic atoms. Specific examples include phenyl, naphthyl, biphenyl, anthryl, tetrahydronaphthyl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S as the aromatic cyclic backbone atom(s), and carbon atom(s) for remaining aromatic cyclic backbone atoms. The heteroaryl may be a 5- or 6-membered monocyclic heteroaryl or a polycyclic heteroaryl which is fused with one or more benzene ring(s), and may be partially saturated. The heteroaryl group may comprise a bivalent aryl group, of which the heteroatoms may be oxidized or quaternized to form N-oxide and quaternary salt. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The naphthyl of Chemical Formula (1) may be 1-naphthyl or 2-naphthyl; the anthryl may be 1-anthryl, 2-anthryl or 9-anthryl; and the fluorenyl may be 1-fluorenyl, 2-fluorenyl, 3-fluorenyl, 4-fluorenyl or 9-fluorenyl.

The substituents comprising "(C1-C60)alkyl" moiety described herein may contain 1 to 60 carbon atoms, 1 to 20 carbon atoms, or 1 to 10 carbon atoms. The substituents comprising "(C6-C60)aryl" moiety may contain 6 to 60 carbon atoms, 6 to 20 carbon atoms, or 6 to 12 carbon atoms. The substituents comprising "(C3-C60)heteroaryl" moiety may contain 3 to 60 carbon atoms, 4 to 20 carbon atoms, or 4 to 12 carbon atoms. The substituents comprising "(C3-C60)cycloalkyl" moiety may contain 3 to 60 carbon atoms, 3 to 20 carbon atoms, or 3 to 7 carbon atoms. The substituents comprising "(C2-C60)alkenyl or alkynyl" moiety may contain 2 to 60 carbon atoms, 2 to 20 carbon atoms, or 2 to 10 carbon atoms.

L₁ may be selected from the following structures, but is not restricted thereto. 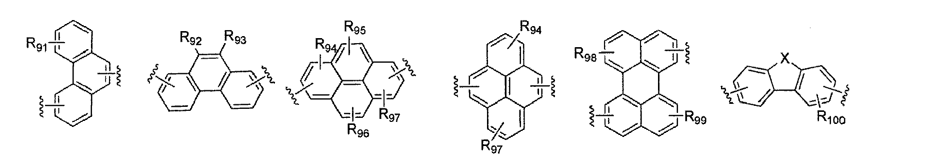 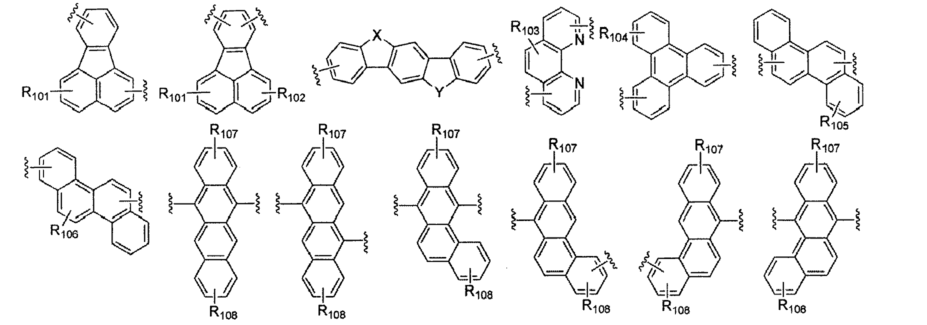 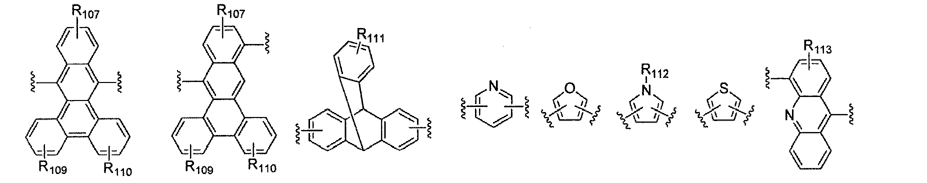 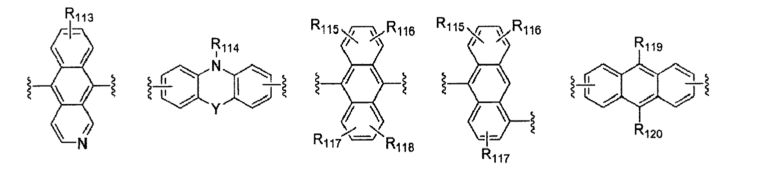 wherein, X and Y independently represent -(CR₇₁R₇₂)ₘ-, -N(R₇₃)-, -S-, -O-, -Si(R₇₄)(R₇₅)-, -P(R₇₆)- or -(R₈₄)C=C(R₈₅)-; R₇₁ through R₇₆, R₈₄, R₈₅ and m are defined as in Chemical Formula 1; R₉₁ through R₁₂₀ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₉₁ through R₁₂₀ may be linked via (C3-C60)alkylene or (C3-C60)alkenylene with or without a fused ring to form an alicyclic ring, or a monocyclic or polycyclic aromatic ring.

More specifically, L₁ is selected from the following structures, but not restricted thereto. 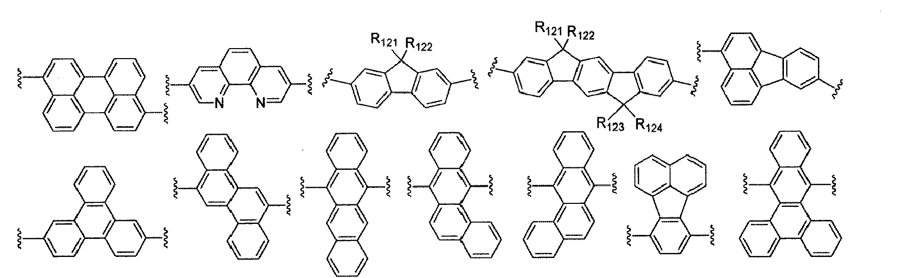 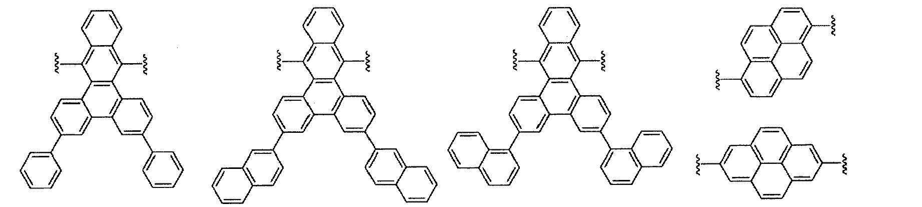 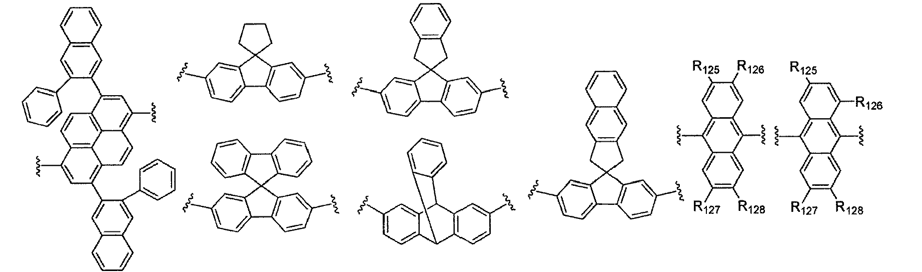 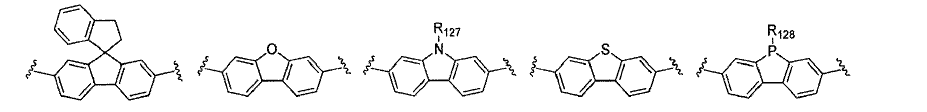 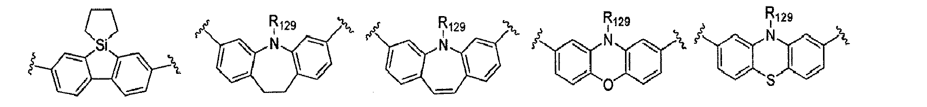 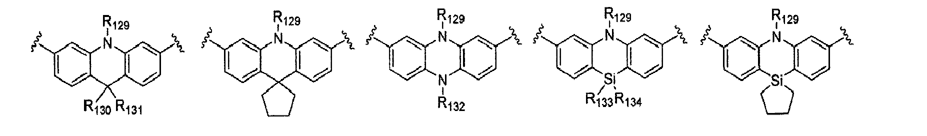 wherein, R₁₂₁ through R₁₃₄ independently represent hydrogen, deuterium, (C1-C60)alkyl or (C6-C60)aryl; the alkyl or aryl of R₁₂₁ through R₁₃₄ may be further substituted by one or more substituent(s) selected from deuterium, halogen, (C1-C60)alkyl, halo(C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl. Group 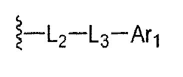 may be selected from the following structures, but is not restricted thereto. 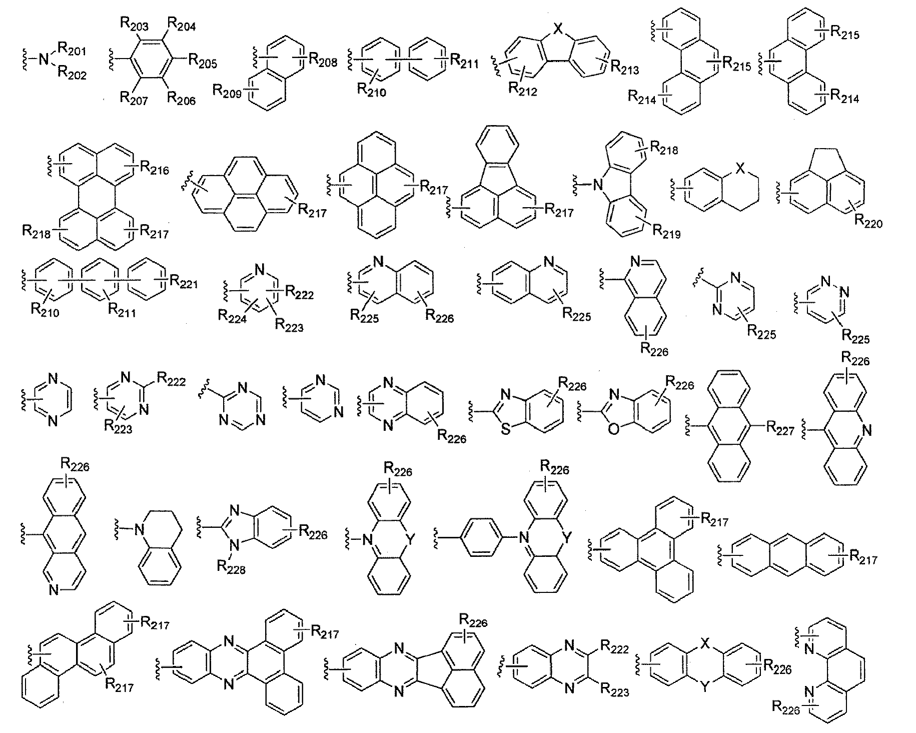 wherein, X and Y independently represent - (CR₇₁R₇₂)m-, -N(R₇₃)-, -S-, -O-, -Si(R₇₄)(R₇₅)-, -P(R₇₆)- or - -(R₈₄)C=C(R₈₅)-; R₇₁ through R₇₆, R₈₄, R₈₅ and m are defined as in Chemical Formula 1; R₂₀₁ and R₂₀₂ independently represent hydrogen, deuterium, (C1-C60)alkyl, (C3-C60)cycloalkyl, (C6-C60)aryl or (C3-C60)heteroaryl containing one or more heteroatom(s) selected from N, O and S; R₂₀₃ through R₂₂₈ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl;
the alkyl, aryl, heteroaryl, heterocycloalkyl, cycloalkyl, trialkylsilyl, dialkylarylsilyl, triarylsilyl, alkenyl, alkynyl, alkylamino or arylamino of R₂₀₁, R₂₀₂ and R₂₀₃ through R₂₂₈ may be further substituted by one or more substituent(s) selected from deuterium, halogen, (C1-C60)alkyl, halo(C1-C60)alkyl, (C6-C60)aryl, (C3-C60)heteroaryl containing one or more heteroatom(s) selected from N, O and S, with or without (C6-C60)aryl substituent(s), morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl. More specifically, group 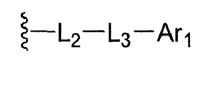 is independently selected from the following structures, but not restricted thereto.

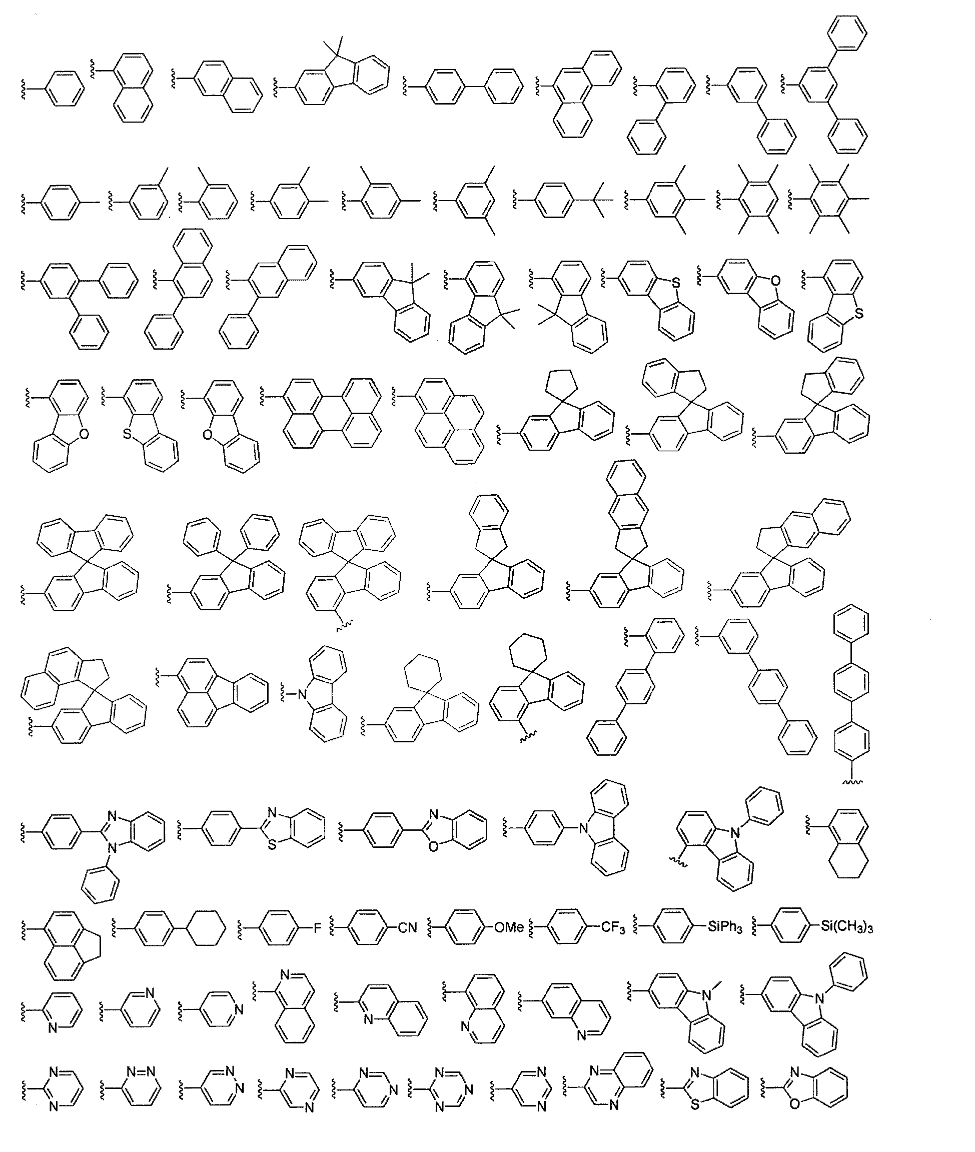

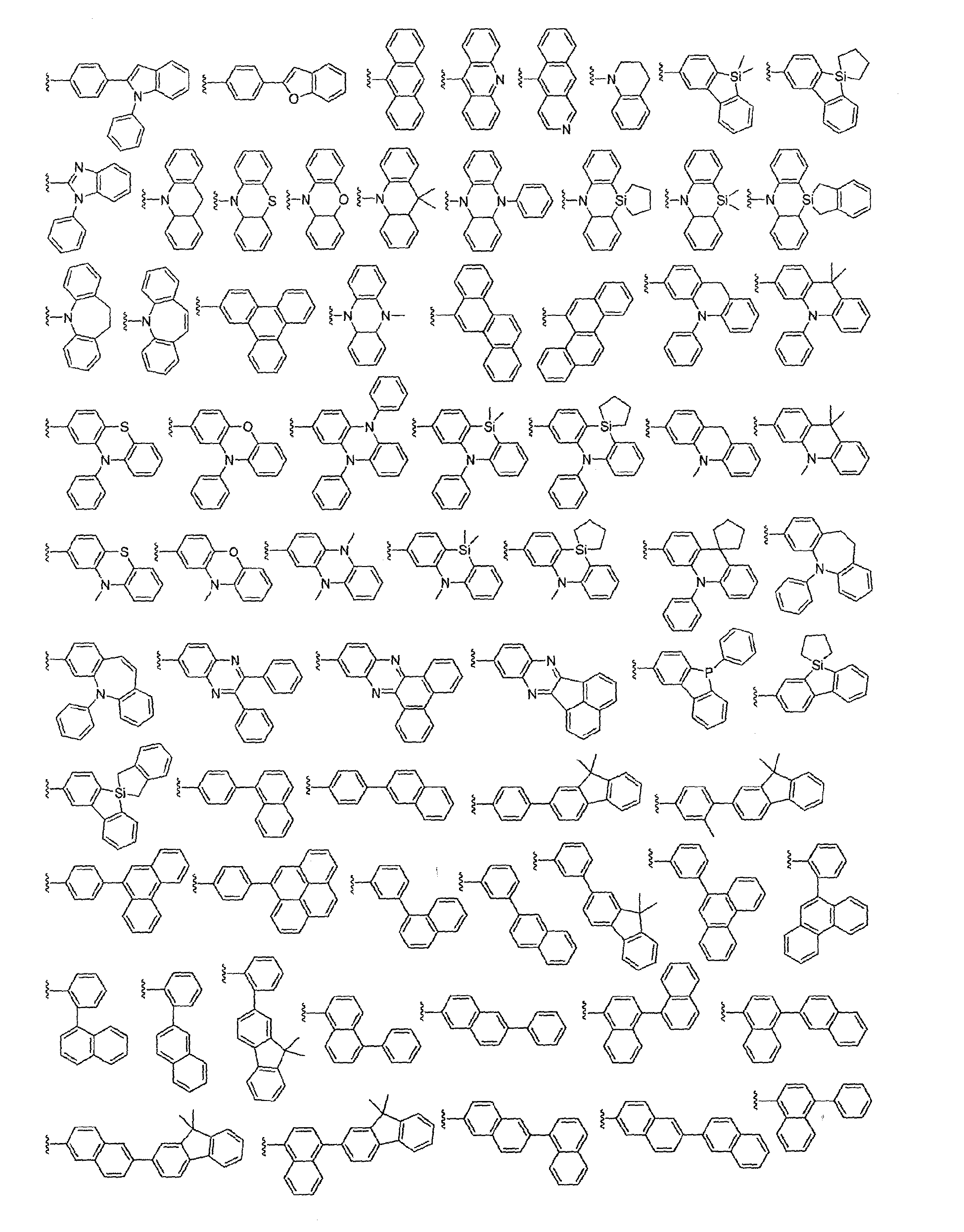

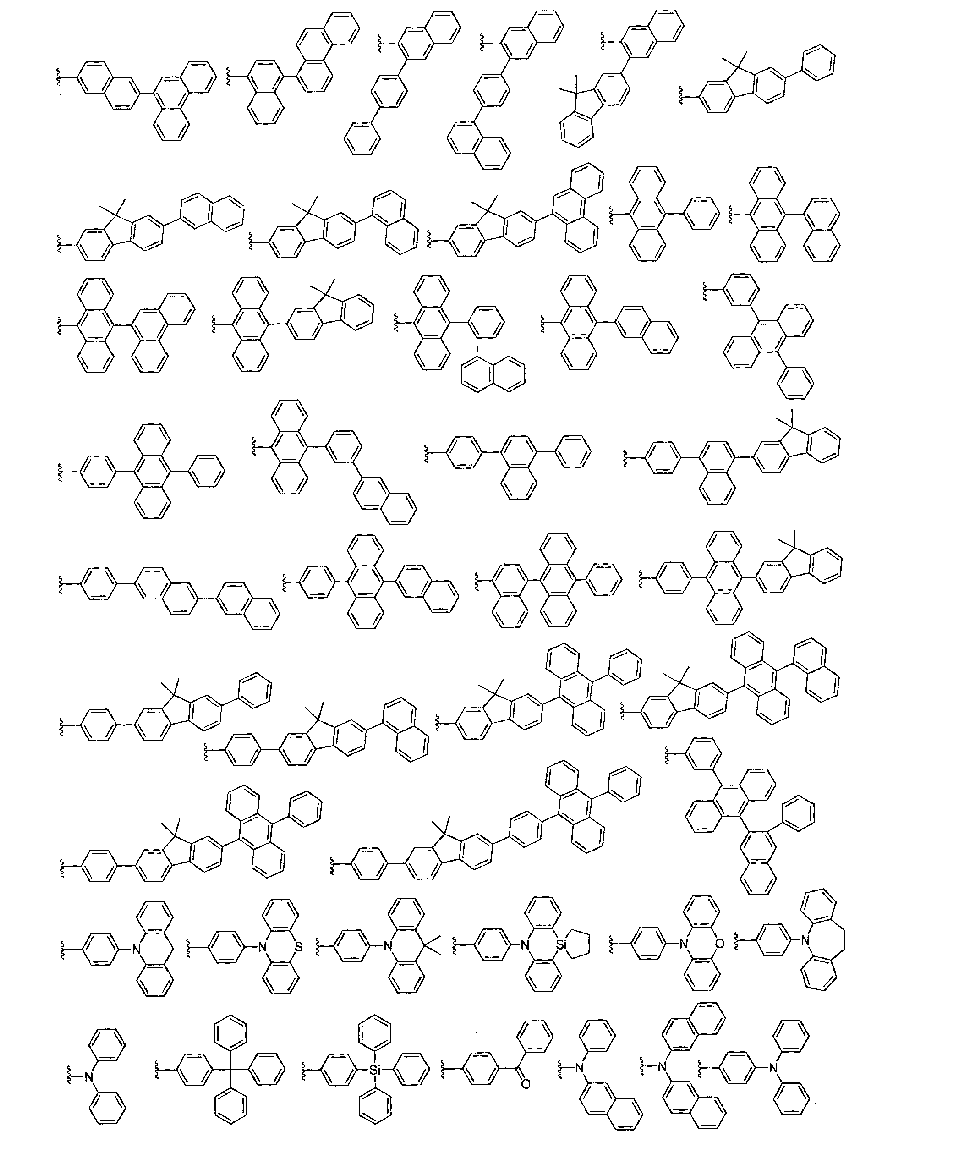

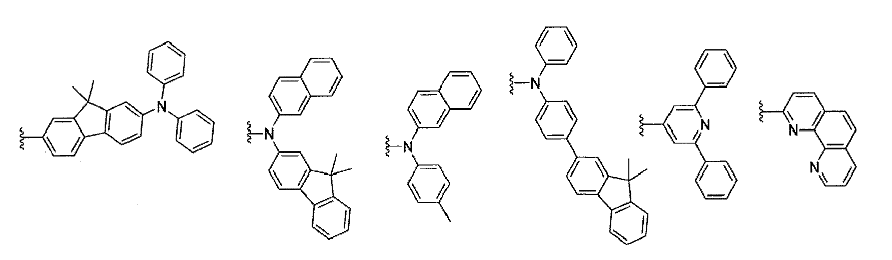

R₁ though R₁₁ independently represent hydrogen, deuterium, fluoro, chloro, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, benzyl, trifluoromethyl, perfluoroethyl, trifluoroethyl, perfluoropropyl, perfluorobutyl, methoxy, ethoxy, n-propoxy, i-propoxy, n-butoxy, i-butoxy, t-butoxy, n-pentoxy, i-pentoxy, n-hexyloxy, n-heptyloxy, cyclopropyl, cyclobutyl, cyclopentyl, cyclohexyl, cycloheptyl, cyclooctyl, cyclononyl, cyclodecyl, morpholino, thiomorpholino, morpholinyl, thiomorpholinyl, trimethylsilyl, triethylsilyl, tripropylsilyl, tri(t-butyl)silyl, t-butyldimethylsilyl, dimethylphenylsilyl, triphenylsilyl, bicycle[2.2.1]heptyl, bicycle[2.2.2]octyl, bicycle[5.2.0]nonyl, bicycle[4.2.2]decyl4-pentylbicycle[2.2.2]octyl, ethenyl, phenylethenyl, ethynyl, phenylethynyl, cyano, methylthio, phenyloxy, phenylthio, methoxycarbonyl, ethoxycarbonyl, t-butoxycarbonyl, methylcarbonyl, ethylcarbonyl, benzylcarbonyl, phenylcarbonyl, carboxyl, nitro or hydroxyl.

The organic electroluminescent compounds according to the present invention can be specifically exemplified by the following compounds, but they are not restricted thereto:

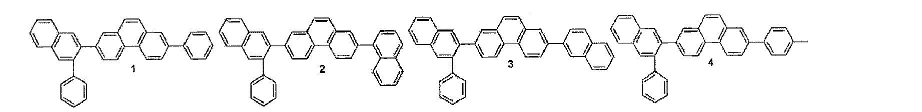

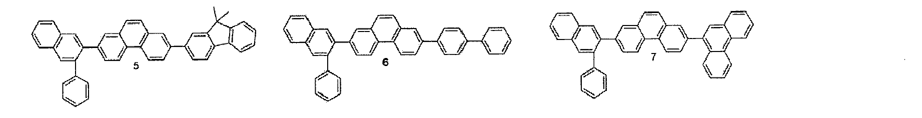

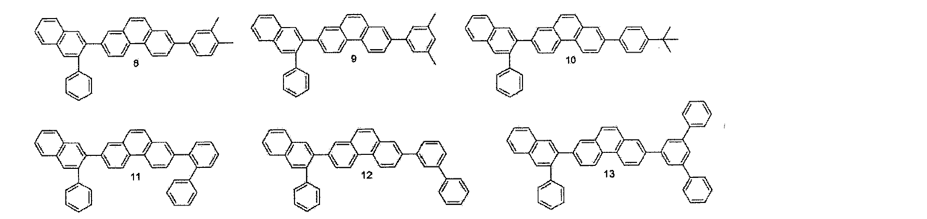

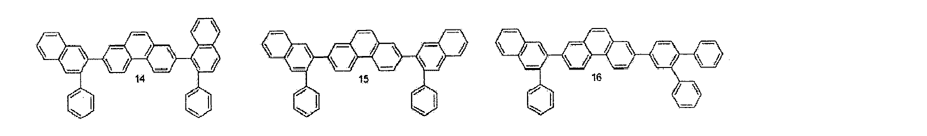

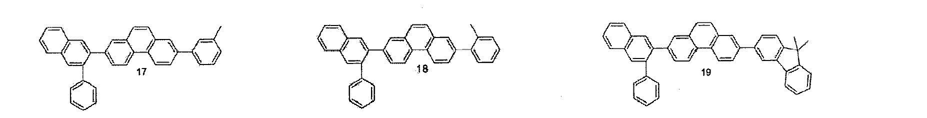

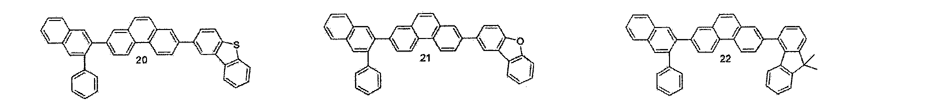

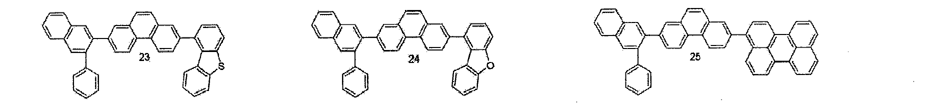

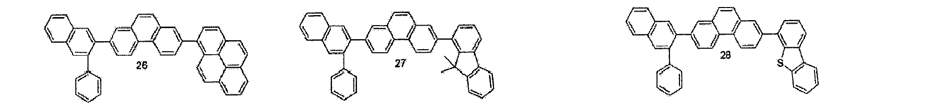

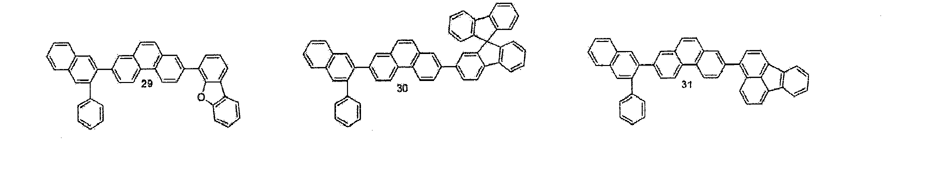

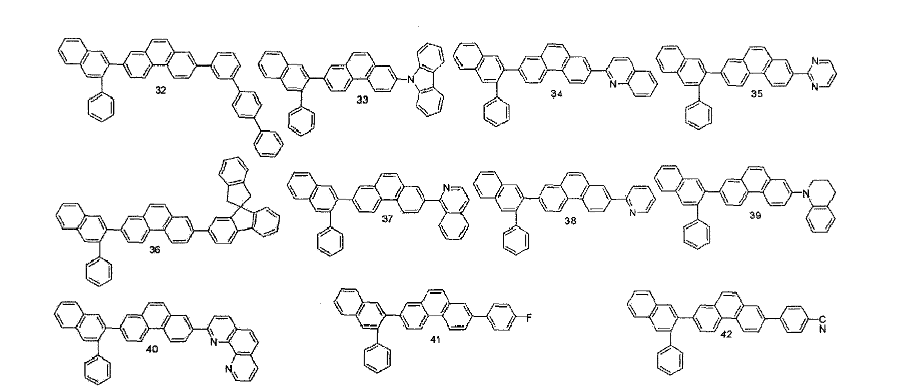

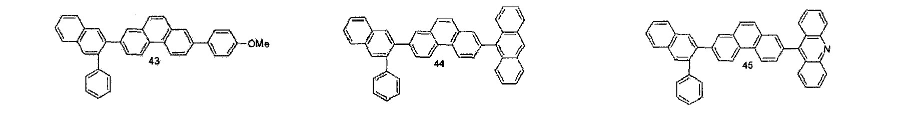

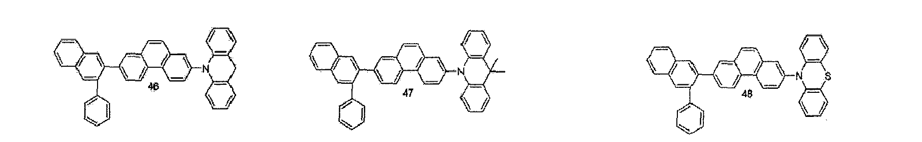

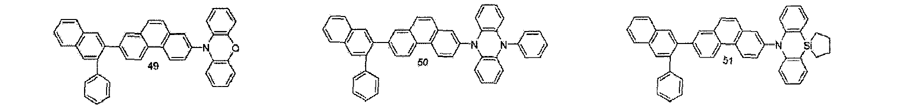

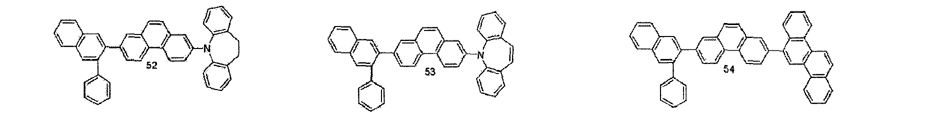

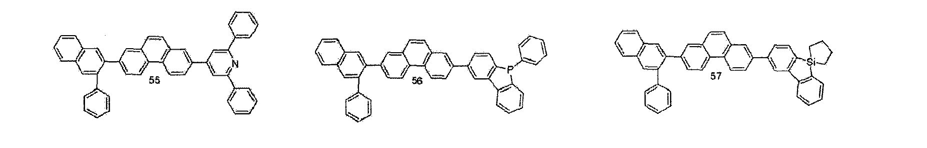

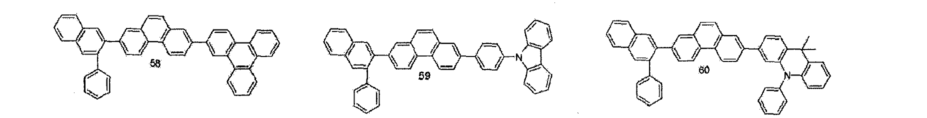

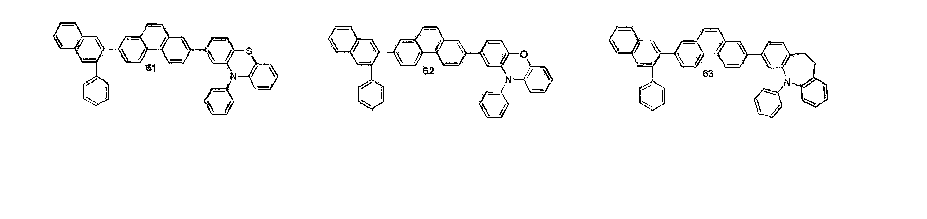

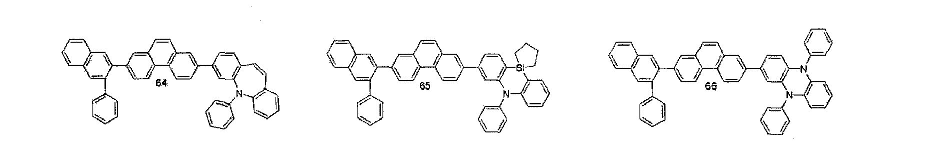

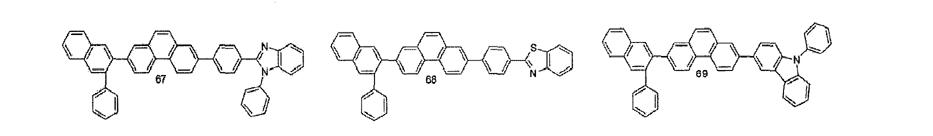

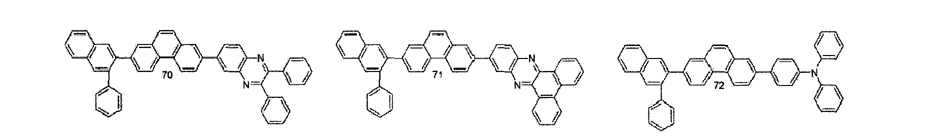

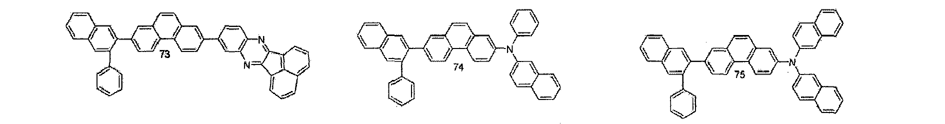

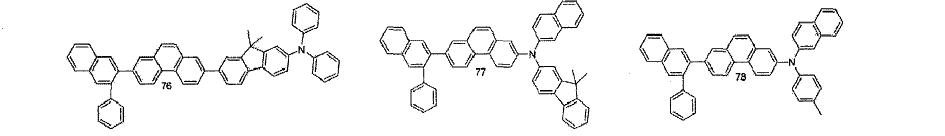

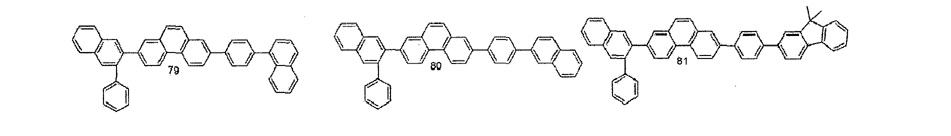

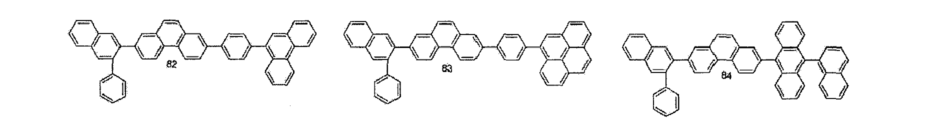

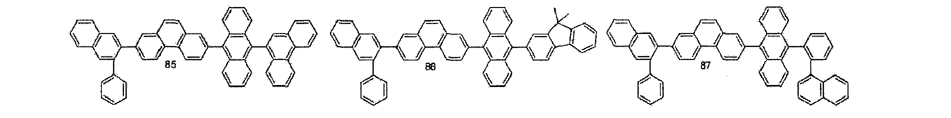

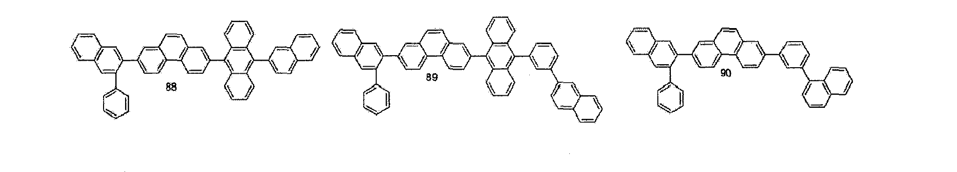

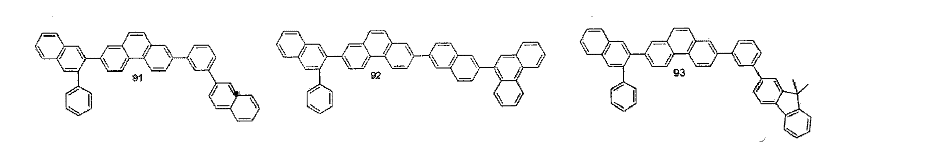

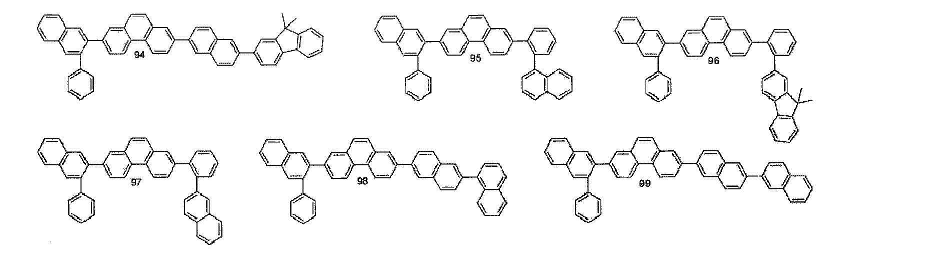

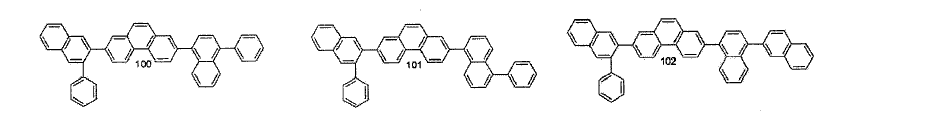

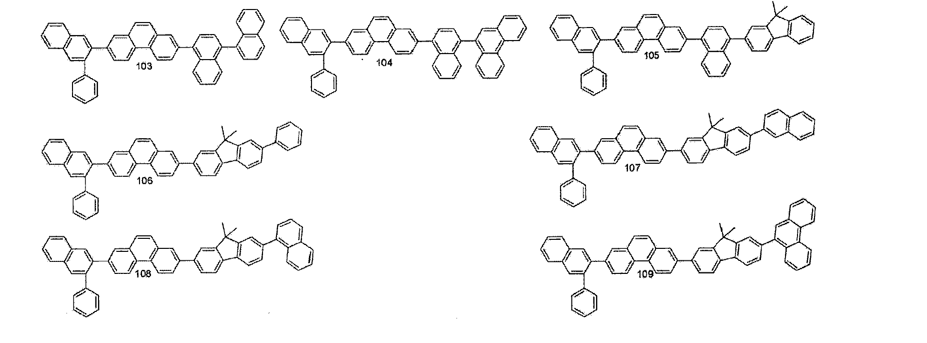

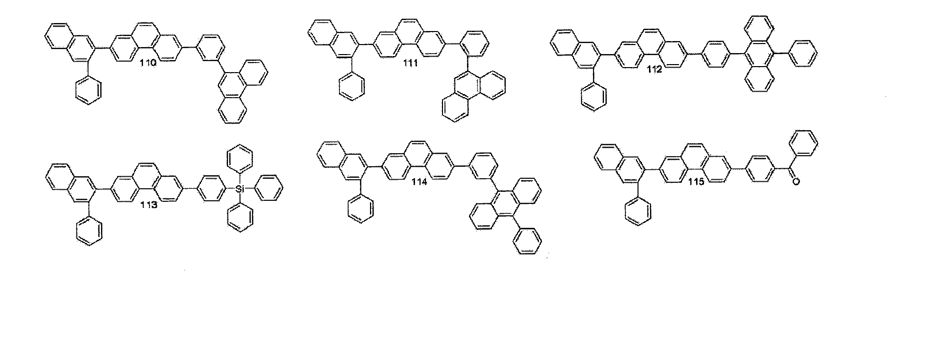

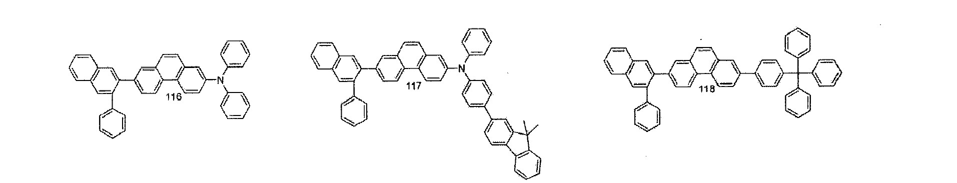

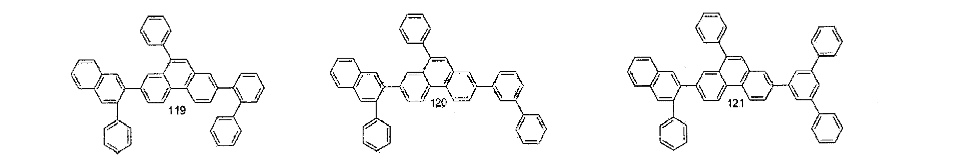

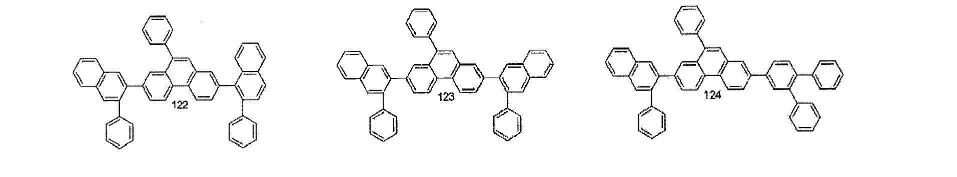

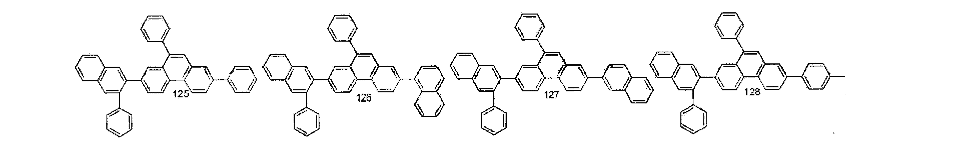

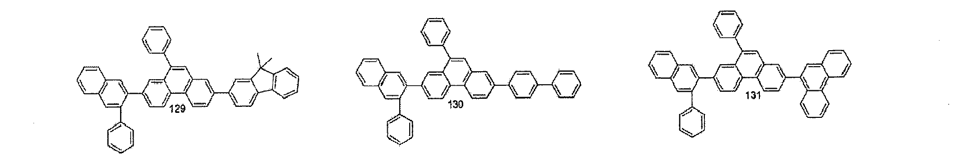

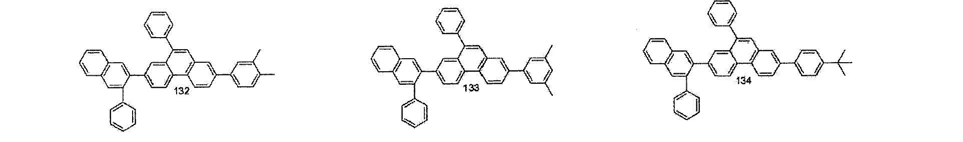

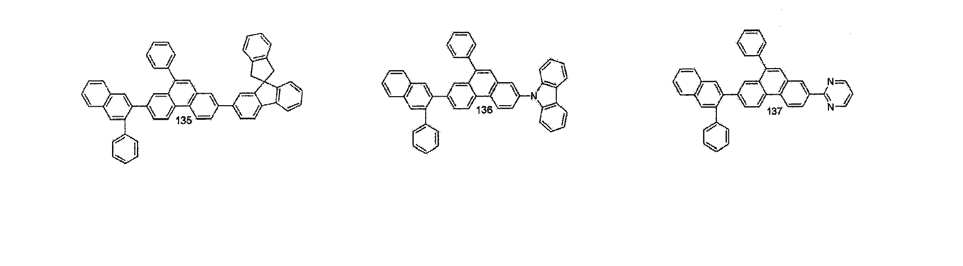

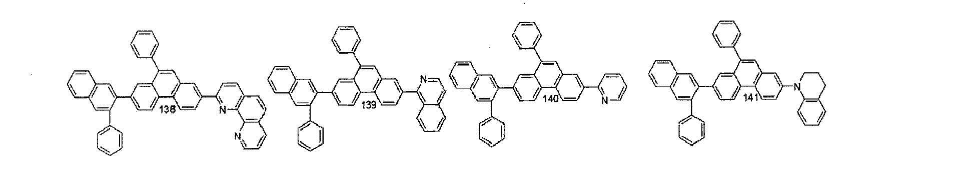

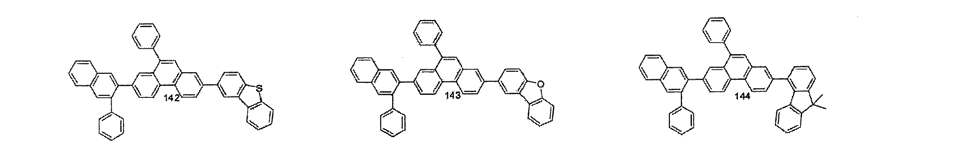

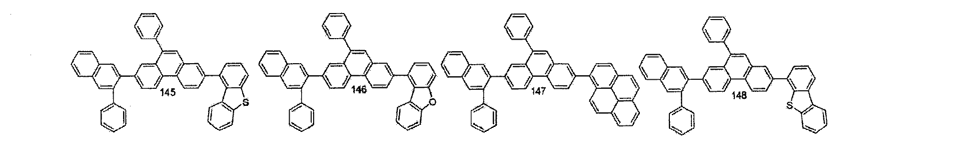

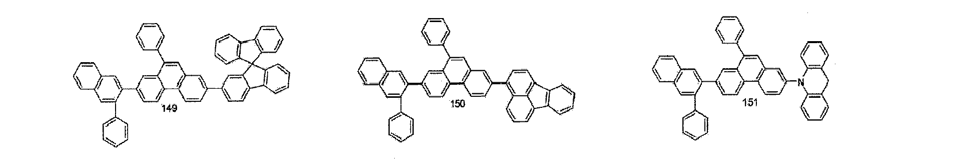

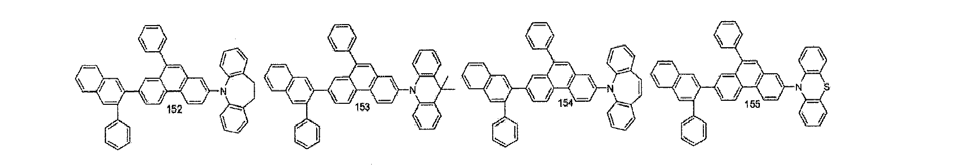

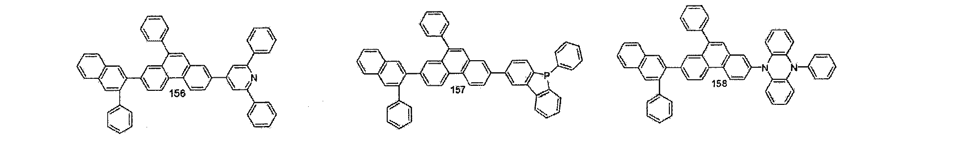

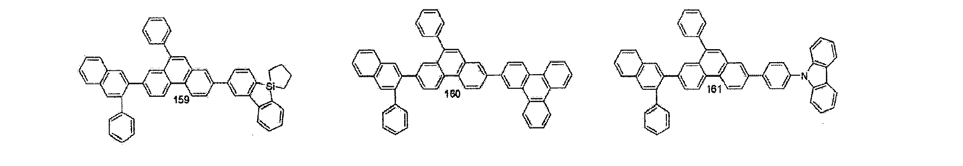

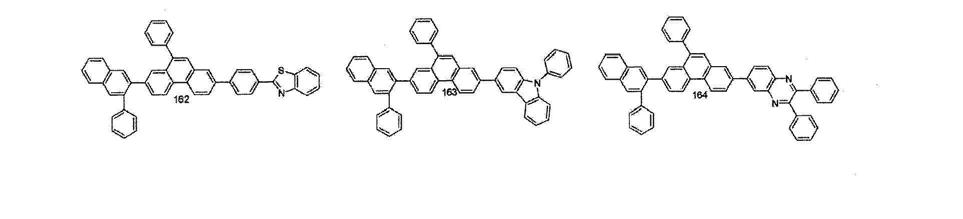

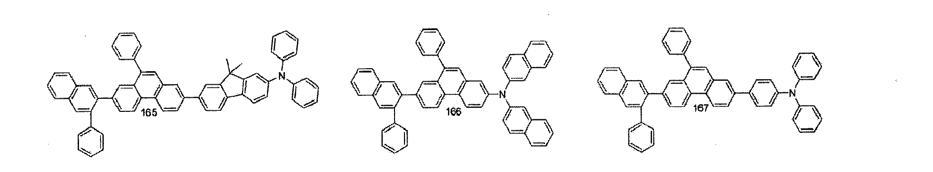

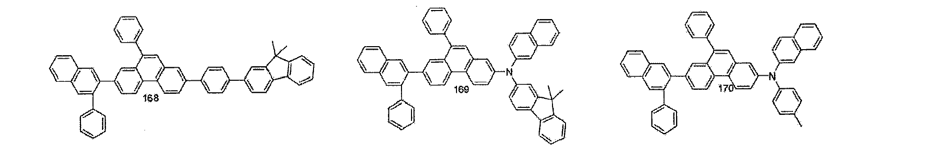

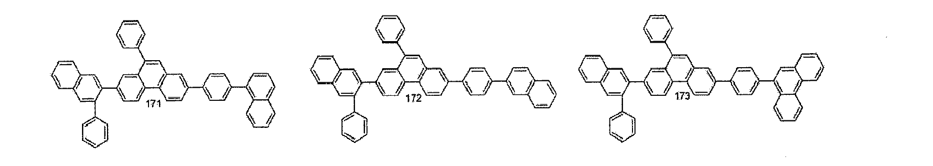

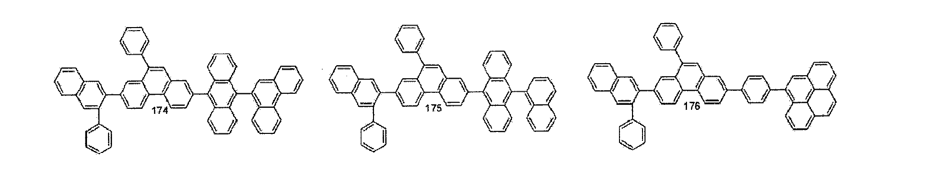

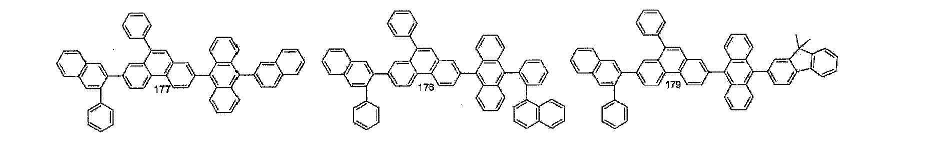

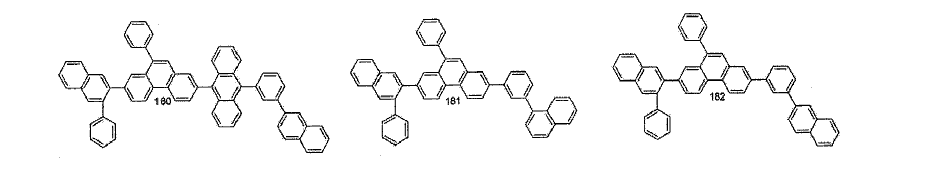

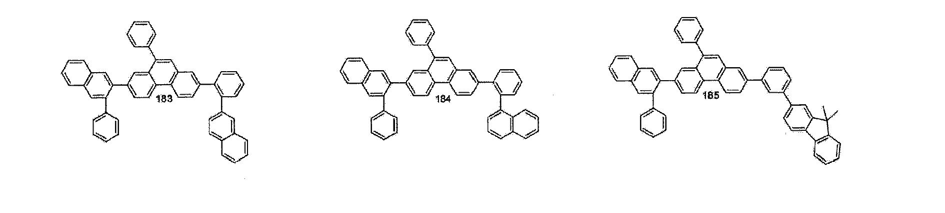

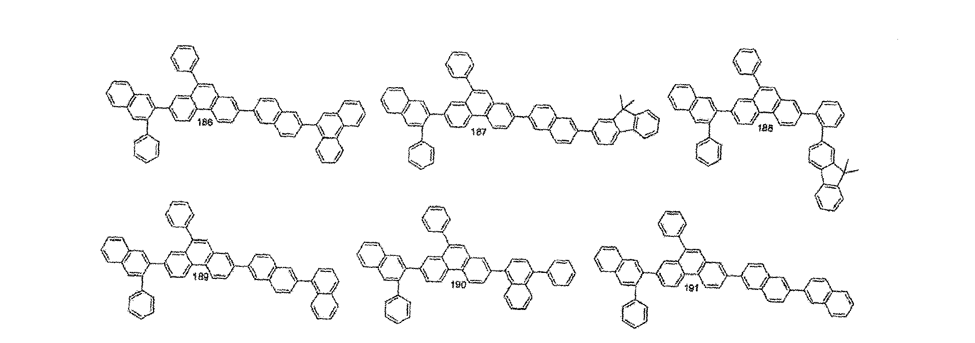

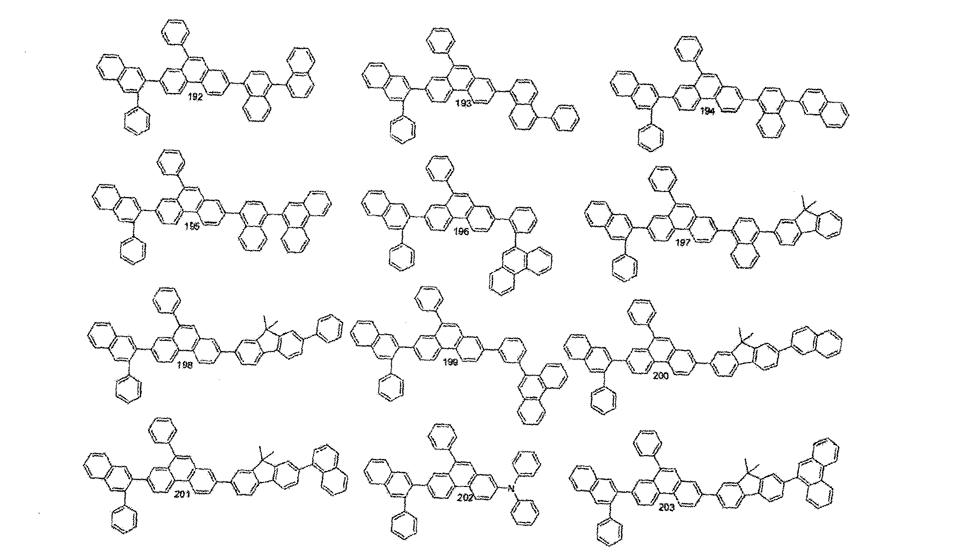

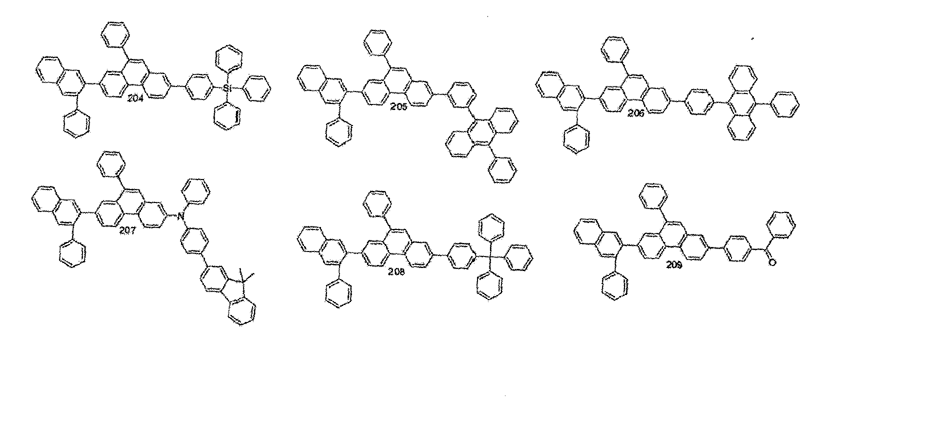

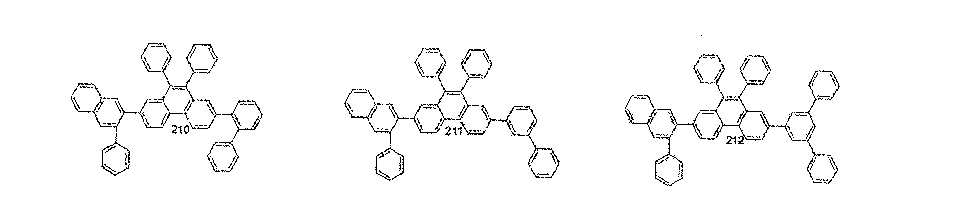

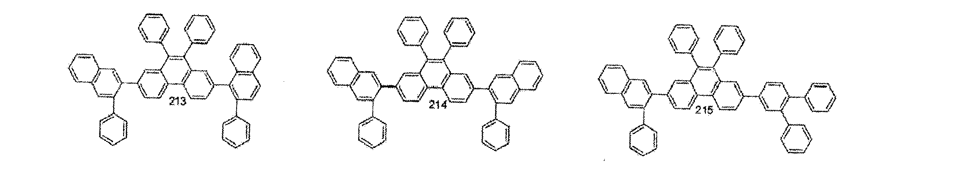

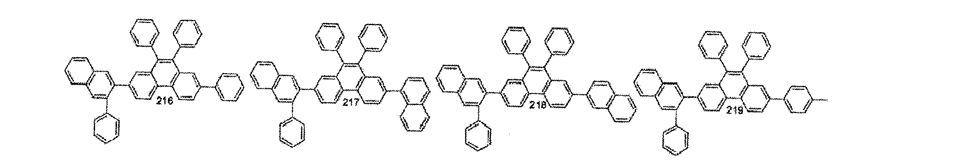

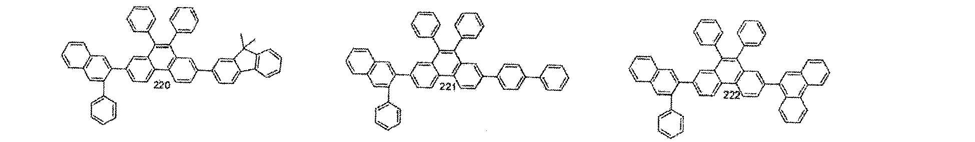

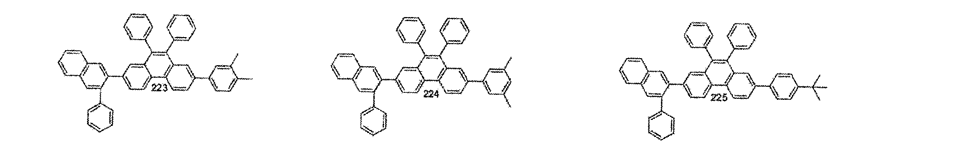

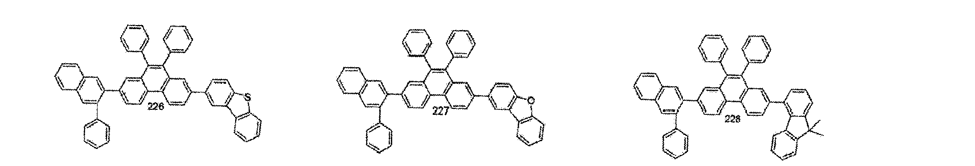

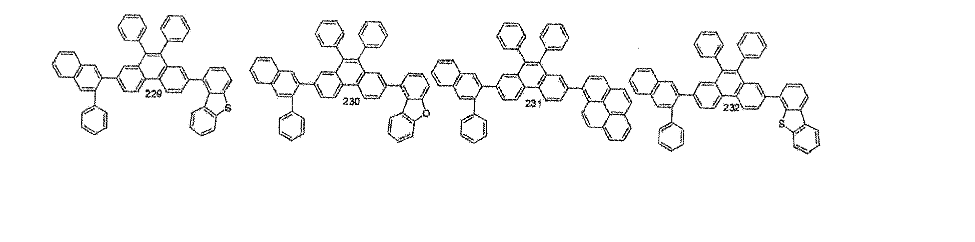

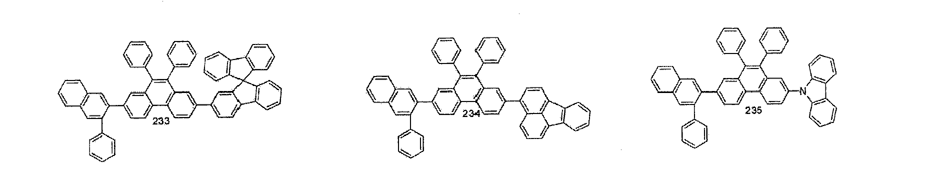

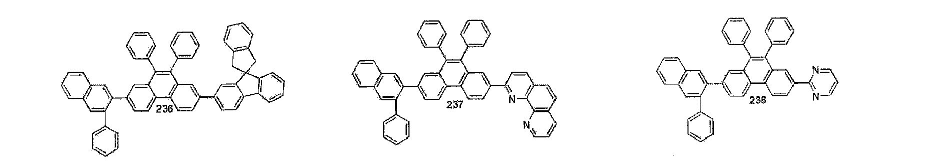

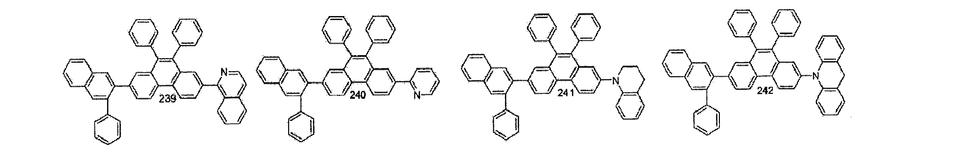

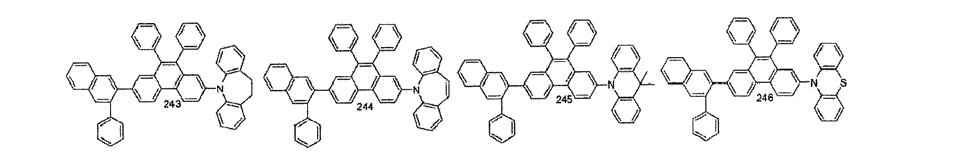

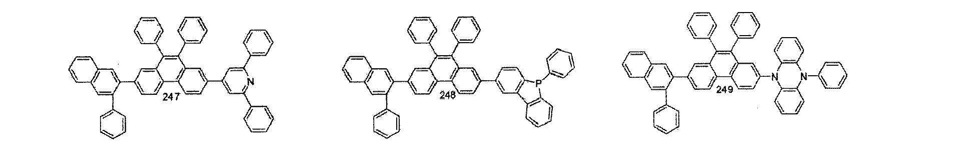

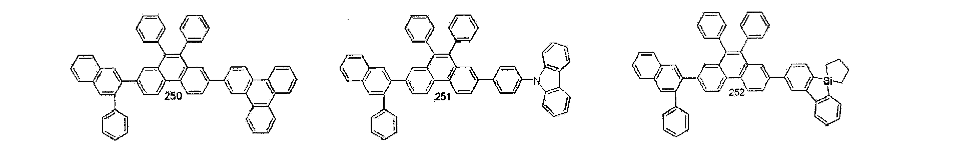

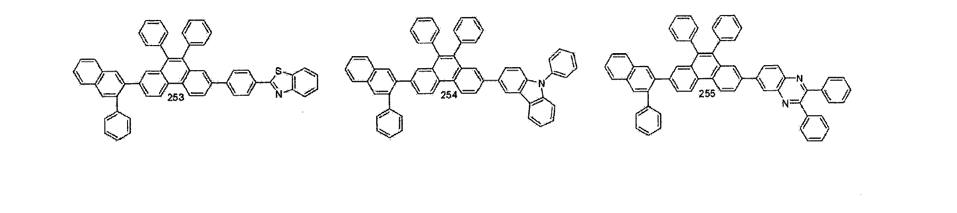

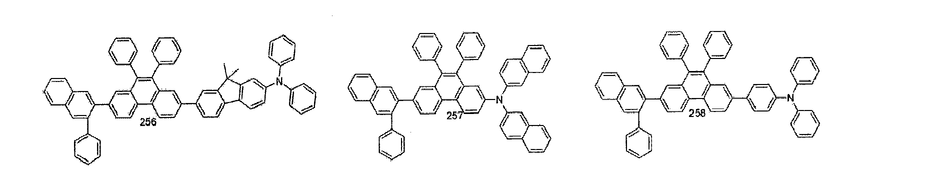

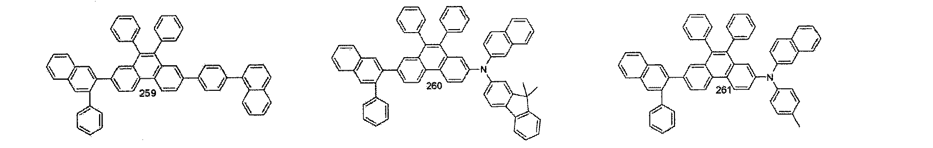

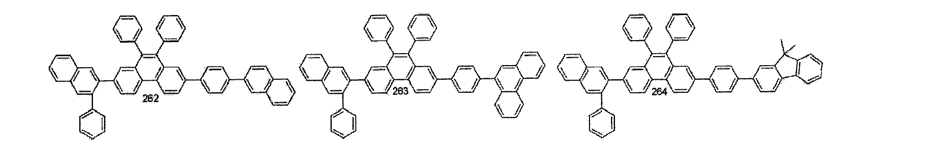

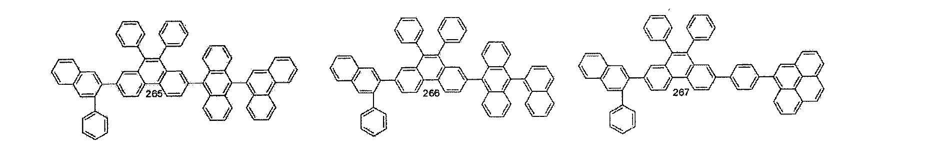

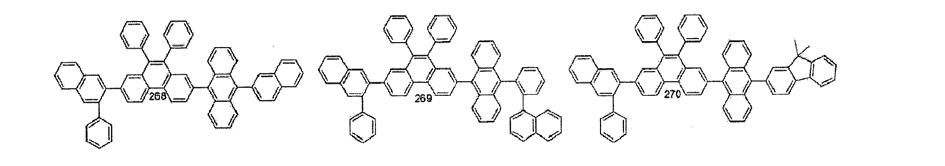

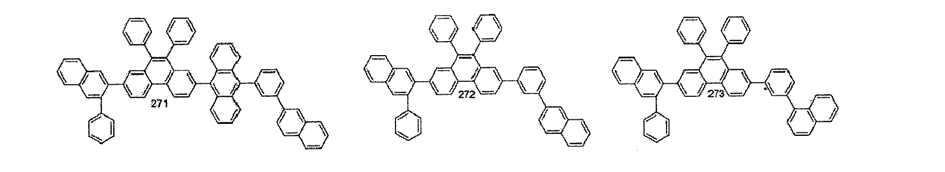

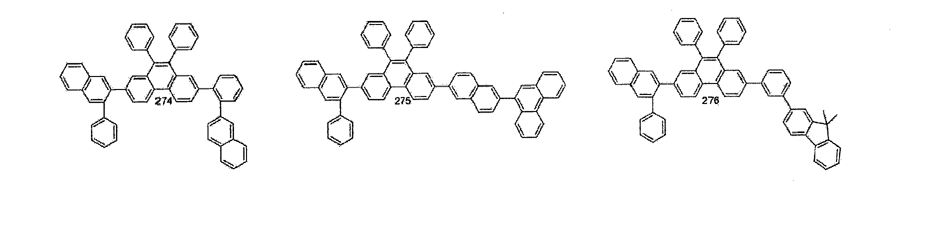

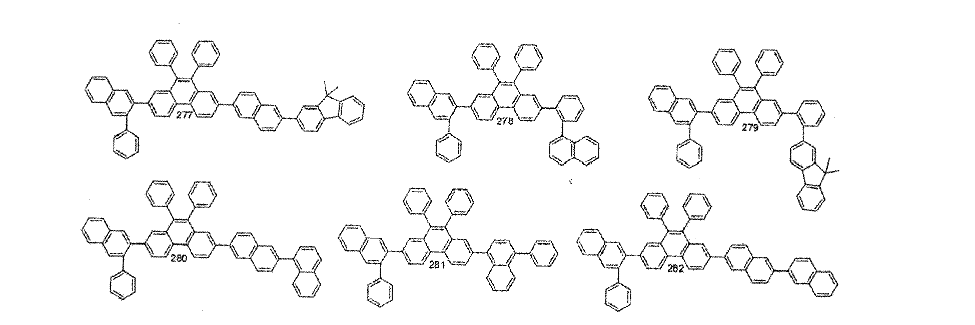

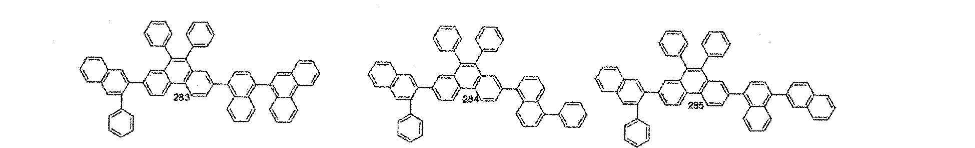

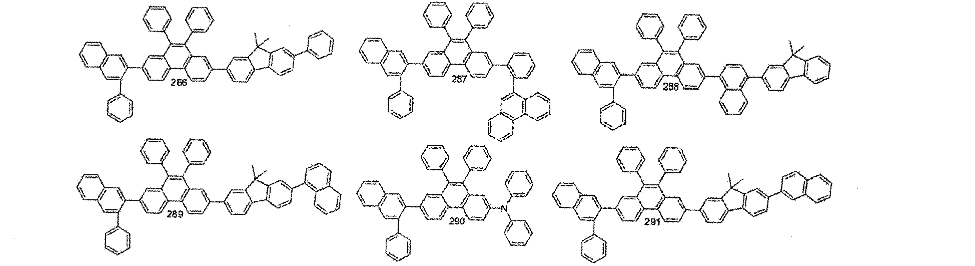

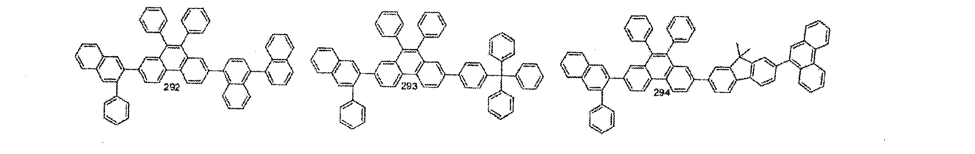

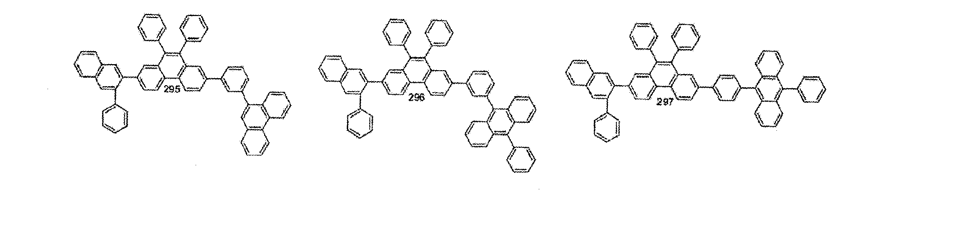

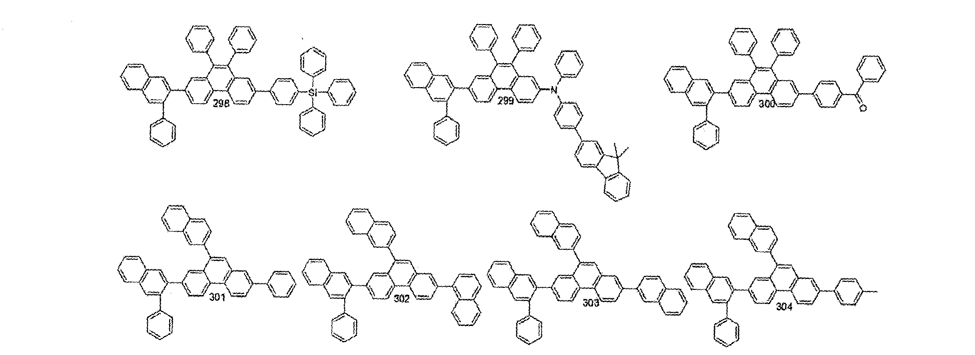

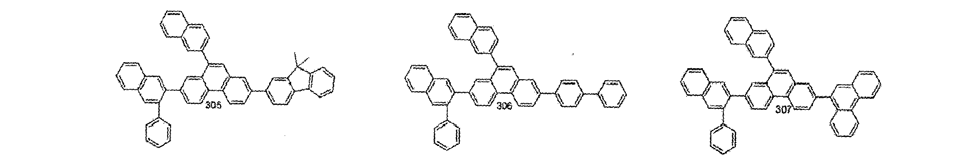

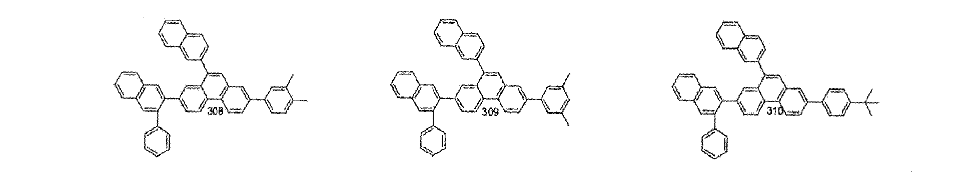

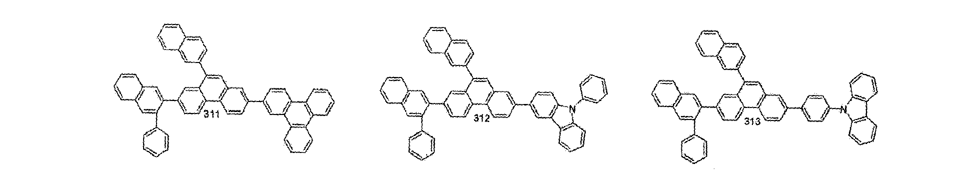

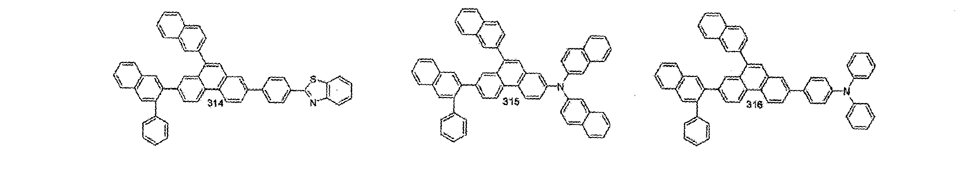

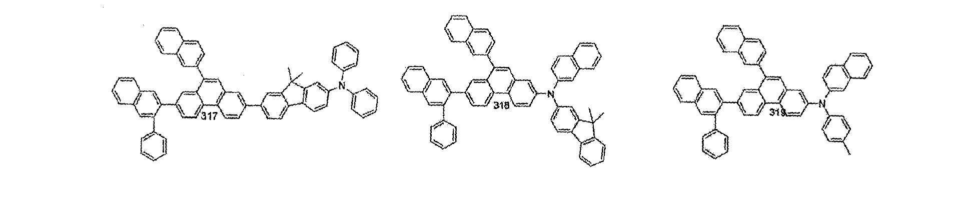

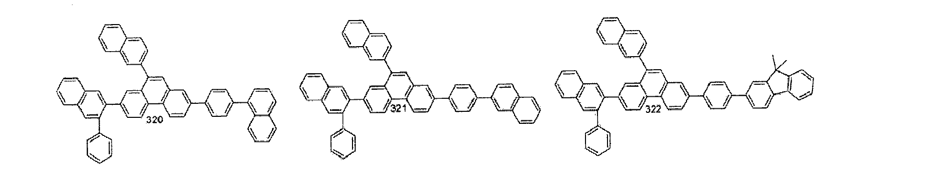

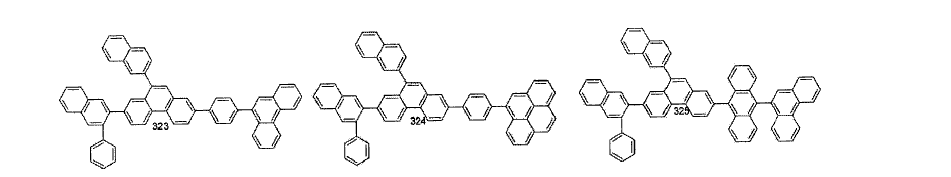

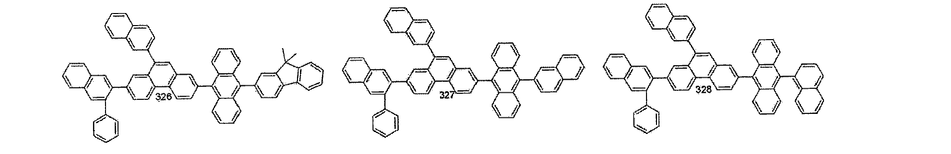

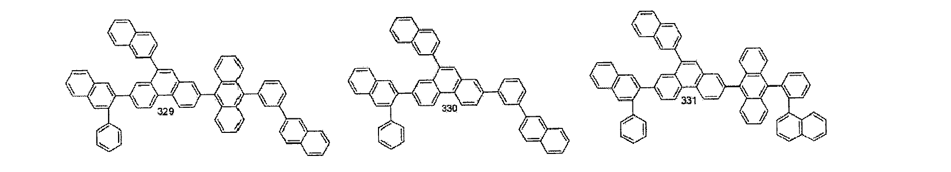

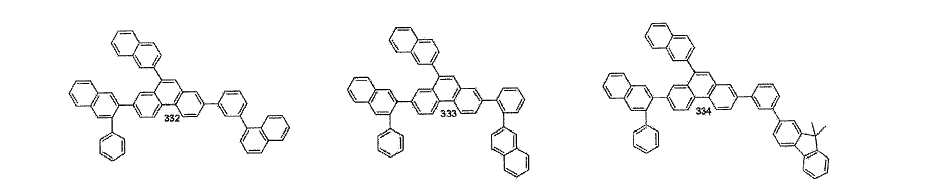

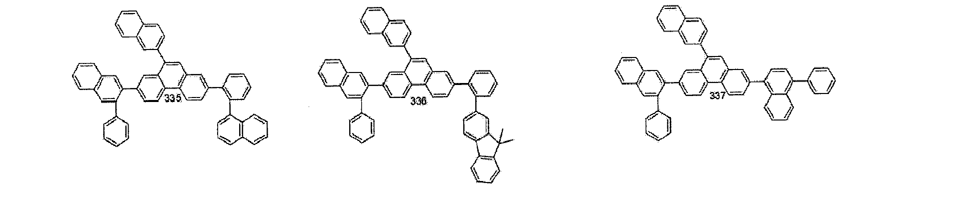

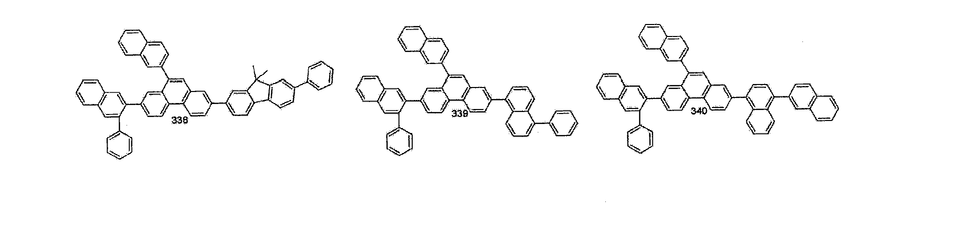

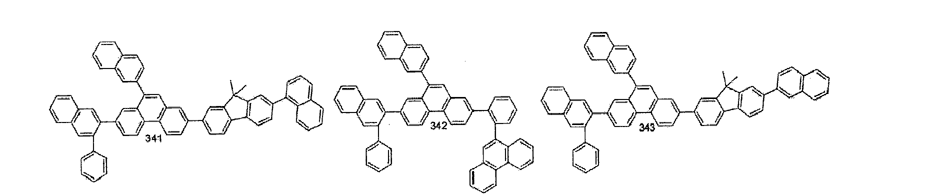

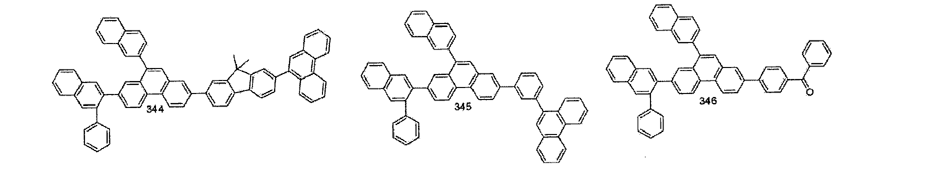

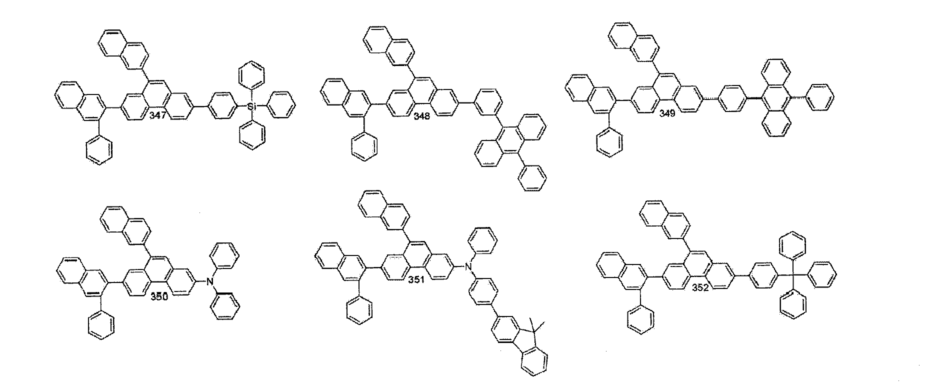

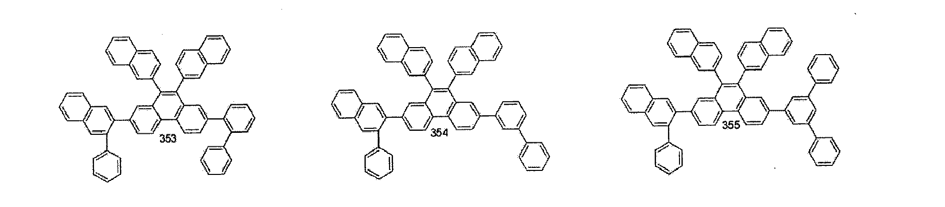

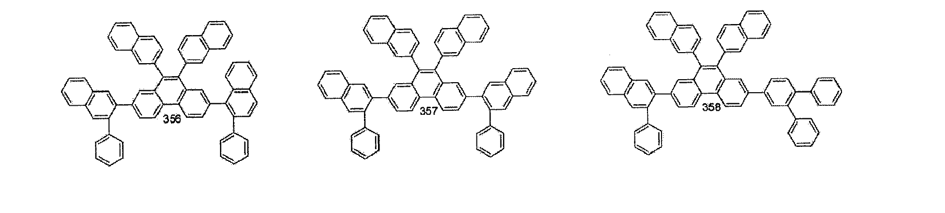

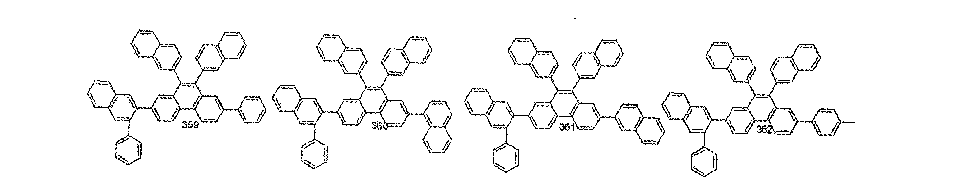

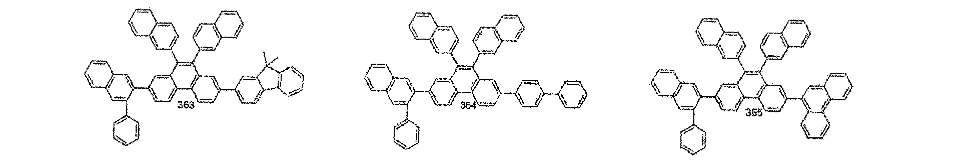

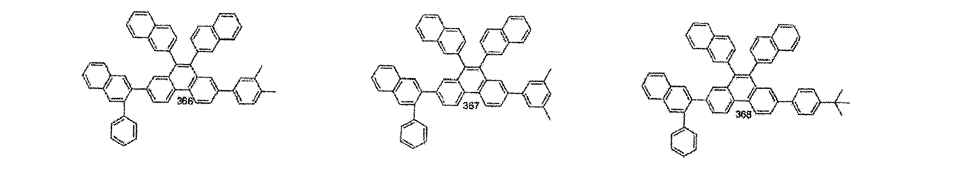

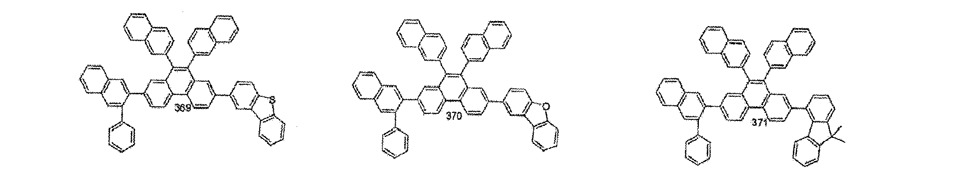

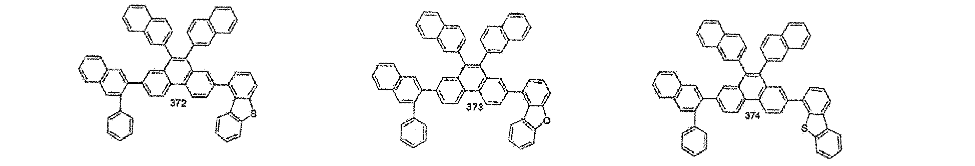

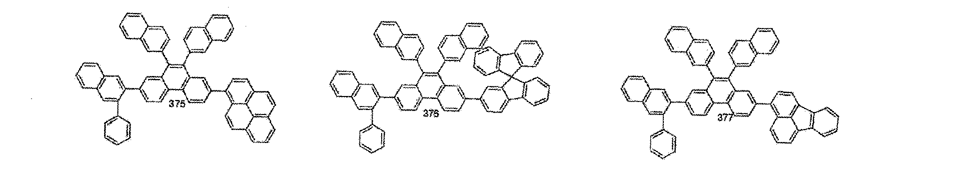

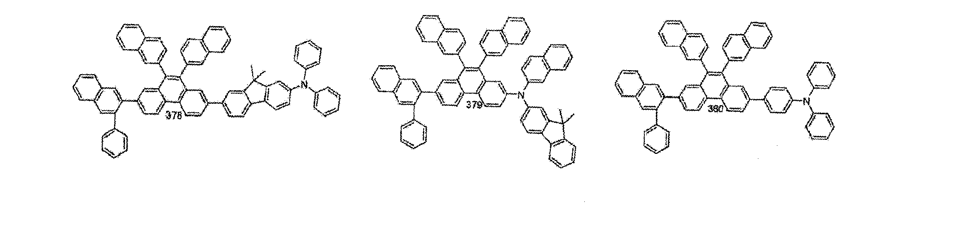

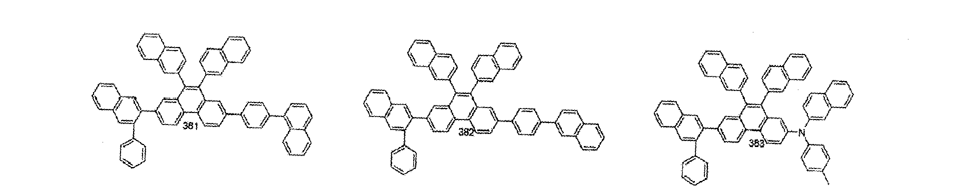

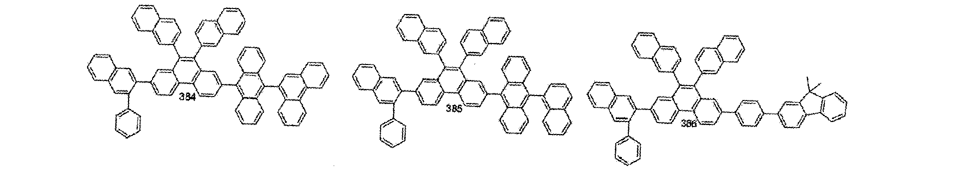

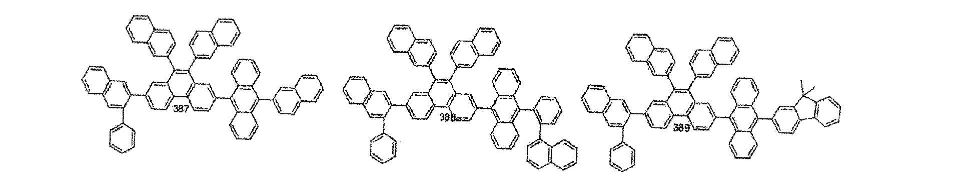

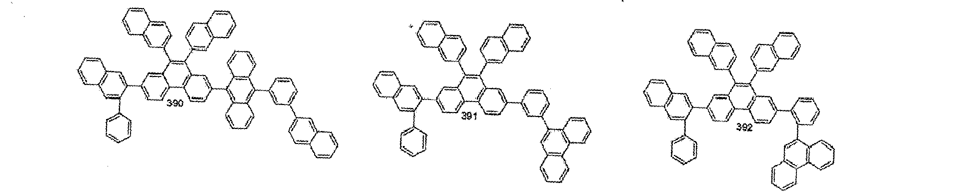

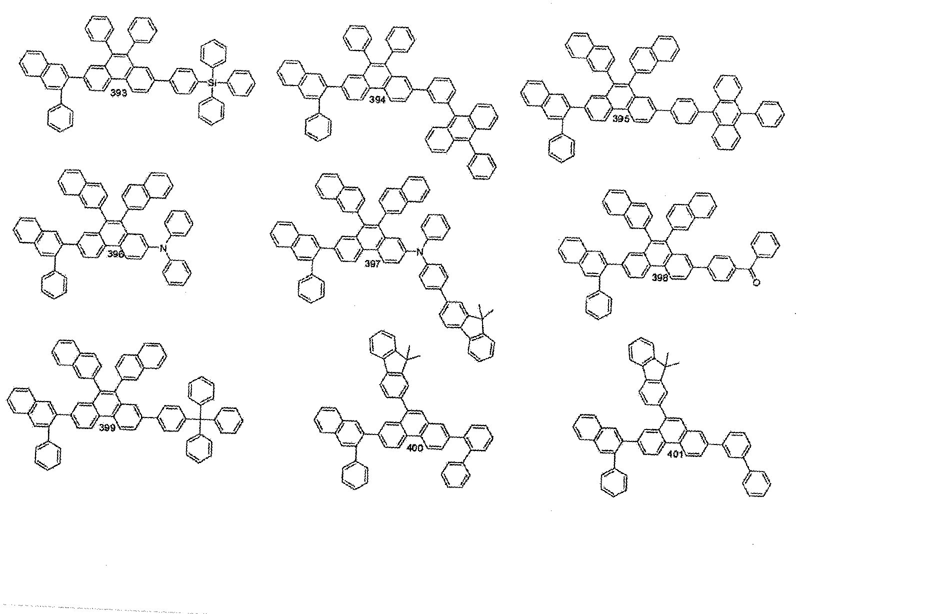

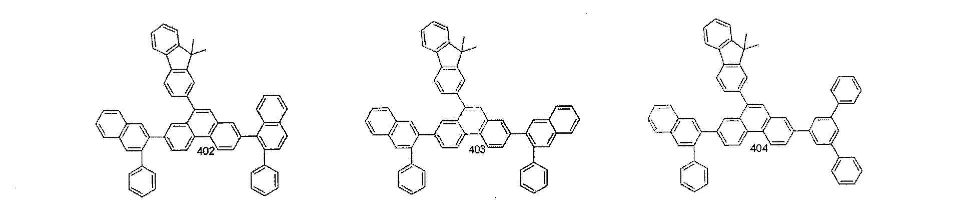

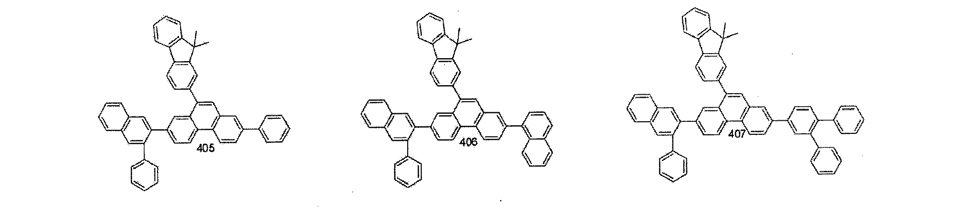

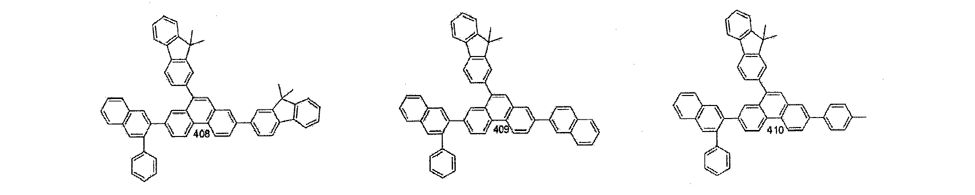

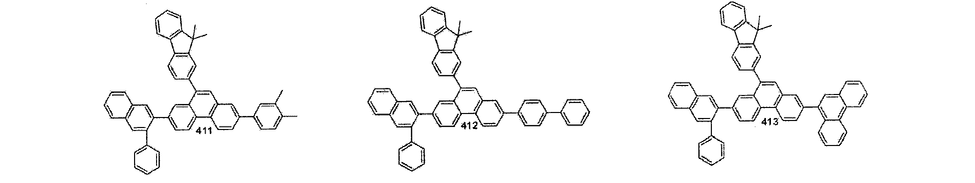

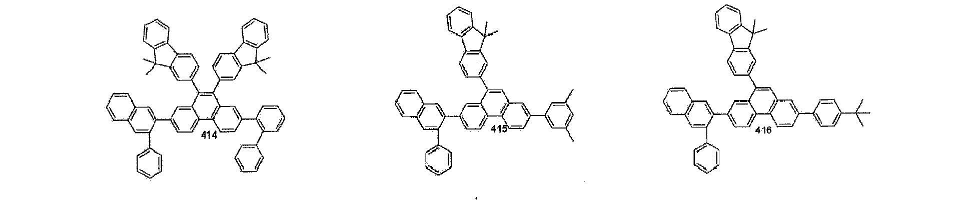

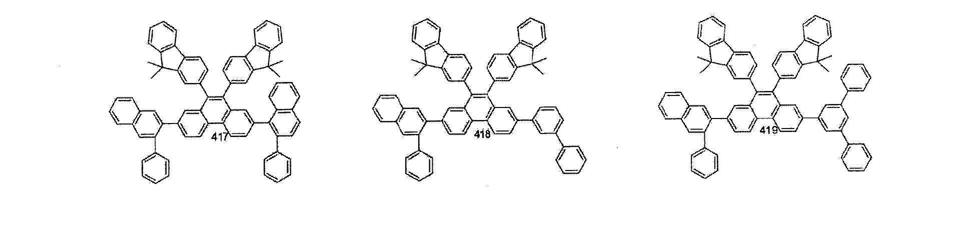

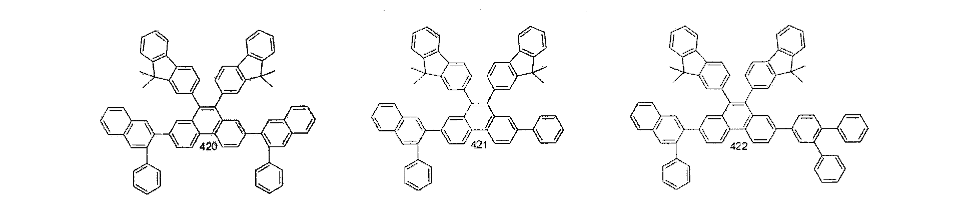

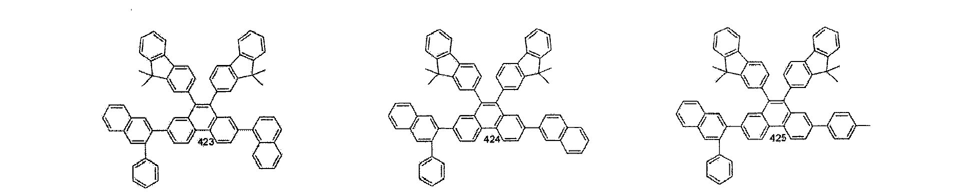

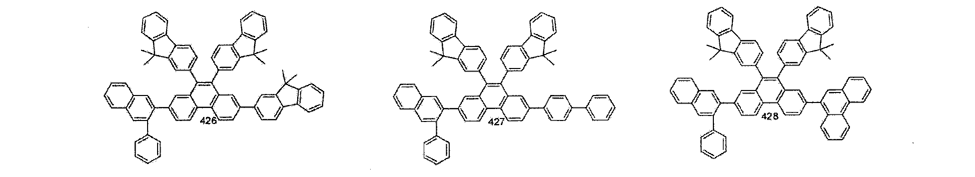

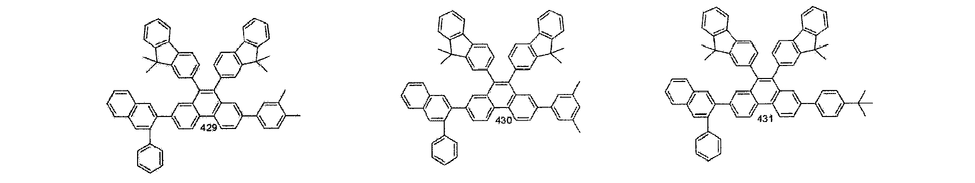

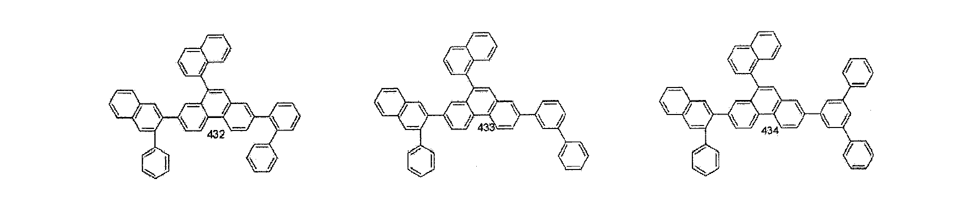

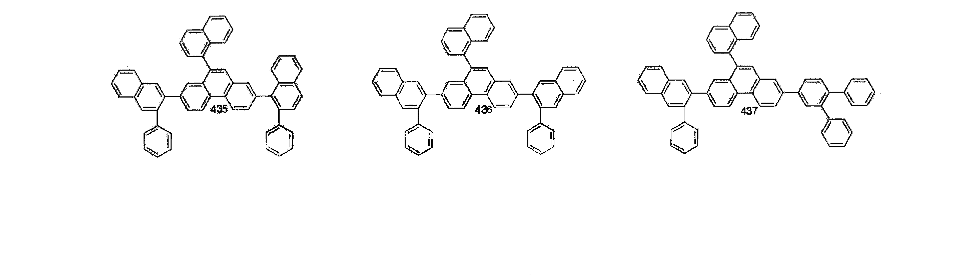

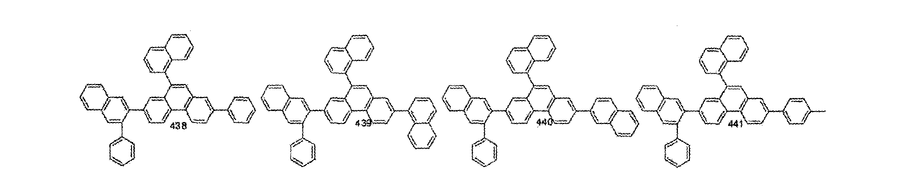

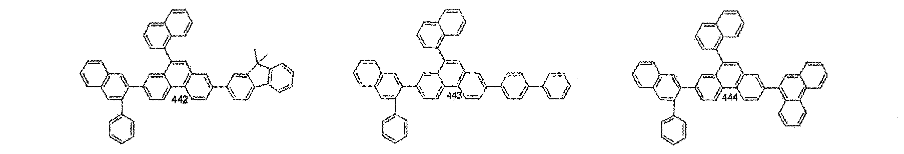

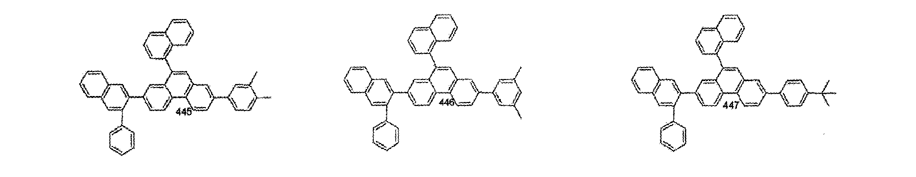

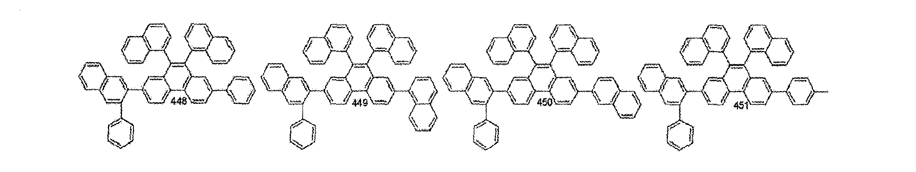

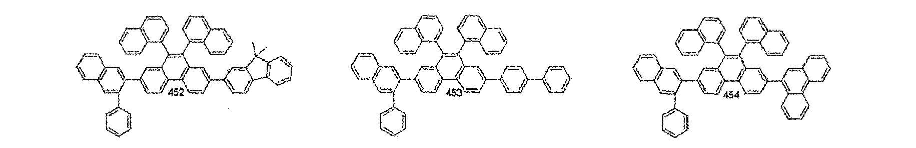

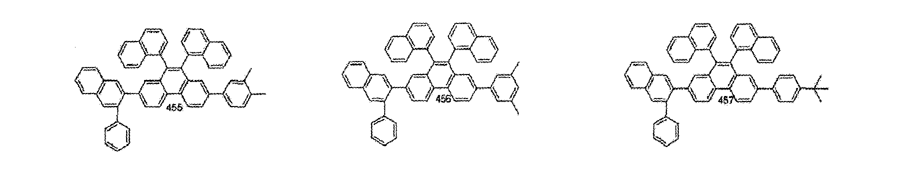

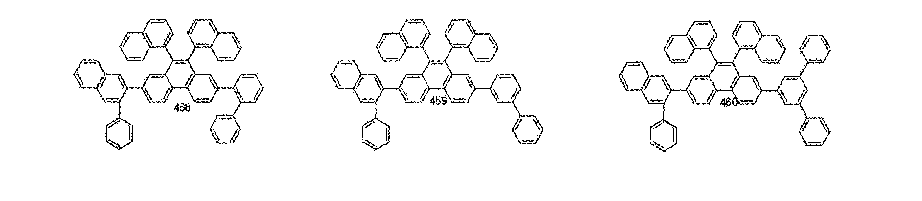

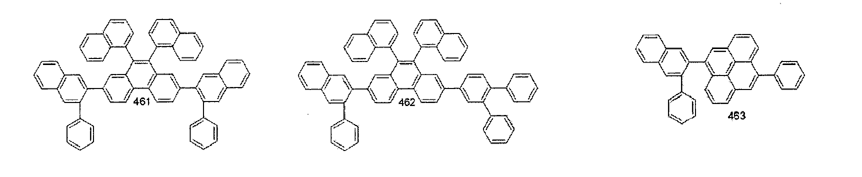

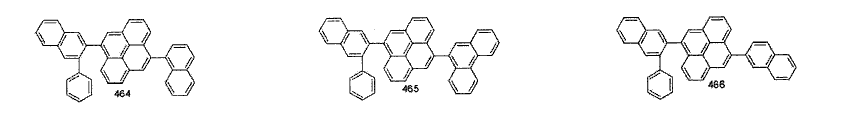

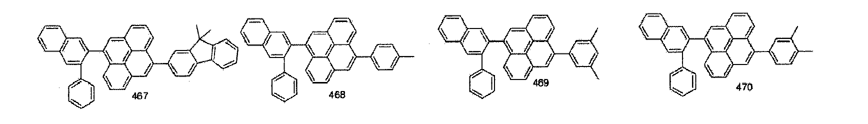

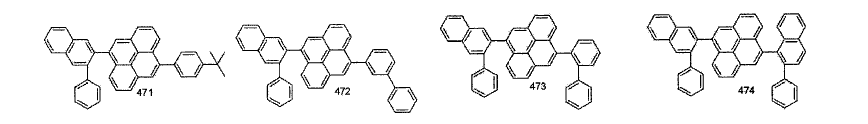

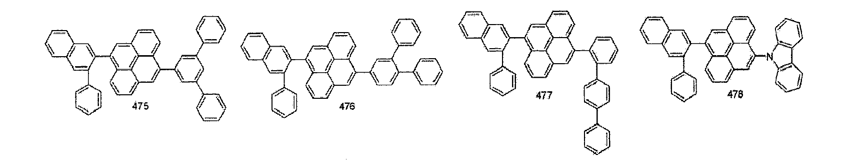

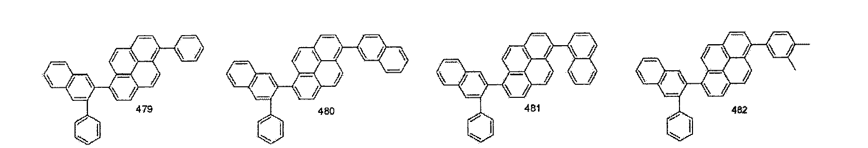

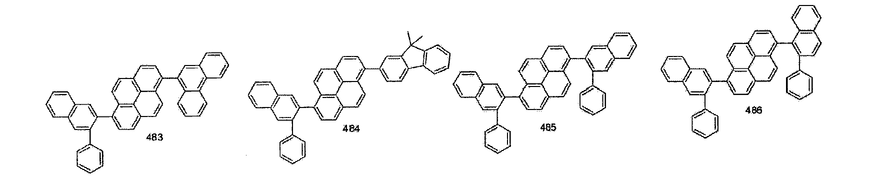

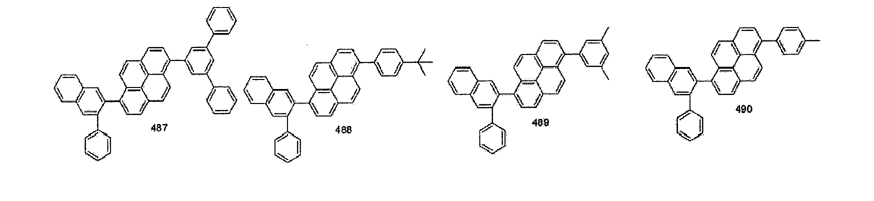

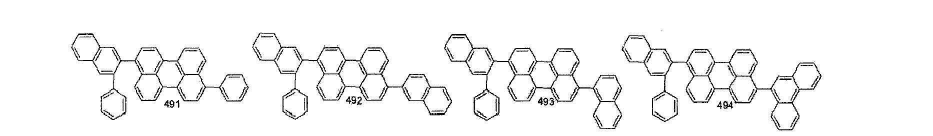

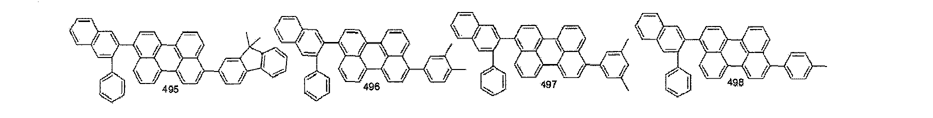

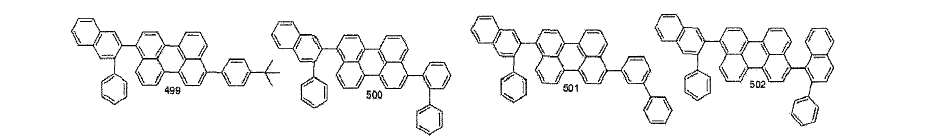

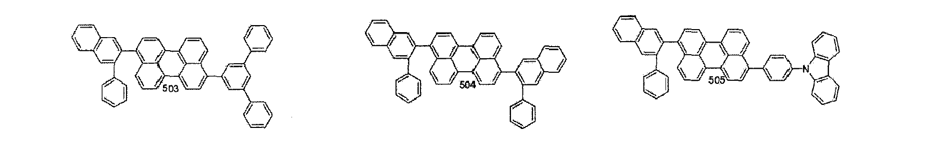

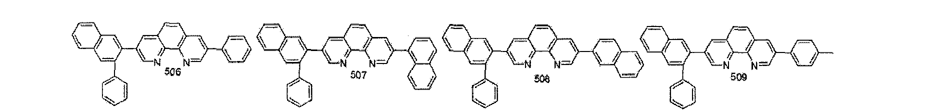

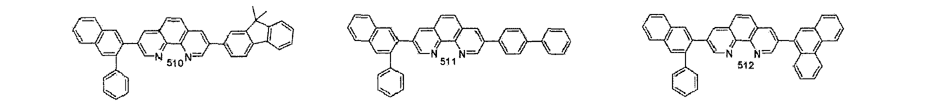

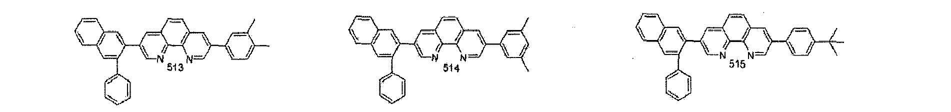

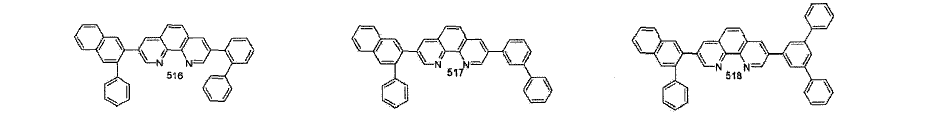

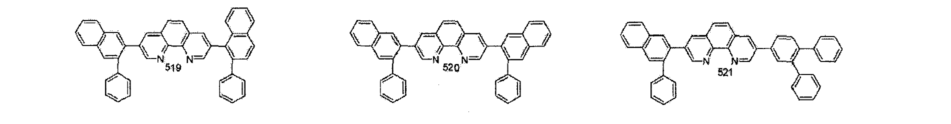

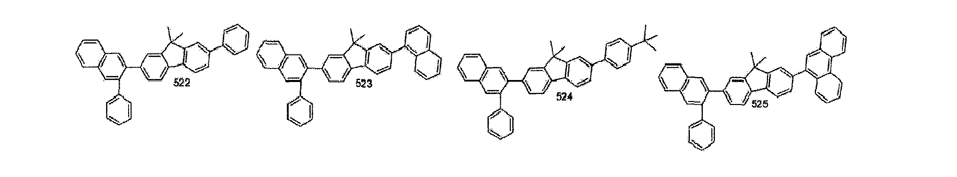

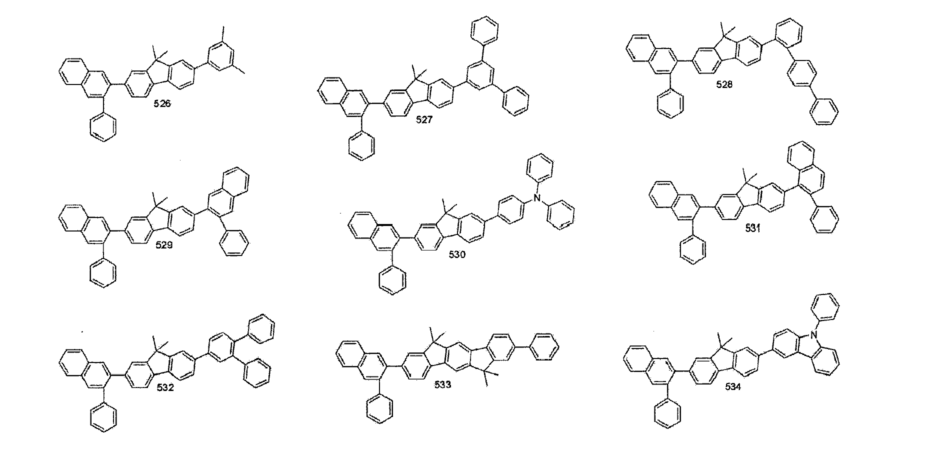

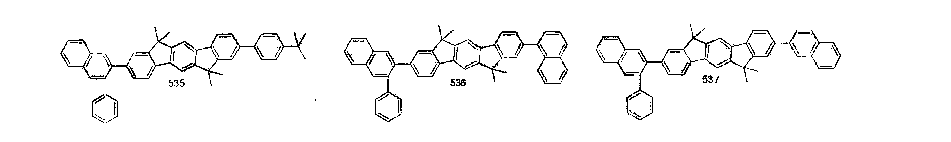

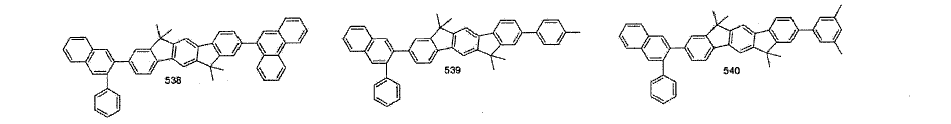

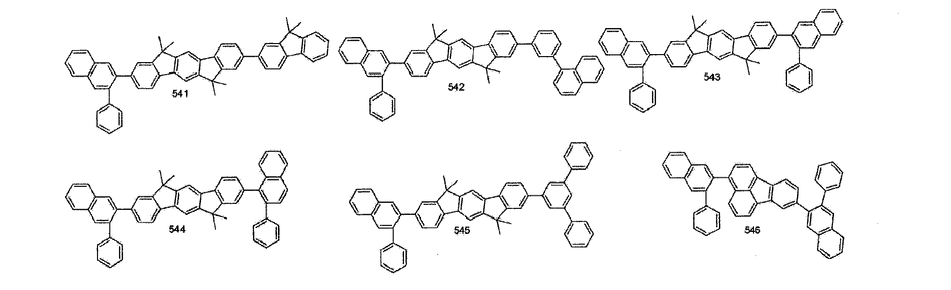

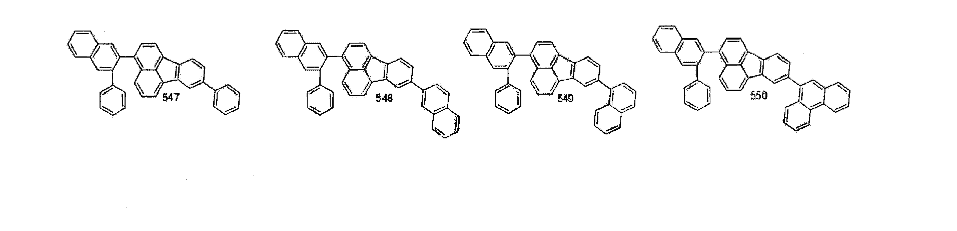

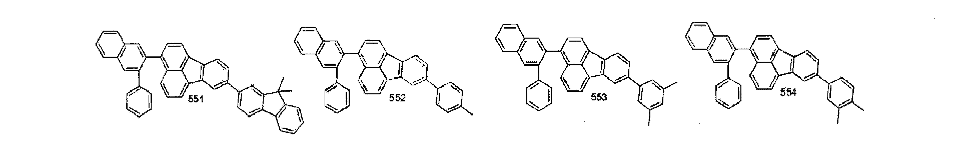

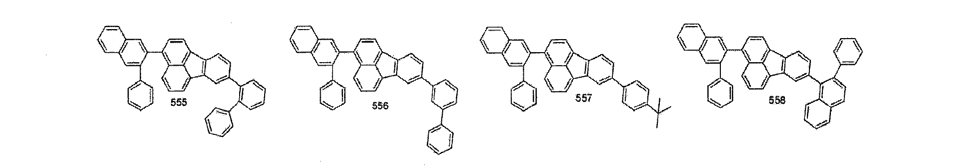

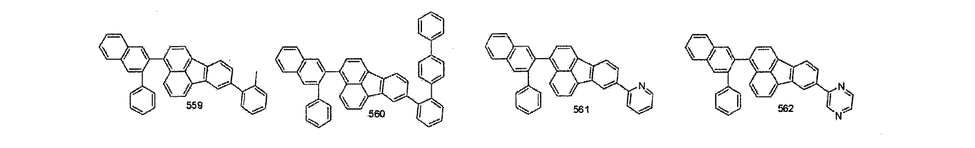

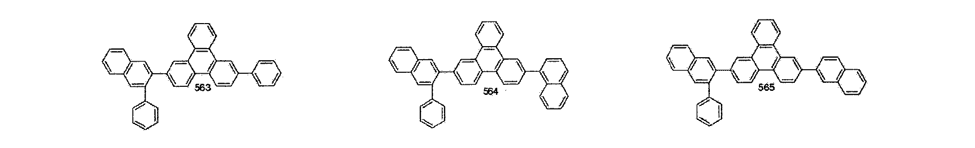

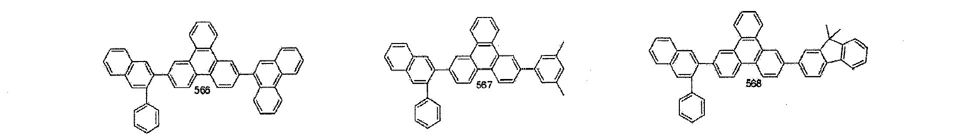

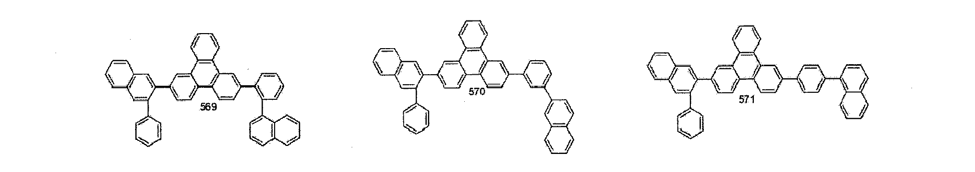

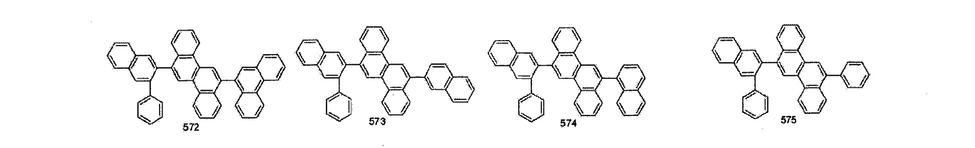

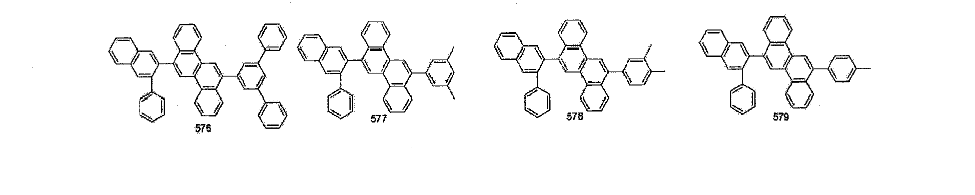

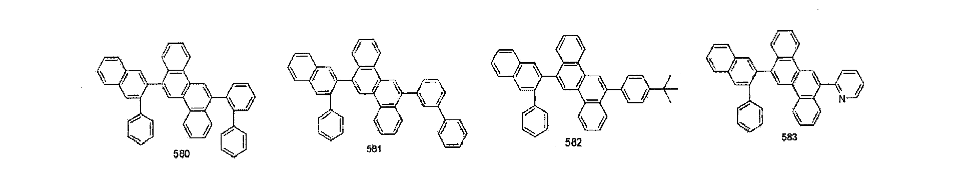

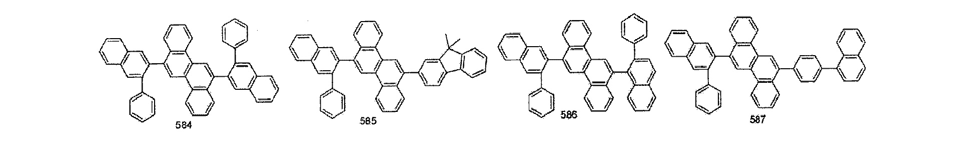

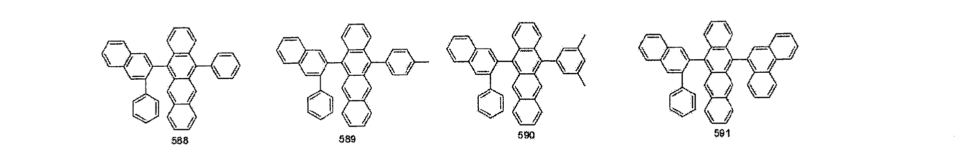

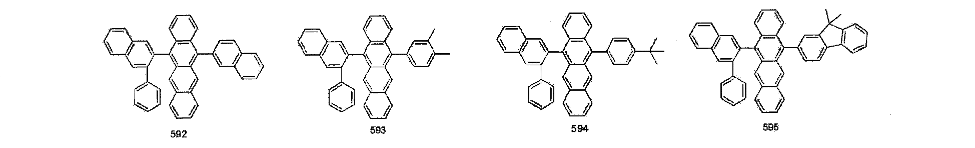

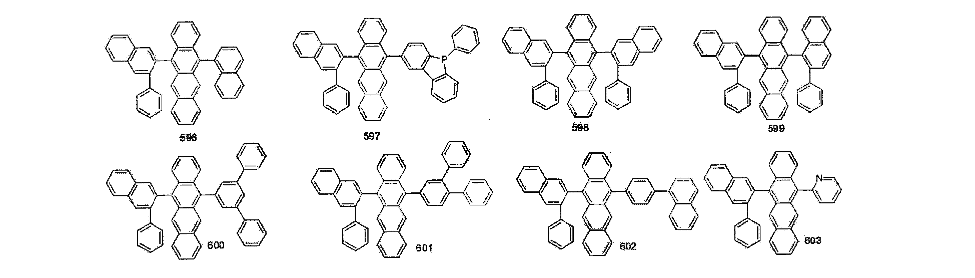

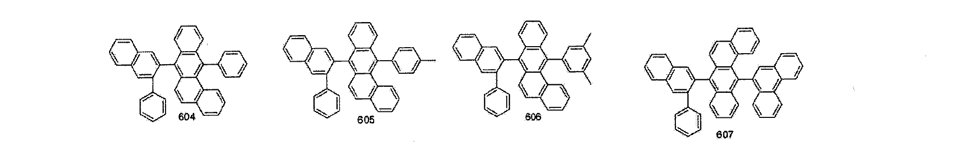

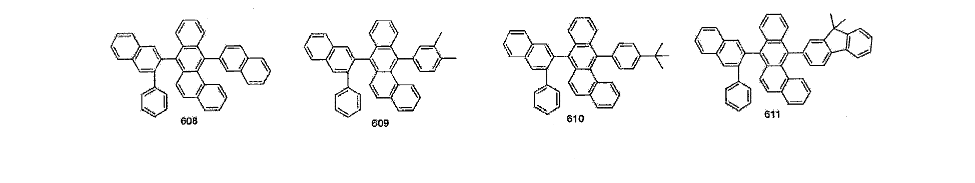

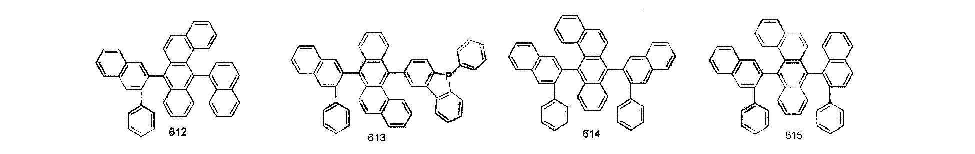

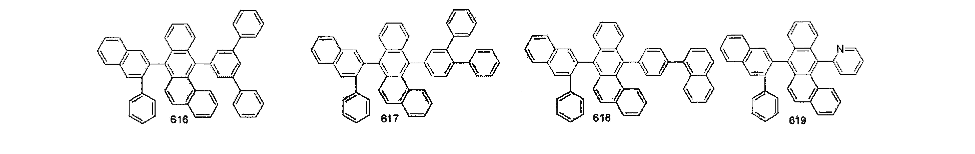

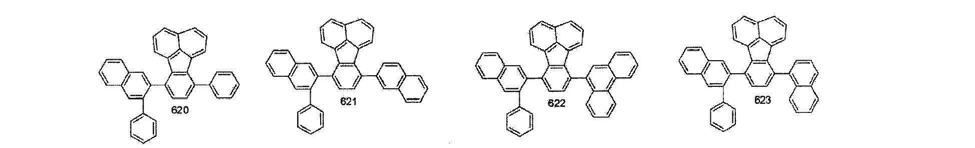

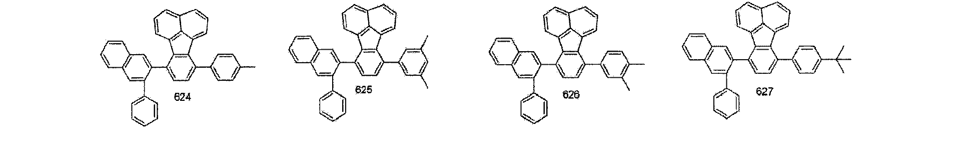

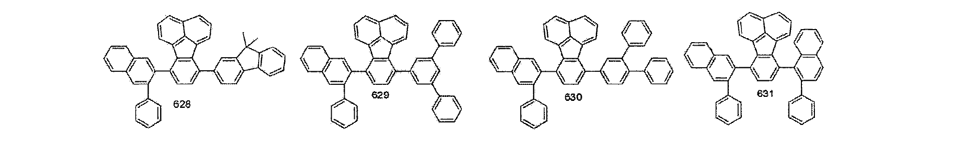

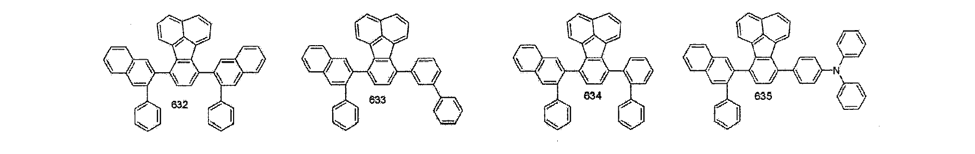

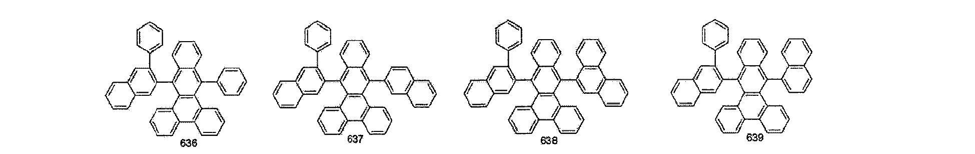

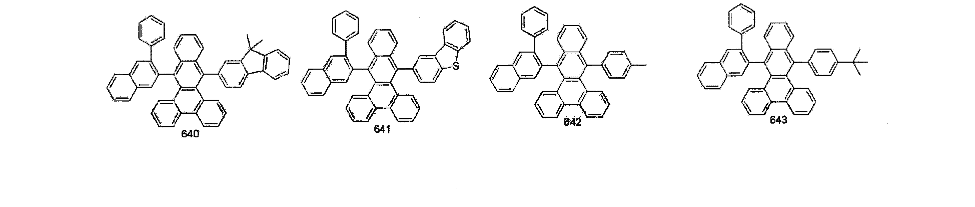

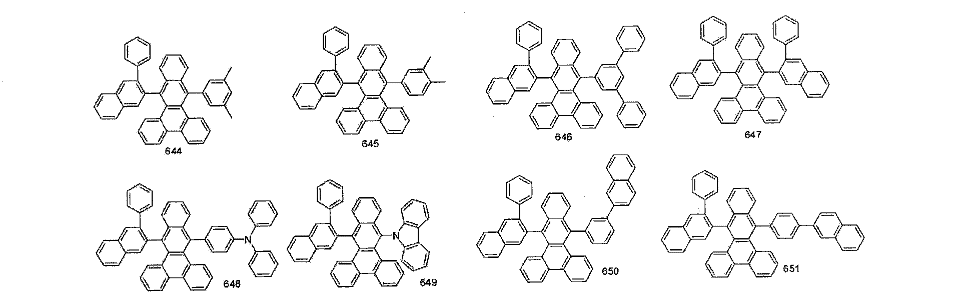

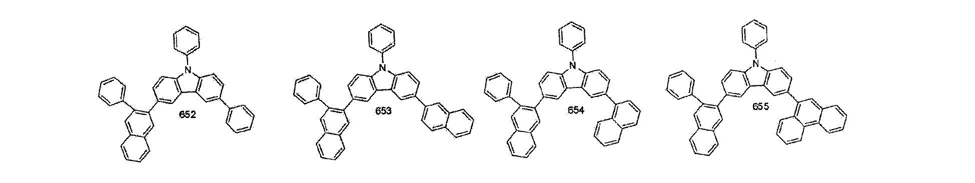

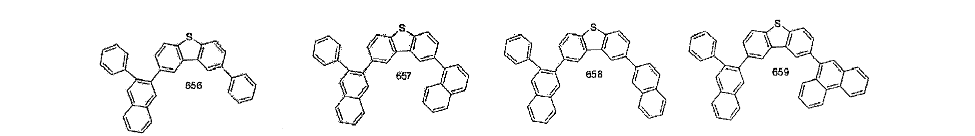

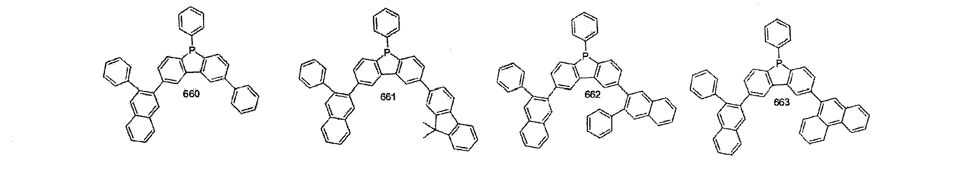

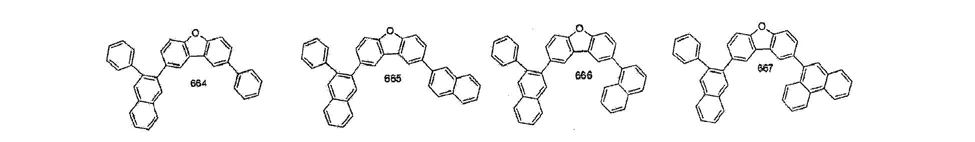

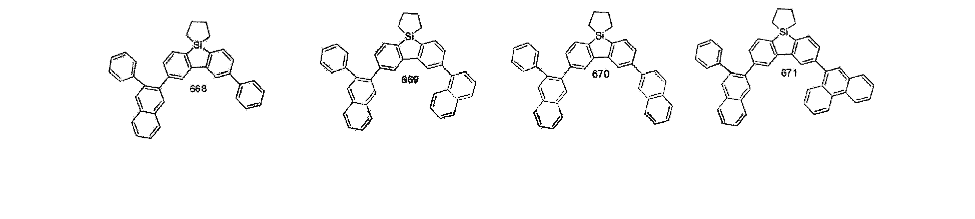

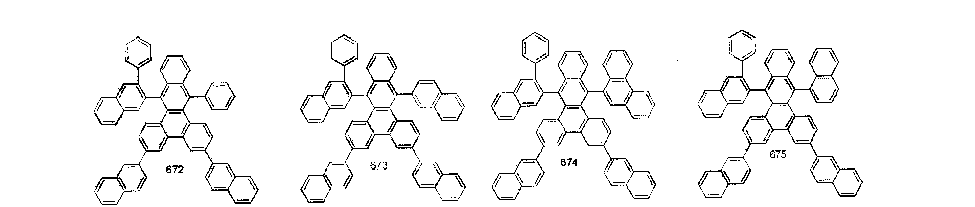

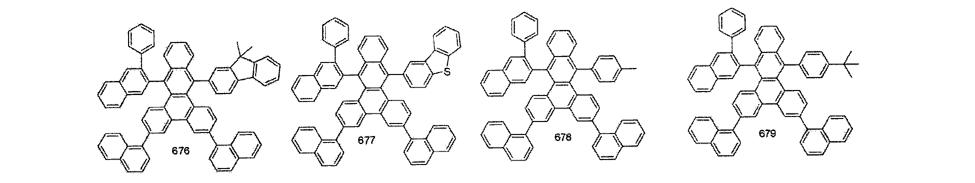

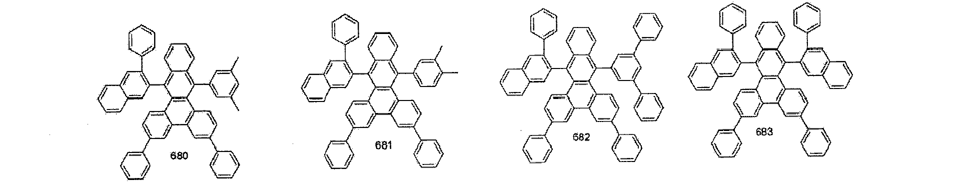

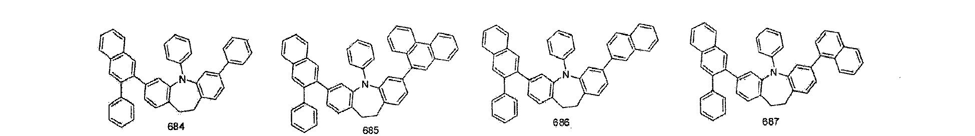

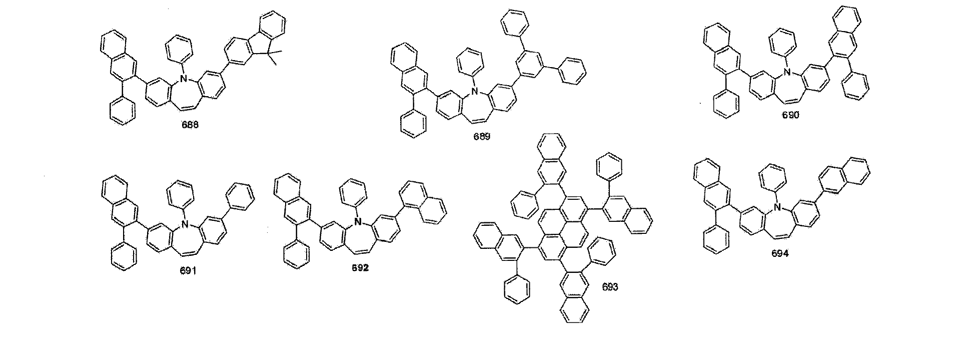

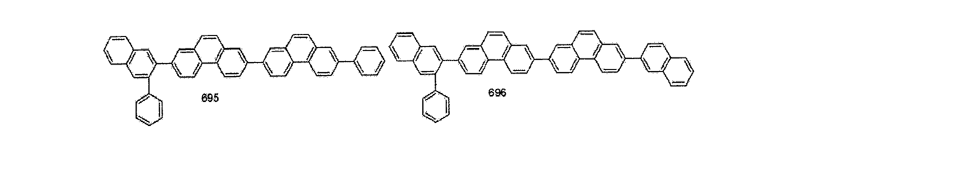

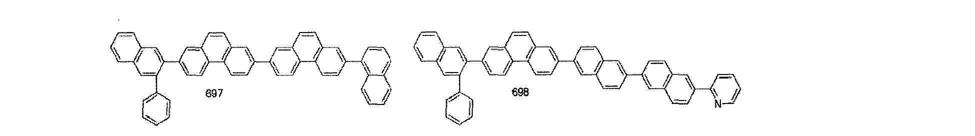

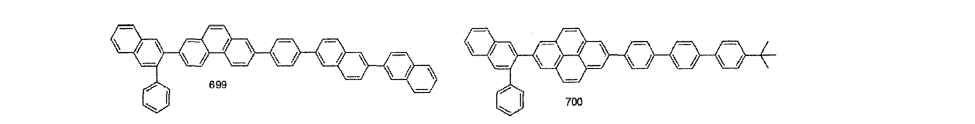

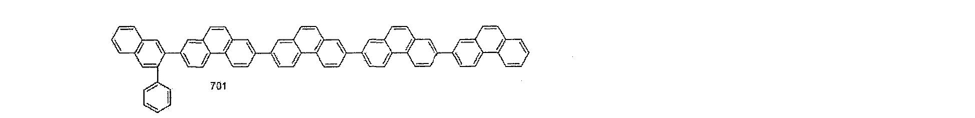

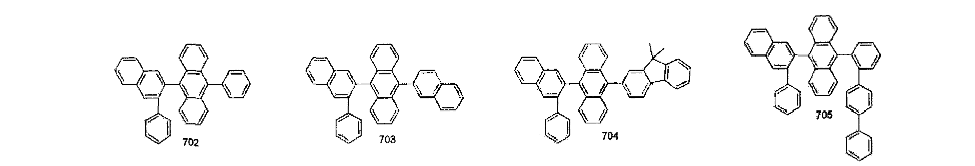

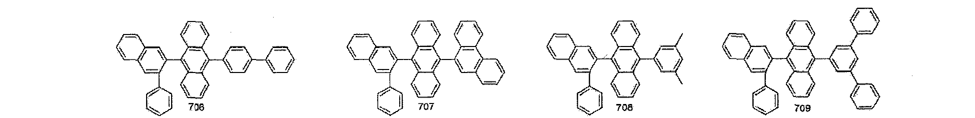

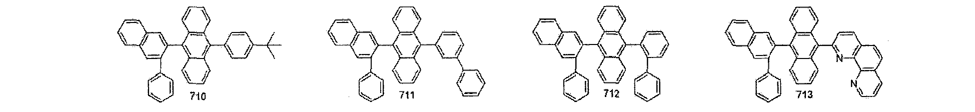

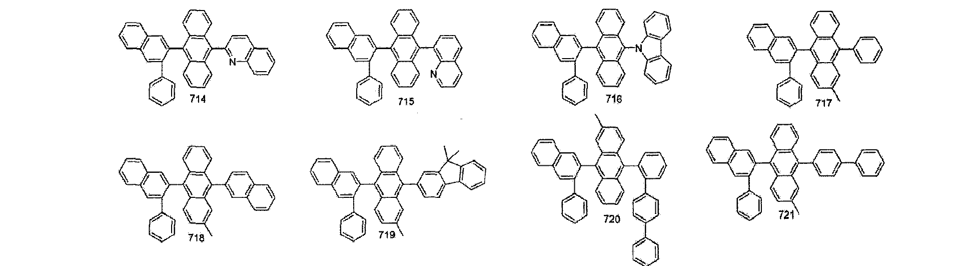

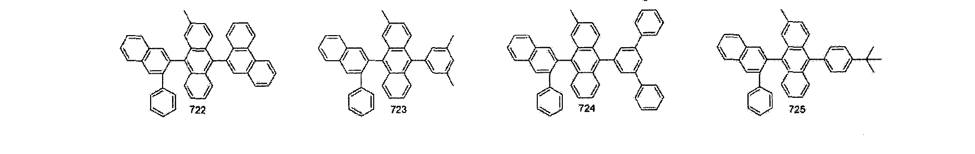

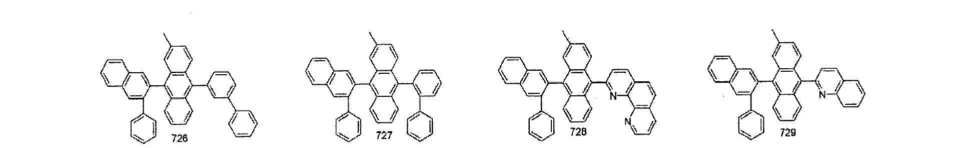

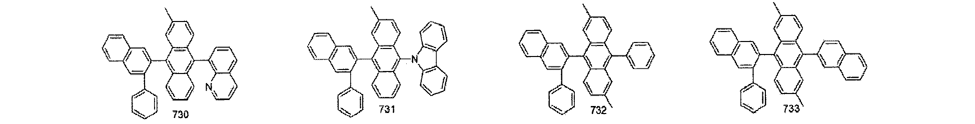

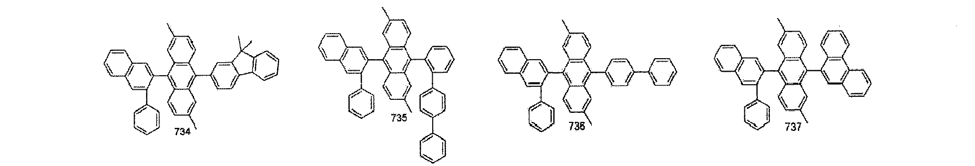

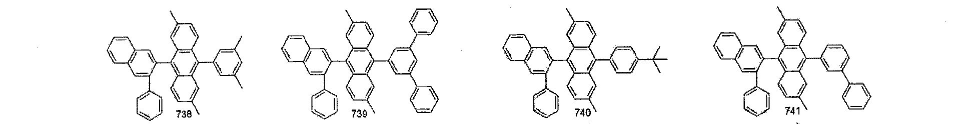

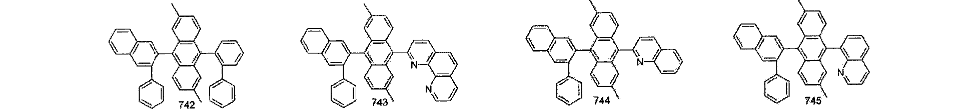

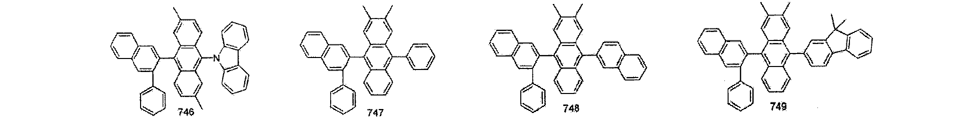

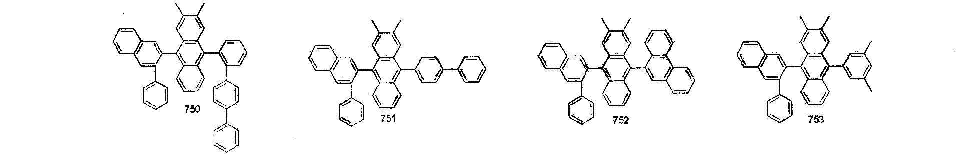

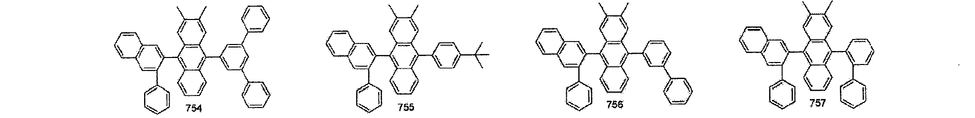

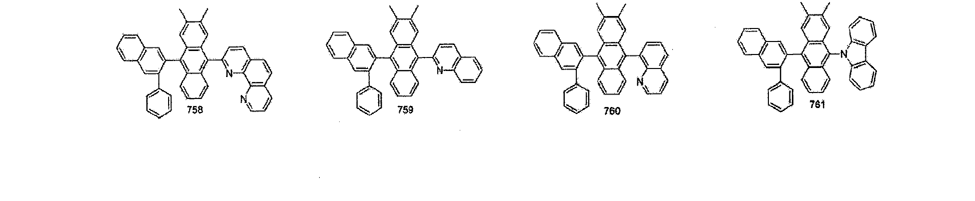

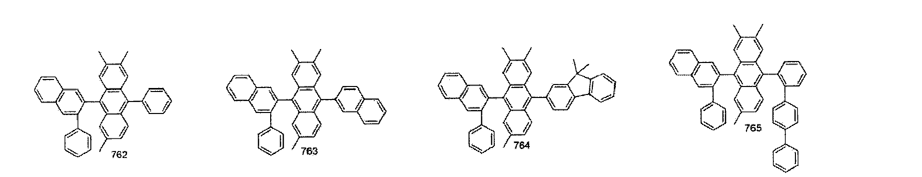

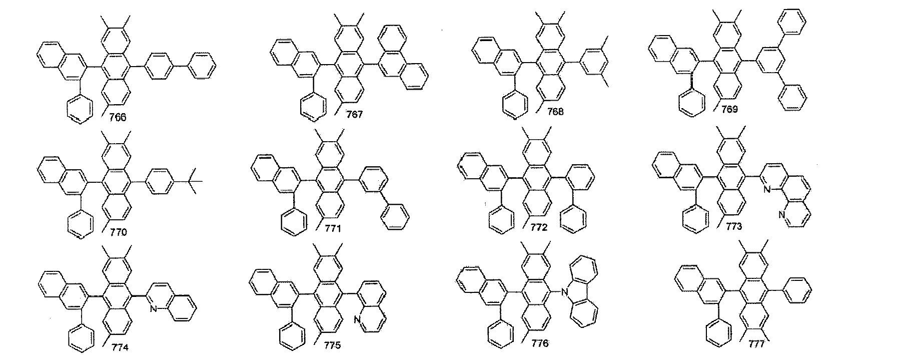

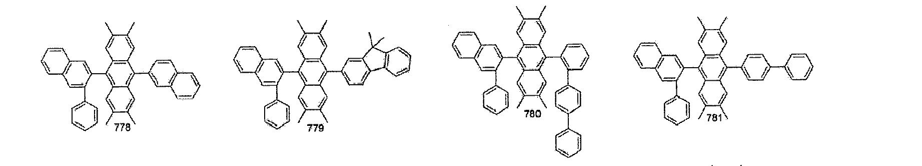

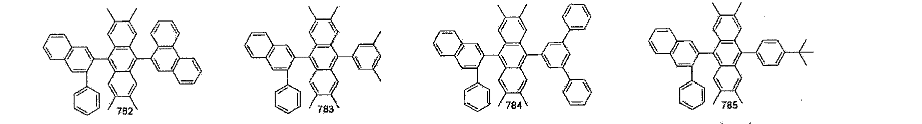

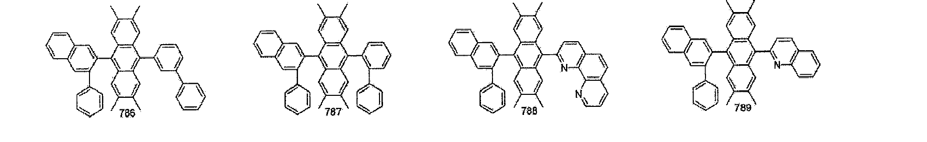

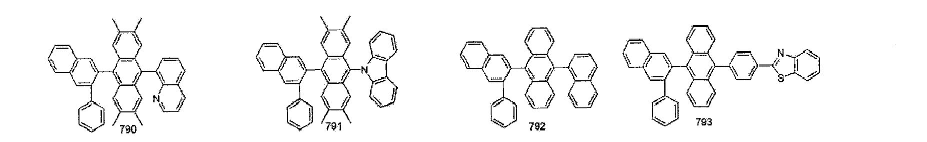

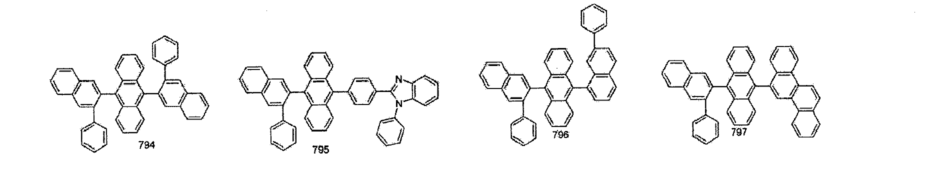

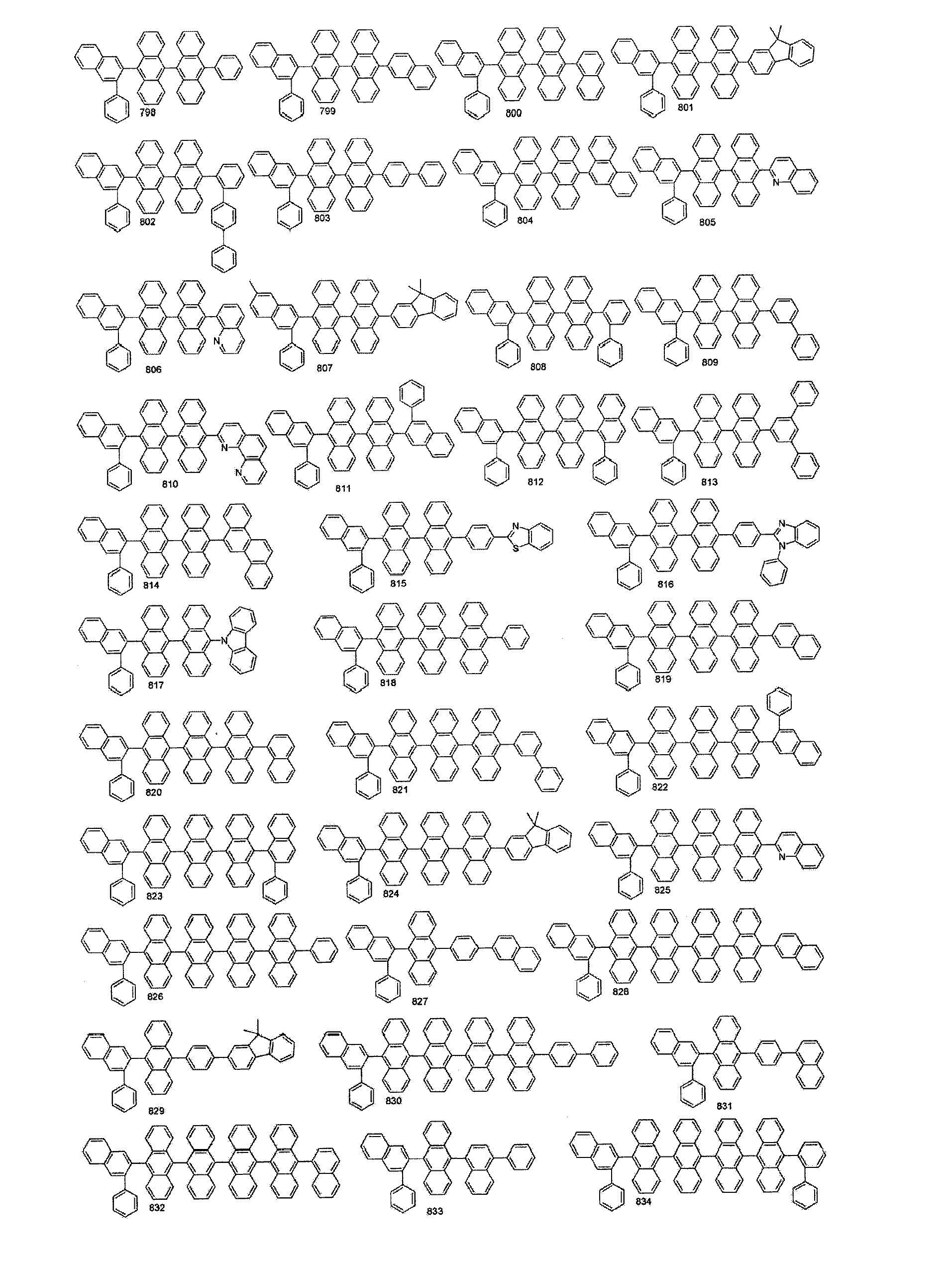

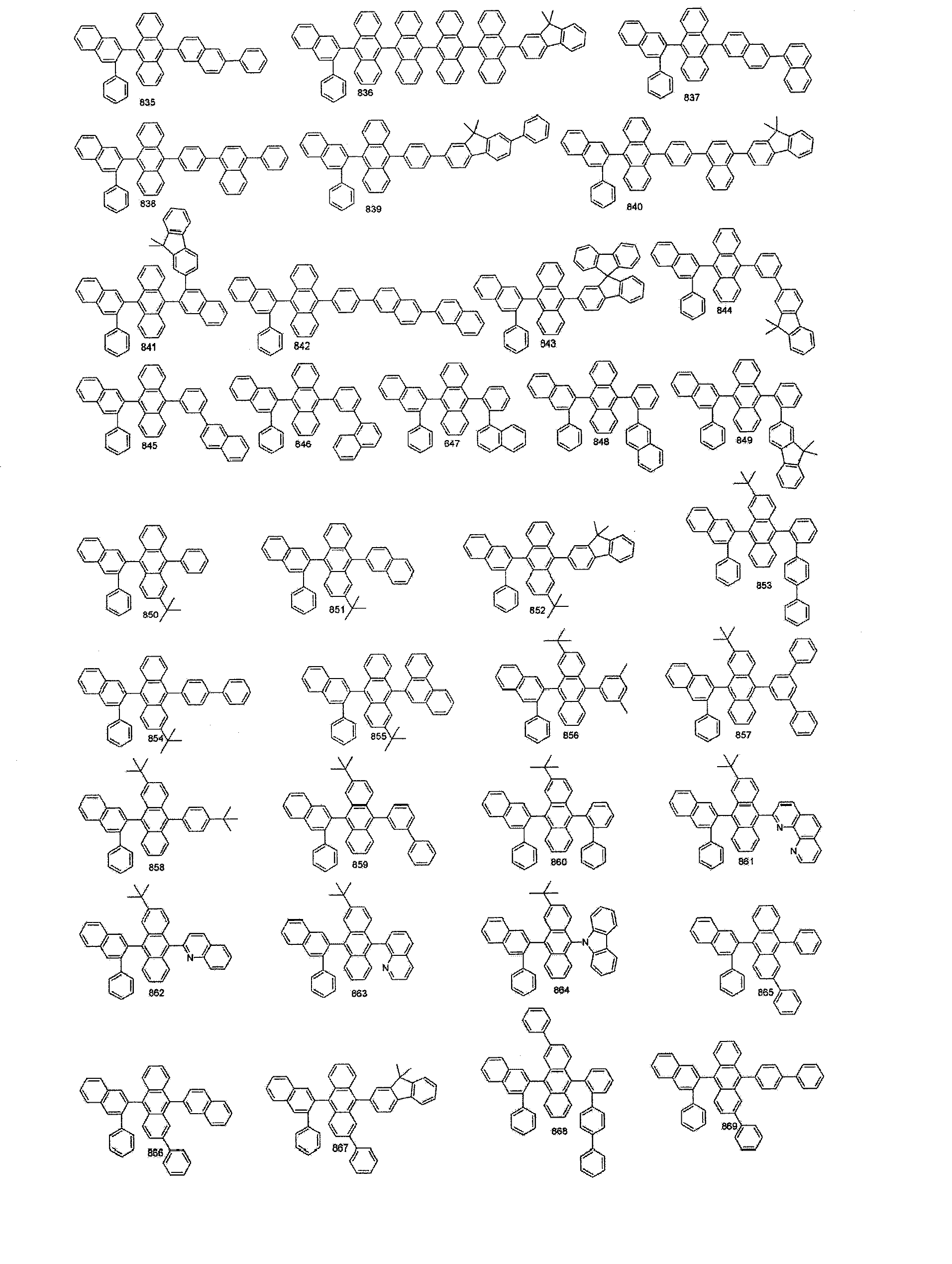

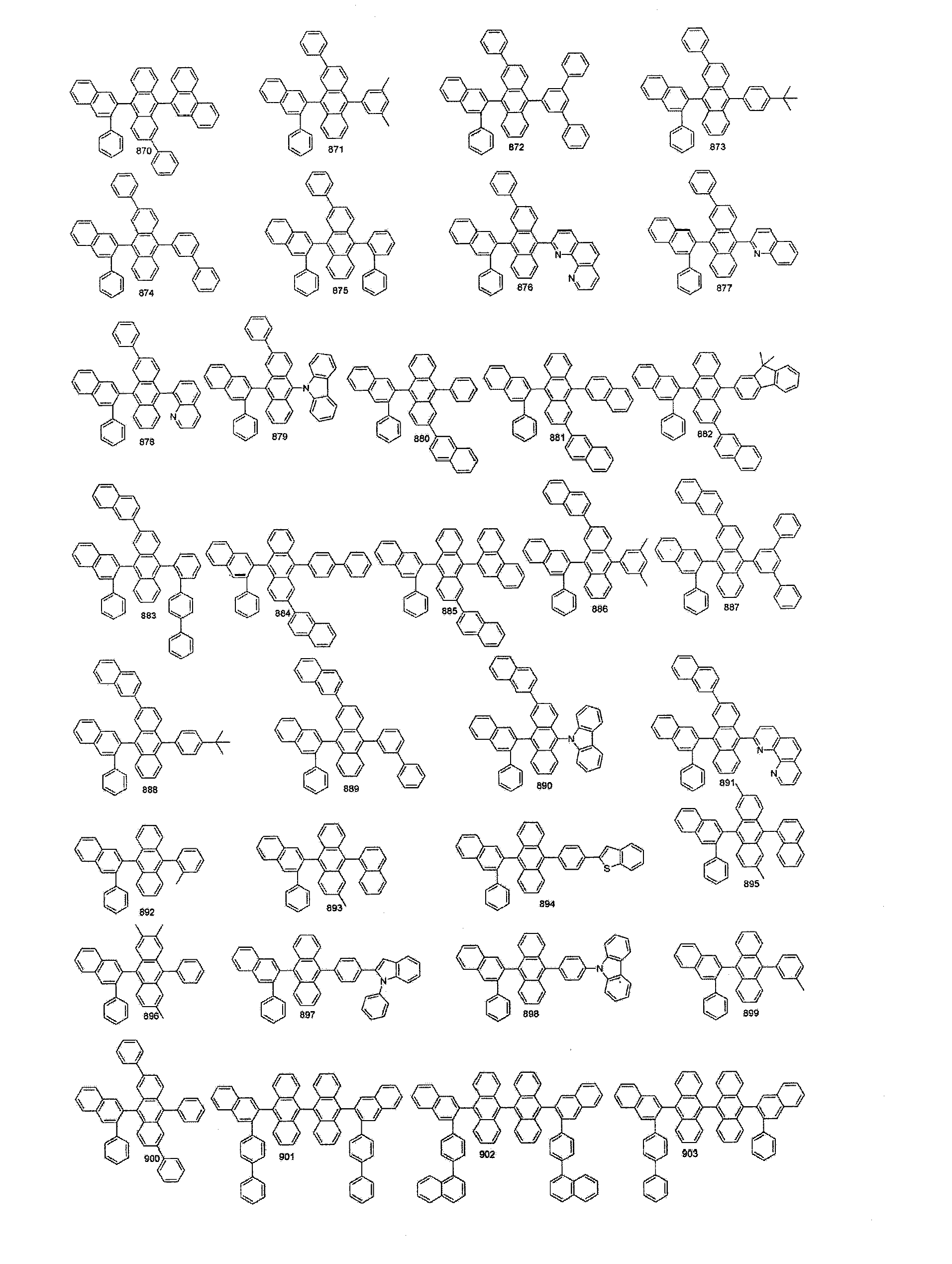

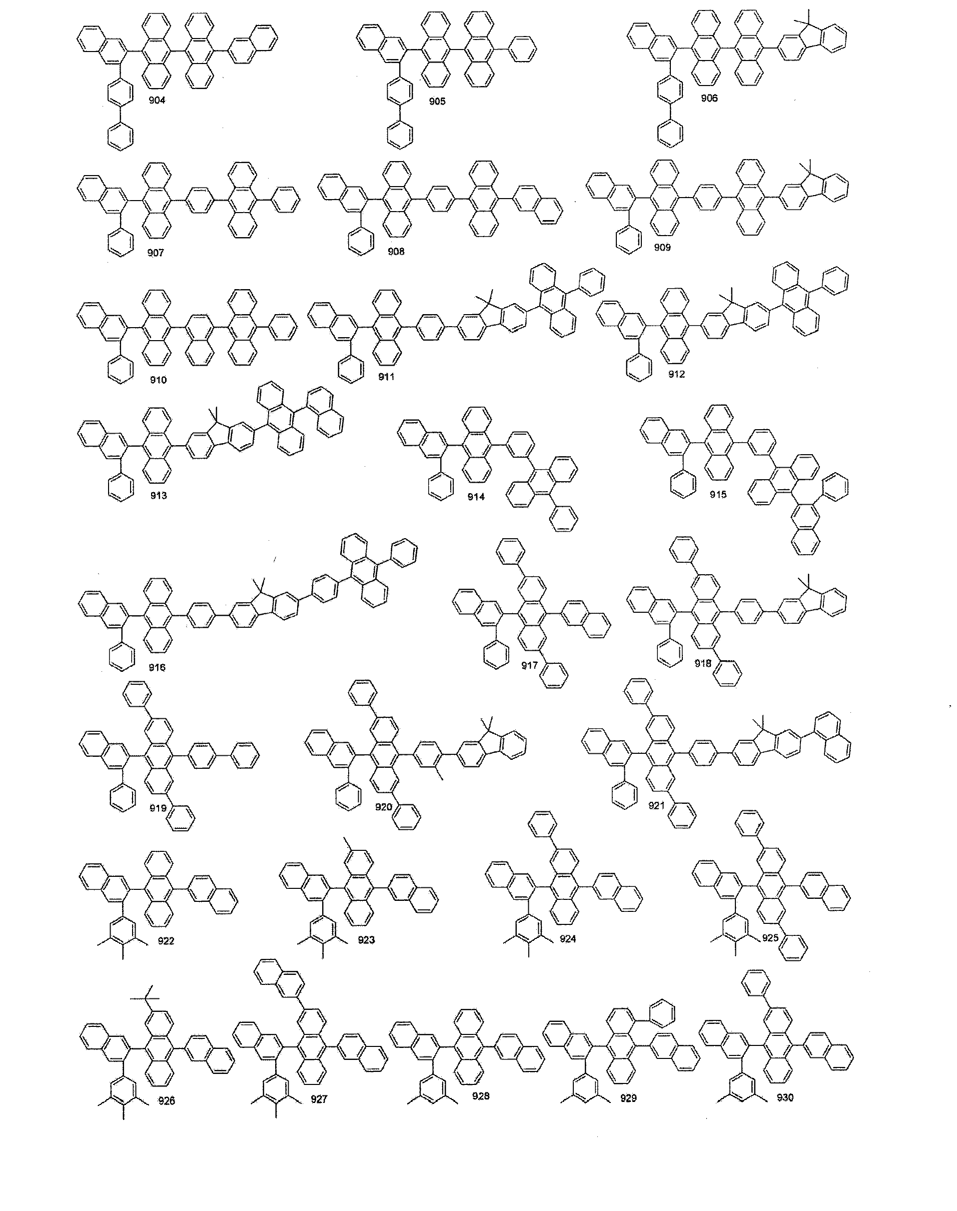

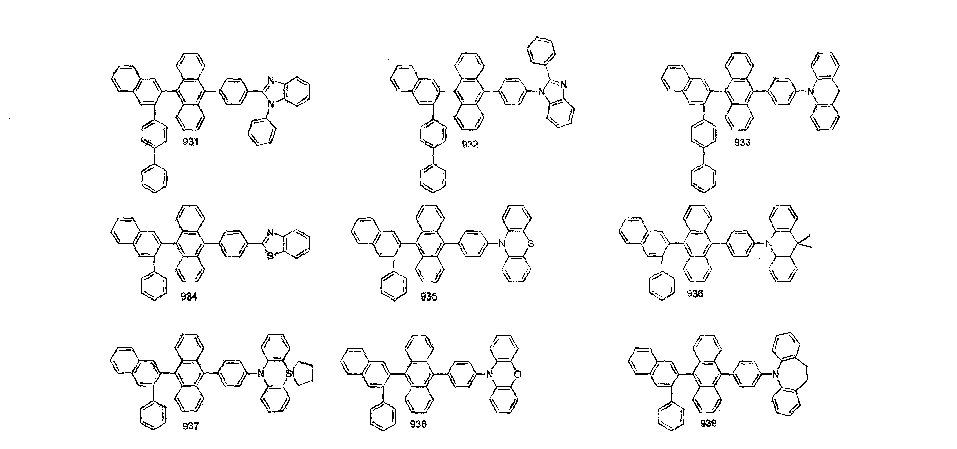

The organic electroluminescent compounds according to the present invention can be prepared as illustrated by Reaction Scheme (1) or (2), which is not restrictive. 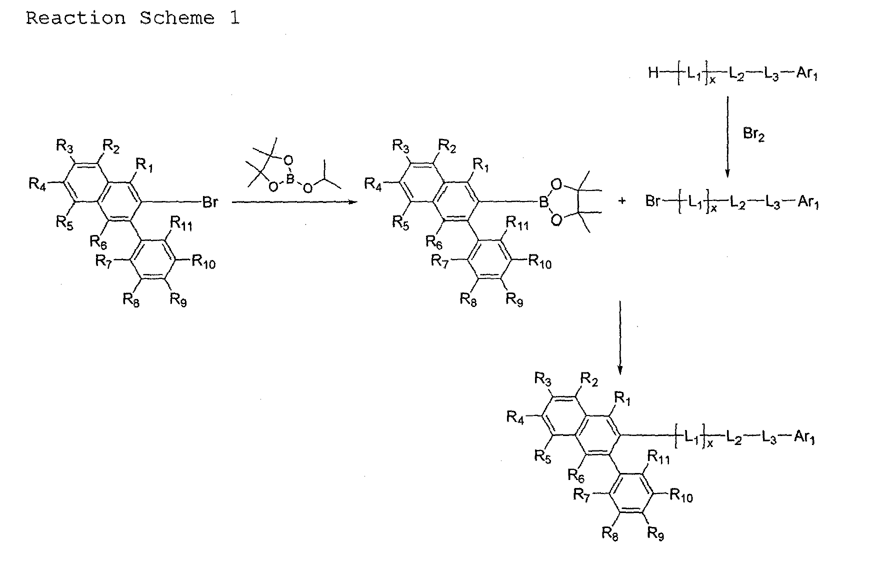 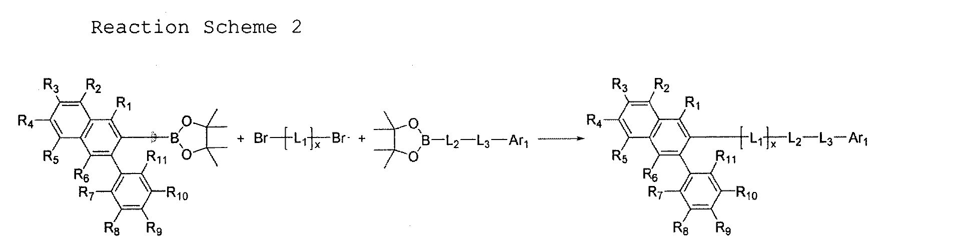 wherein, R₁ through R₁₁, L₁, L₂, L₃, Ar₁ and x are defined as in Chemical Formula (1).

The present invention also provides organic solar cells, which comprises one or more organic electroluminescent compound(s) represented by Chemical Formula (1).

The present invention provides an organic electroluminescent device which is comprised of a first electrode; a second electrode; and at least one organic layer(s) interposed between the first electrode and the second electrode; wherein the organic layer comprises one or more organic electroluminescent compound(s) represented by Chemical Formula (1). The organic electroluminescent compound is used as host material for the electroluminescent layer.

Further, the organic layer comprises an electroluminescent layer, which further comprises one or more dopant(s) in addition to one or more organic electroluminescent compound(s) represented by Chemical Formula (1). The dopant employed in an organic electroluminescent device according to the invention is not particularly restricted.

The dopant employed to an organic electroluminescent device according to the invention is preferably selected from the compounds represented by one of Chemical Formulas (2) to (4). 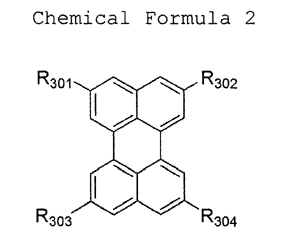 wherein, R₃₀₁ through R₃₀₄ independently represent hydrogen, deuterium, halogen, (C1-C60)alkyl, (C6-C60)aryl, (C4-C60)heteroaryl containing one or more heteroatom(s) selected from N, O and S, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C1-C60)alkylthio, (C6-C60)arylthio, (C1-C60)alkoxycarbonyl, (C1-C60)alkylcarbonyl, (C6-C60)arylcarbonyl, carboxyl, nitro or hydroxyl, or R₃₀₁ through R₃₀₄ may be linked to an adjacent substituent via (C3-C60)alkylene or (C3-C60)alkenylene with or without a fused ring to form an alicyclic ring, or a monocyclic or polycyclic aromatic ring; the alkyl, alkenyl, alkynyl, cycloalkyl, heterocycloalkyl, aryl, heteroaryl, arylsilyl, alkylsilyl, alkyloxy, aryloxy, arylthio, alkylamino, arylamino of R₃₀₁ through R₃₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C600heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C1-C60)alkylthio, (C6-C60)arylthio, (C1-C60)alkoxycarbonyl, (C1-C60)alkylcarbonyl, (C6-C60)arylcarbonyl, carboxyl, nitro and hydroxyl; 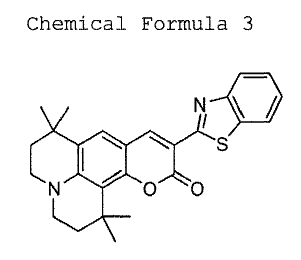 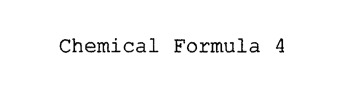 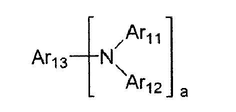 wherein, Ar₁₁ and Ar₁₂ independently represent (C1-C60)alkyl, (C6-C60)aryl, (C4-C60)heteroaryl containing one or more heteroatom(s) selected from N, O and S, (C6-C60)arylamino, (C1-C60)alkylamino, a 5- or 6-membered heterocycloalkyl containing one or more heteroatom(s) selected from N, O and S, or (C3-C60)cycloalkyl, or Ar₁₁ and Ar₁₂ may be linked via (C3-C60)alkylene or (C3-C60)alkenylene with or without a fused ring to form an alicyclic ring or a monocyclic or polycyclic aromatic ring;
when a is 1, Ar₁₃ represents (C6-C60)aryl, (C9-C60)heteroaryl, or a substituent selected from the following structures: 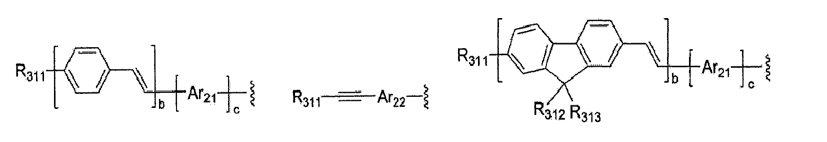 when a is 2, Ar₁₃ represents (C6-C60)arylene, (C4-C60)heteroarylene containing one or more heteroatom(s) selected from N, O and S, or a substituent selected from the following structures: 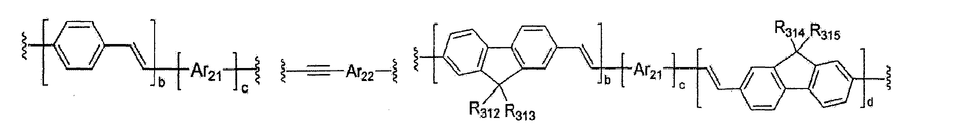 wherein, Ar₂₁ and Ar₂₂ independently represent (C6-C60)arylene or (C4-C60)heteroarylene containing one or more heteroatom(s) selected from N, O and S;
R₃₁₁ through R₃₁₅ independently represent hydrogen, deuterium, (C1-C60)alkyl or (C6-C60)aryl;
b is an integer from 1 to 4, c is an integer of 0 or 1, d is an integer of 0 or 1;
and the alkyl, aryl, heteroaryl, arylamino, alkylamino, cycloalkyl or heterocycloalkyl of Ar₁₁ and Ar₁₂, the aryl, heteroaryl, arylene or heteroarylene of Ar₁₃, the arylene or heteroarylene of Ar₂₁ and Ar₂₂, or the alkyl or aryl of R₃₁₁ through R₃₁₅ may be further substituted by one or more substituent(s) selected from deuterium, halogen, (C1-C60)alkyl, (C6-C60)aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C1-C60)arylthio, (C6-C60)alkylthio, (C1-C60)alkoxycarbonyl, (C1-C60)alkylcarbonyl, (C6-C60)arylcarbonyl, carboxyl, nitro and hydroxyl.

The dopant compounds represented by one of Chemical Formulas (2) to (4) may be specifically exemplified by the compounds with one of the following structures, but they are not restricted thereto.

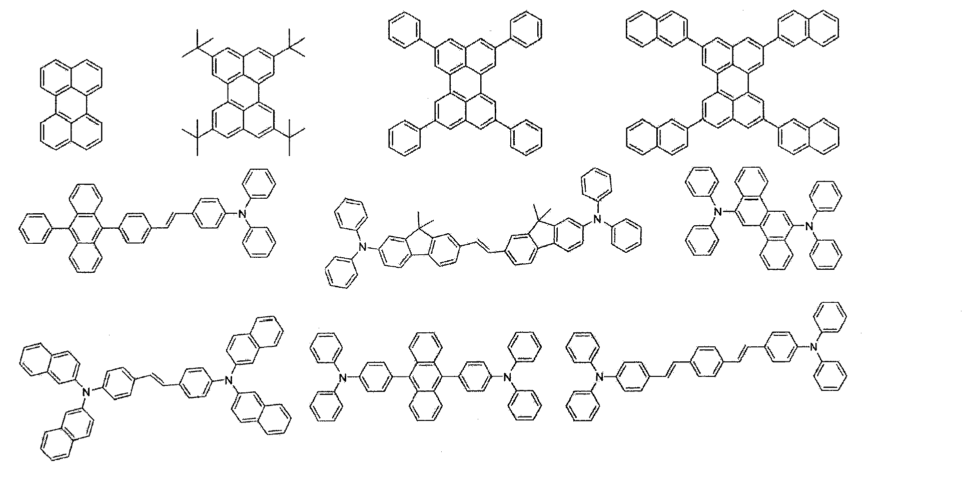

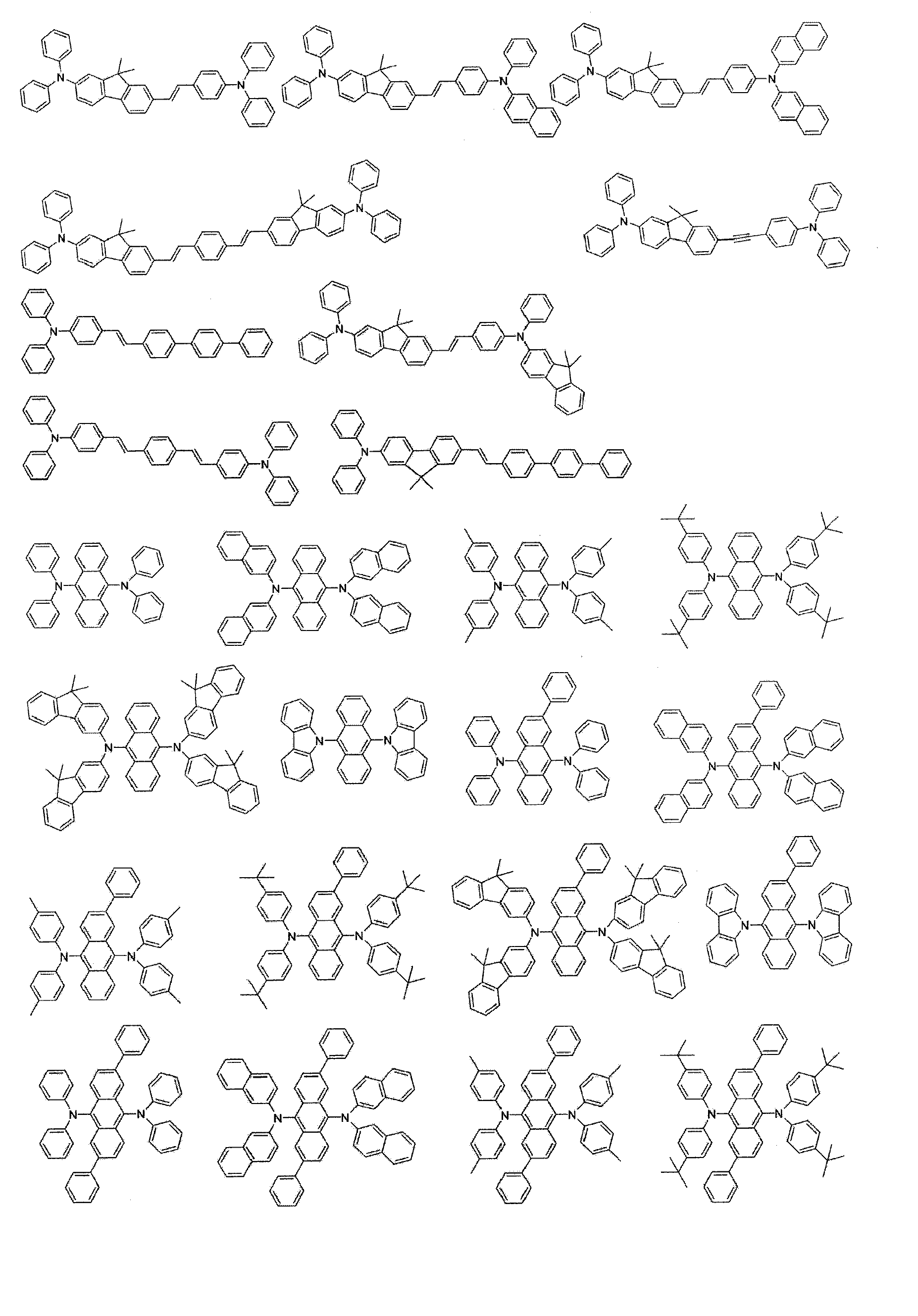

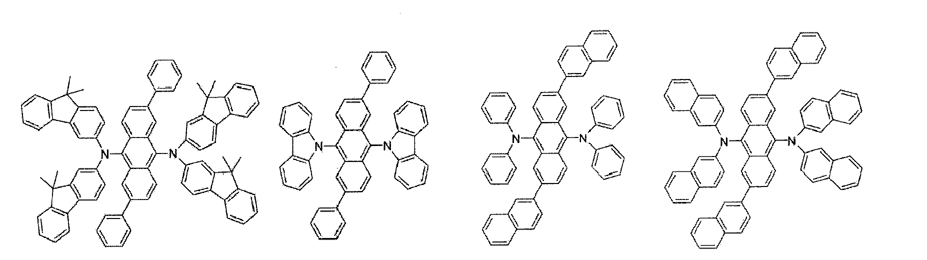

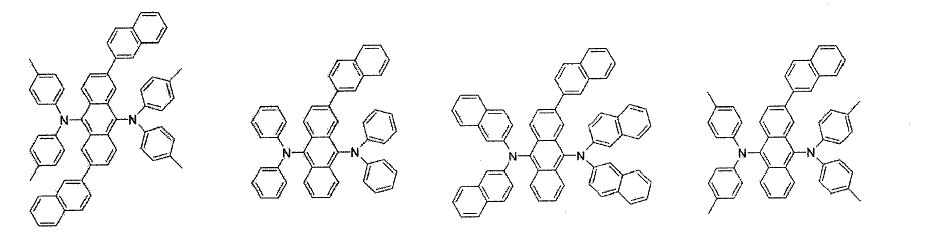

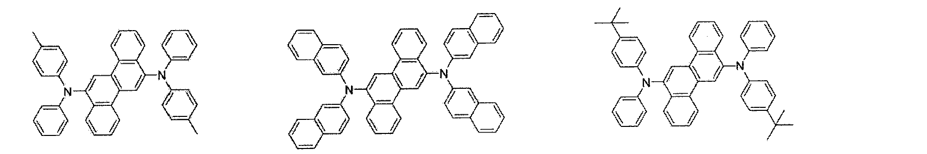

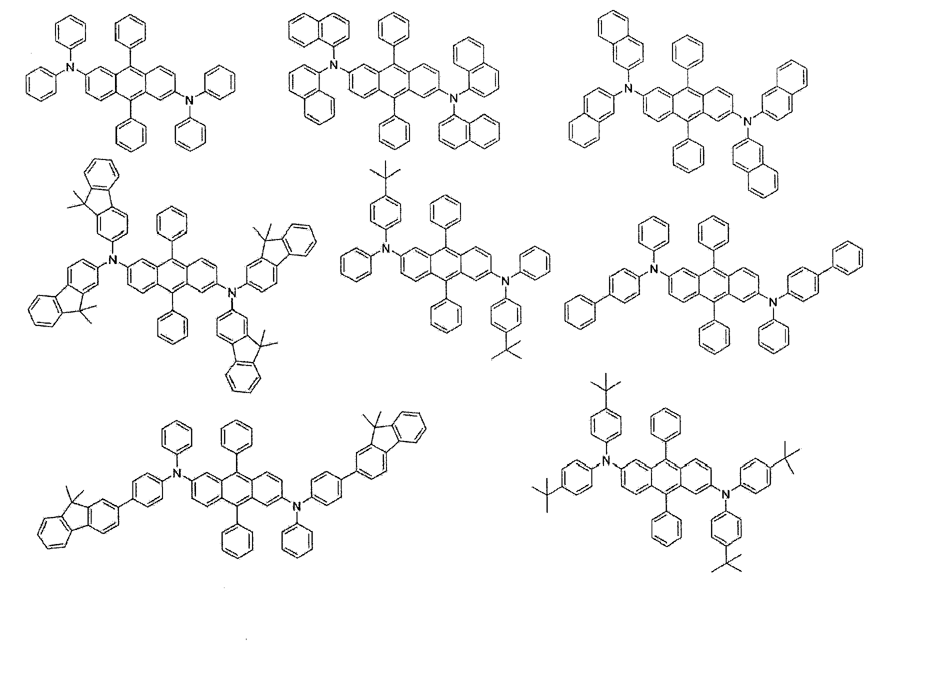

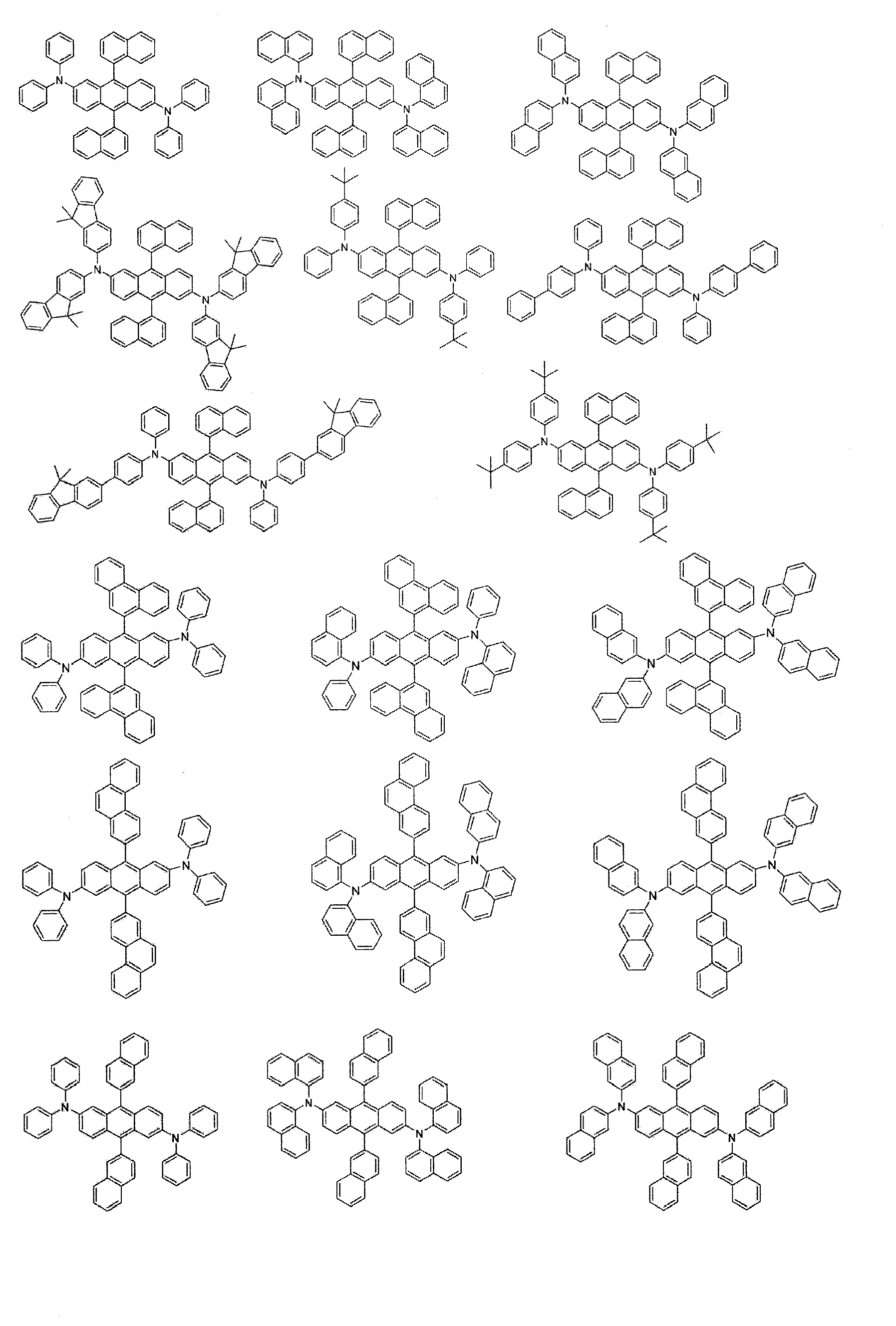

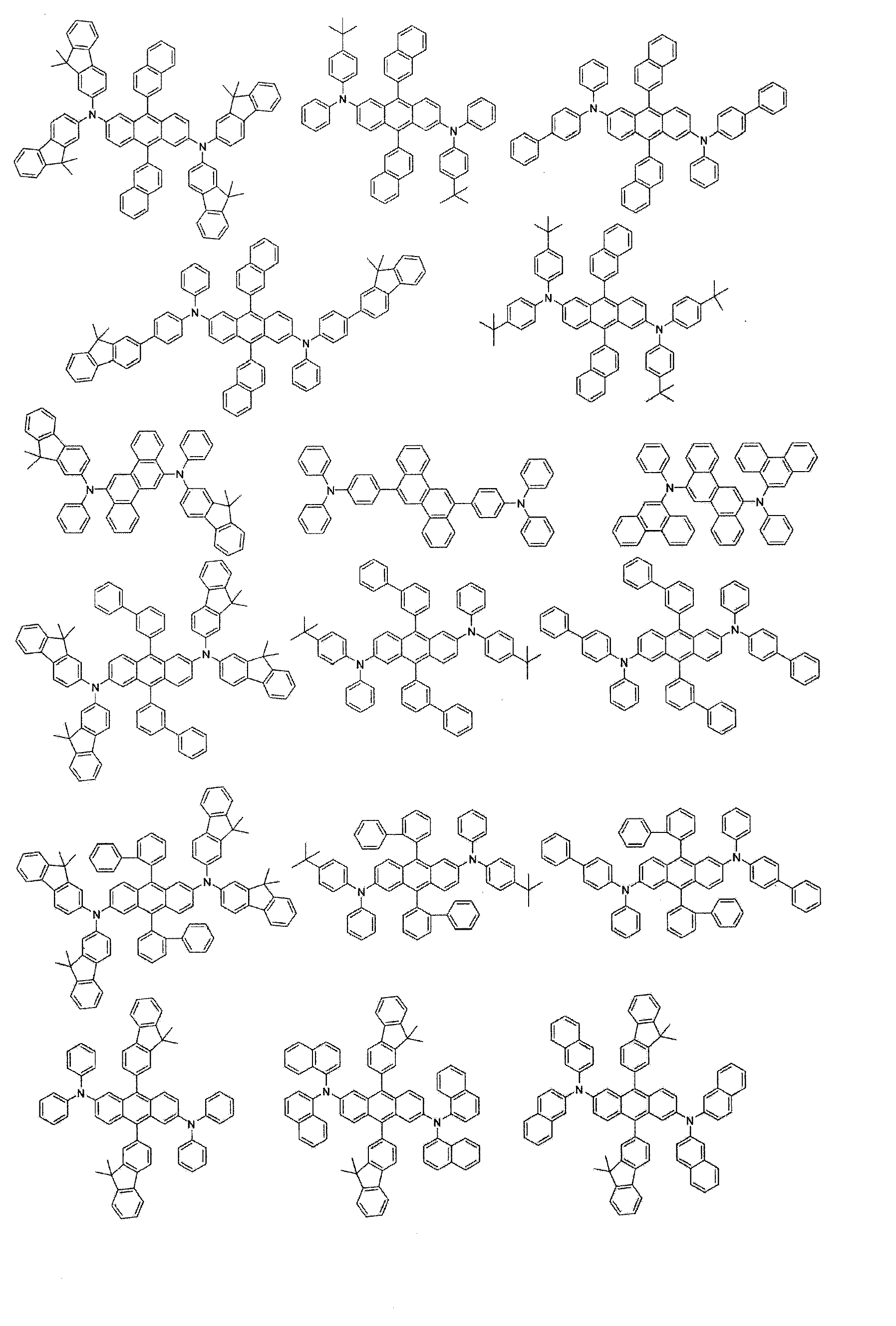

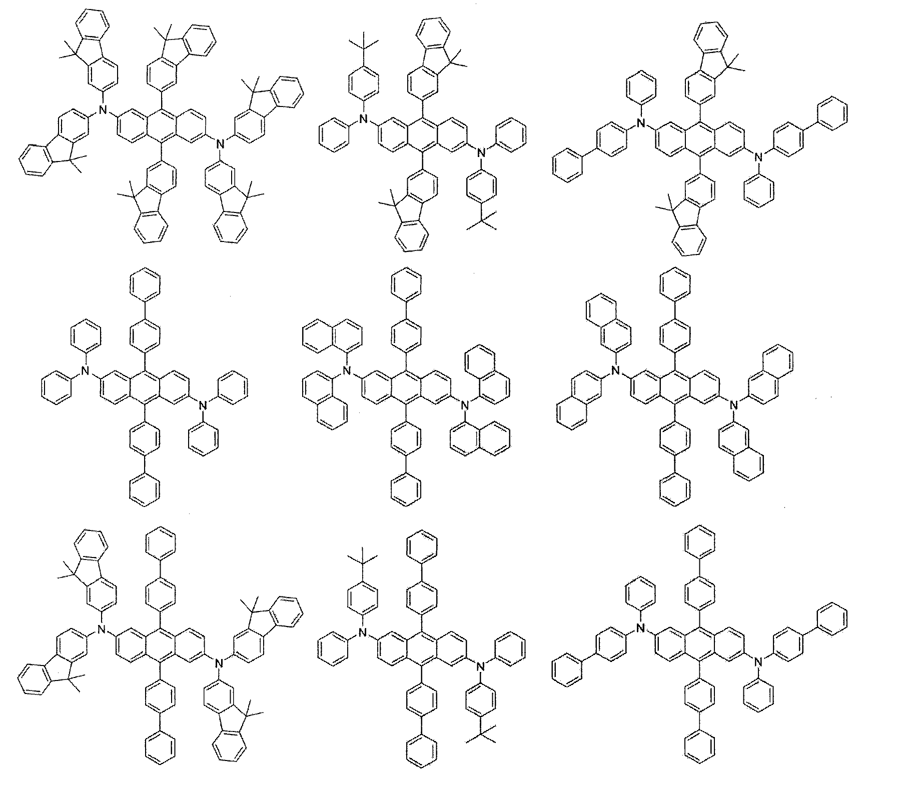

The electroluminescent layer means the layer where electroluminescence occurs, and it may be a single layer or a multilayer consisting of two or more layers laminated. When a mixture of host-dopant is used according to the construction of the present invention, noticeable improvement in luminous efficiency due to the inventive electroluminescent host could be confirmed. This can be achieved by the doping concentration of 0.5 to 10% by weight. The host according to the present invention exhibits higher hole and electron conductivity, and excellent stability of the material as compared to other conventional host materials, and provides improved device life as well as luminous efficiency.

The organic electroluminescent device according to the invention may further comprise one or more compound(s) selected from arylamine compounds and styrylarylamine compounds, as well as the organic electroluminescent compound represented by Chemical Formula (1). Examples of arylamine or styrylarylamine compounds include the compounds represented by Chemical Formula (5), but they are not restricted thereto: 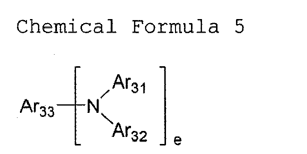 wherein, Ar₃₁ and Ar₃₂ independently represent (C1-C60)alkyl, (C6-C60)aryl, (C4-C60)heteroaryl, (C6-C60)arylamino, (C1-C60)alkylamino, a 5- or 6-membered heterocycloalkyl containing one or more heteroatom(s) selected from N, O and S, or (C3-C60)cycloalkyl, or Ar₃₁ and Ar₃₂ may be linked via (C3-C60)alkylene or (C3-C60)alkenylene with or without a fused ring to form an alicyclic ring, or a monocyclic or polycyclic aromatic ring; when e is 1, Ar₃₃ represents (C6-C60)aryl, (C4-C60)heteroaryl, or a substituent selected from the following structures: 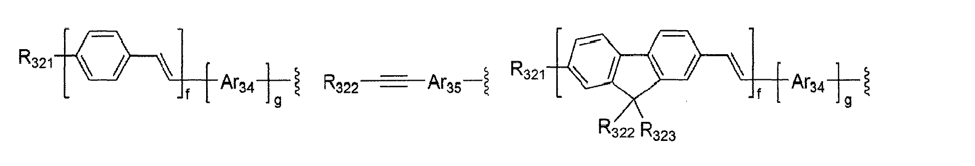 when e is 2, Ar₃₃ represents (C6-C60)arylene, (C4-C60)heteroarylene, or a substituent selected from the following structures: 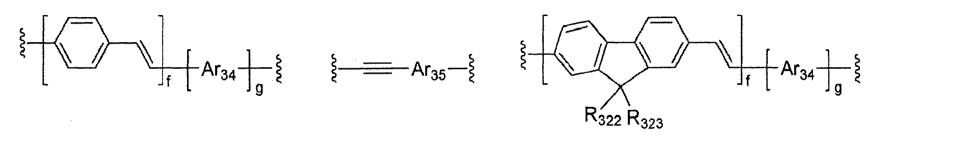 wherein Ar₃₄ and Ar₃₅ independently represent (C6-C60)arylene or (C4-C60)heteroarylene;
R₃₂₁, R₃₂₂ and R₃₂₃ independently represent hydrogen, (C1-C60)alkyl or (C6-C60)aryl;
f is an integer from 1 to 4, g is an integer of 0 or 1; and
the alkyl, aryl, heteroaryl, arylamino, alkylamino, cycloalkyl or heterocycloalkyl of Ar₃₁ and Ar₃₂, or the aryl, heteroaryl, arylene or heteroarylene of Ar₃₃, or the arylene or heteroarylene of Ar₃₄ and Ar₃₅, or the alkyl or aryl of R₃₂₁ through R₃₂₃ may be further substituted by one or more substituent(s) selected from a group consisting of deuterium, halogen, (C1-C60)alkyl, (C6-C60)aryl, (C4-C60)heteraaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

The arylamine compounds and styrylarylamine compounds may be more specifically exemplified by the following compounds, but are not restricted thereto.

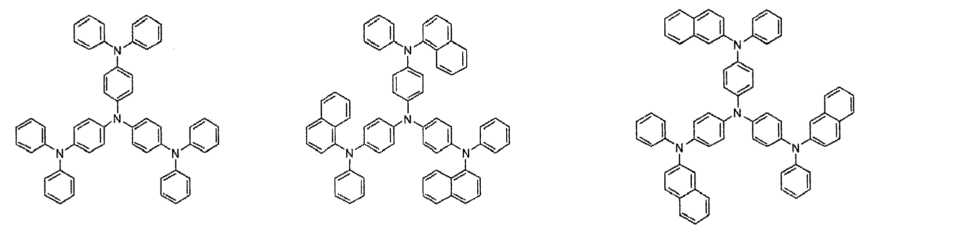

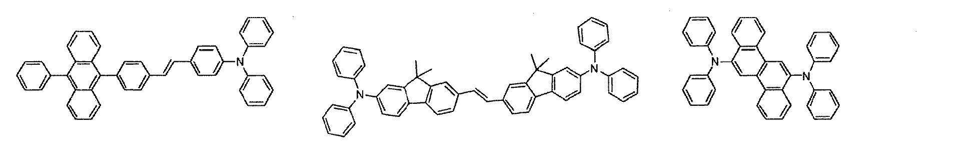

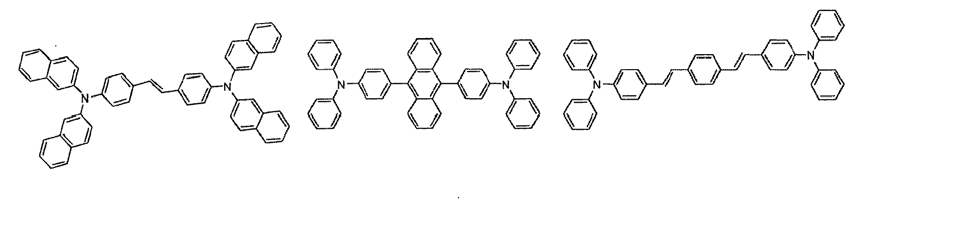

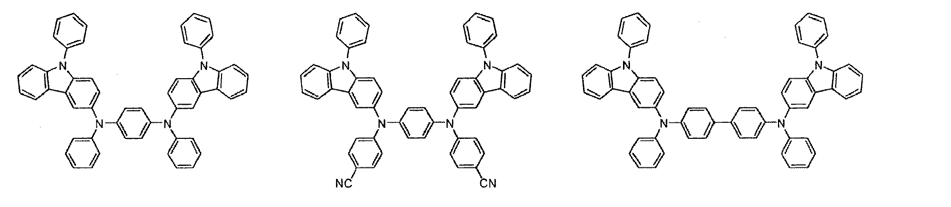

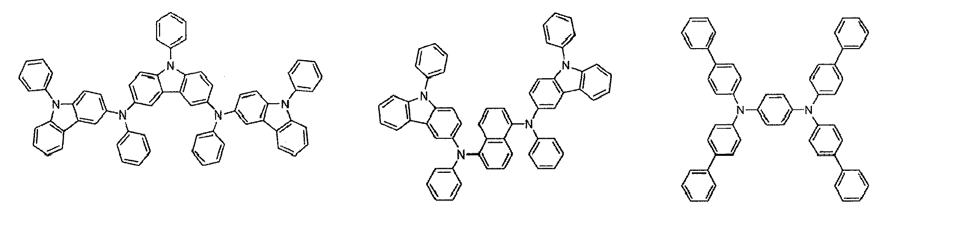

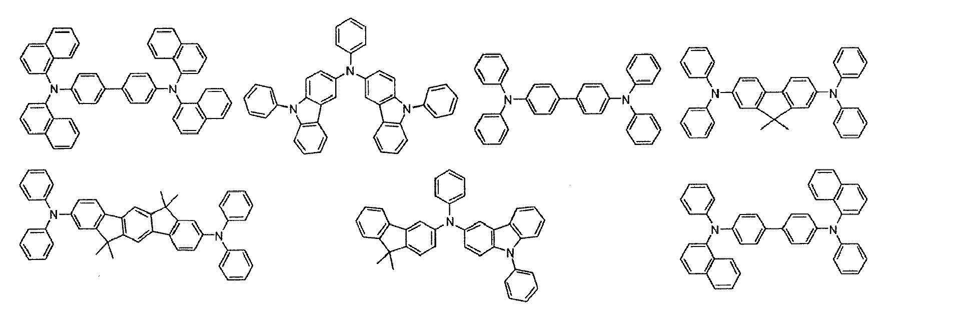

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements, as well as the organic electroluminescent compound represented by Chemical Formula (1). The organic layer may comprise a charge generating layer in addition to the electroluminescent layer.

The present invention can realize an electroluminescent device having a pixel structure of independent light-emitting mode, which comprises an organic electroluminescent device containing the compound of Chemical Formula (1) as a sub-pixel and one or more sub-pixel(s) comprising one or more compound(s) selected from a group consisting of arylamine compounds and styrylarylamine compounds, patterned in parallel at the same time.

Further, the organic electroluminescent device according to the invention is an organic display further comprising a compound having the electroluminescent peak of wavelength of not less than 560 nm in the organic layer. The compounds having the EL peak of wavelength of not less than 560 nm can be exemplified by the compounds represented by one of Chemical Formulas (6) to (10), but they are not restricted thereto. Chemical Formula 6

M¹L¹⁰¹L¹⁰²L¹⁰³

In Chemical Formula (6), M¹ is selected from Group 7, 8, 9, 10, 11, 13, 14, 15 and 16 metals in the Periodic Table of Elements, and ligands L¹⁰¹, L¹⁰² and L¹⁰³ are independently selected from the following structures: 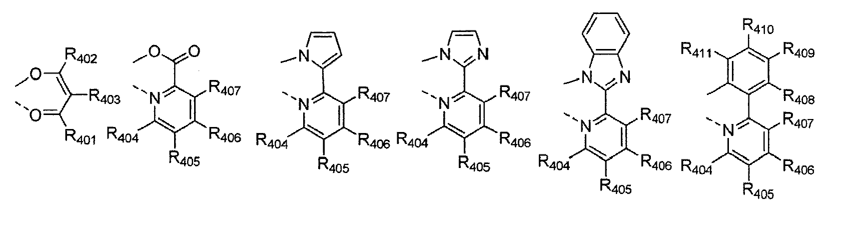 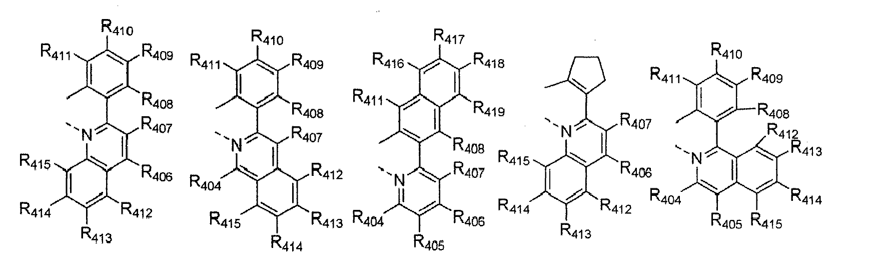 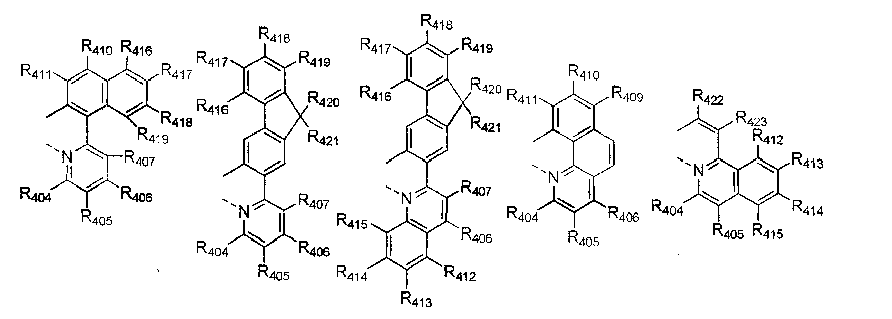 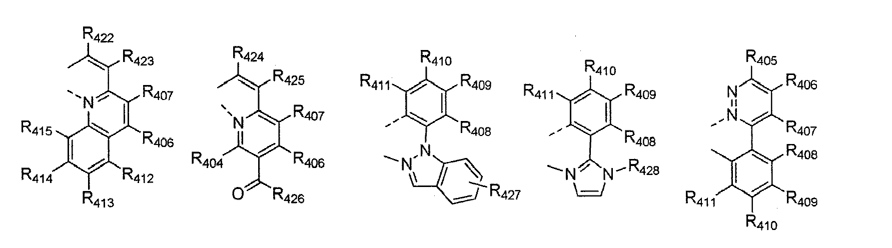 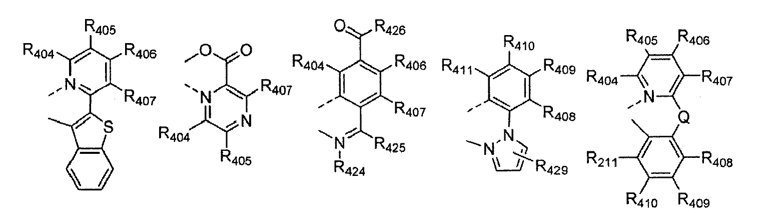 wherein, R₄₀₁ through R₄₀₃ independently represent hydrogen, deuterium, (C1-C60)alkyl with or without halogen substituent(s), (C6-C60)aryl with or without (C1-C60)alkyl substituent(s), or halogen;
R₄₀₄ through R₄₁₉ independently represent hydrogen, deuterium, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(Cl-C30)alkylsilyl, di(C1-C30)alkyl(C6-C30)arylsilyl, tri(C6-C30)arylsilyl, cyano or halogen, and the alkyl, cycloalkyl, alkenyl or aryl of R₄₀₄ through R₄₁₉ may be further substituted by one or more substituent(s) selected from deuterium, (C1-C60)alkyl, (C6-C60)aryl and halogen;
R₄₂₀ through R₄₂₃ independently represent hydrogen, deuterium, (C1-C60)alkyl with or without halogen substituent(s), (C6-C60)aryl with or without (C1-C60)alkyl substituent(s);
R₄₂₄ and R₄₂₅ independently represent hydrogen, deuterium, (C1-C60)alkyl, (C6-C60)aryl or halogen, or R₄₂₄ and R₄₂₅ may be linked via (C3-C12)alkylene or (C3-C12)alkenylene with or without a fused ring to form an alicyclic ring, or a monocyclic or polycyclic aromatic ring; and the alkyl or aryl of R₄₂₄ and R₄₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from deuterium, (C1-C60)alkyl with or without halogen substituent(s), (C1-C30)alkoxy, halogen, tri(C1-C30)alkylsil tri(C6-C30)arylsilyl and (C6-C60)aryl;
R₄₂₆ represents (C1-C60)alkyl, (C6-C60)aryl, or (C5-C60)heteroaryl or halogen;
R₄₂₇ through R₄₂₉ independently represent hydrogen, deuterium, (C1-C60)alkyl, (C6-C60)aryl or halogen, and the alkyl or aryl of R₄₂₆ through R₄₂₉ may be further substituted by halogen or (C1-C60)alkyl; Q represents 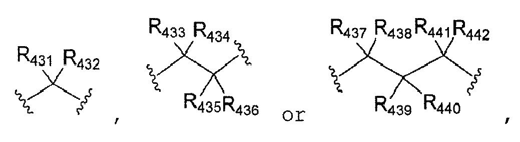 and R₄₃₁ through R₄₄₂ independently represent hydrogen, deuterium, (C1-C60)alkyl with or without halogen substituent(s), (C1-C30)alkoxy, halogen, (C6-C60)aryl, cyano or (C5-C60)cycloalkyl, or each of R₄₃₁ through R₄₄₂ may be linked to an adjacent substituent via alkylene or alkenylene to form a (C5-C7) spiro-ring or (C5-C9) fused ring, or each of them may be linked to R₄₀₇ or R₄₀₈ via alkylene or alkenylene to form a (C5-C7) fused ring.

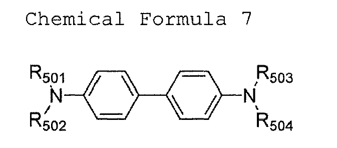

In Chemical Formula (7), R₅₀₁ through R₅₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₅₀₁ through R₅₀₄, or the alicyclic ring, or the monocyclic or polycyclic aromatic ring formed therefrom by linkage via (C3-C60)alkylene or (C3-C60)alkenylene with or without a fused ring may be further substituted by one or more substituent(s) selected from deuterium, (C1-C60)alkyl with or without halogen substituent(s), (C1-C60)alkoxy, halogen, tri(C1-C60)alkylsilyl, tri(C6-C60)arylsilyl and (C6-C60)aryl.

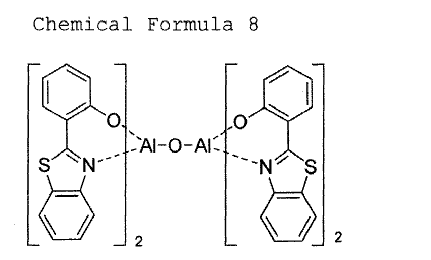

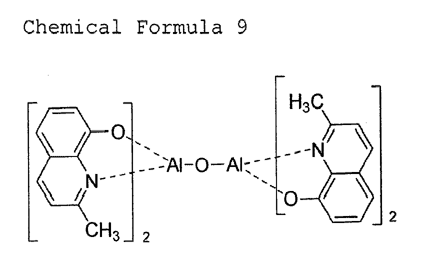

Chemical Formula 10 L²⁰¹L²⁰²M(T)ₕ

In Chemical Formula (10), the ligands, L²⁰¹ and L²⁰² are independently selected from the following structures: 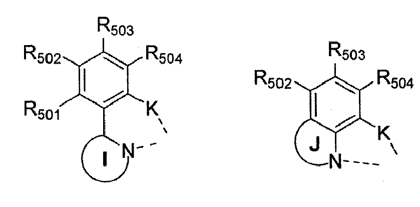 M² is a bivalent or trivalent metal;
h is 0 when M² is a bivalent metal, while h is 1 when M² is a trivalent metal;
T represents (C6-C60)aryloxy or tri(C6-C60)arylsilyl, and the aryloxy and triarylsilyl of T may be further substituted by (C1-C60)alkyl or (C6-C60)aryl;
K represents O, S or Se;
ring I represents oxazole, thiazole, imidazole, oxadiazole, thiadiazole, benzoxazole, benzothiazole, benzimidazole, pyridine or quinoline;
ring J represents pyridine or quinoline, and ring J may be further substituted by (C1-C60)alkyl, or phenyl or naphthyl with or without (C1-C60)alkyl substituent(s);
R₅₀₁ through R₅₀₄ independently represent hydrogen, deuterium, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may form a chemical bond with R₅₀₁ to form a fused ring;
ring I or the aryl group of R₅₀₁ through R₅₀₄ may be further substituted by deuterium, (C1-C60)alkyl, halogen, (C1-C60)alkyl with halogen substituent(s), phenyl, naphthyl, tri(C1-C60)alkylsilyl, tri(C6-C60)arylsilyl or amino group.

The compounds having the electroluminescent peak of the wavelength of not less than 560 nm can be exemplified by the following compounds, but they are not restricted thereto.

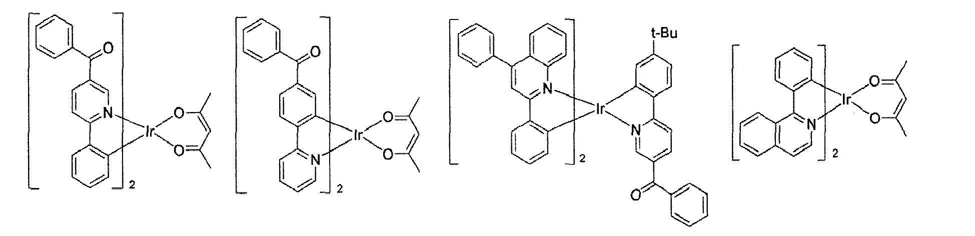

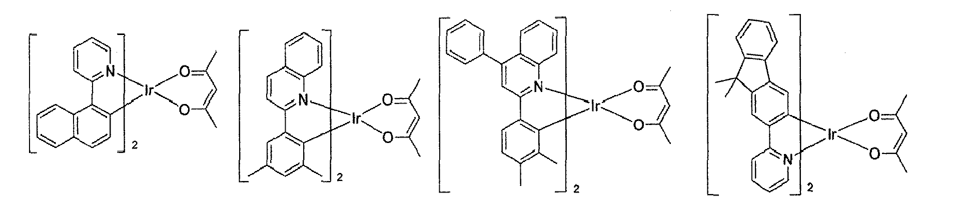

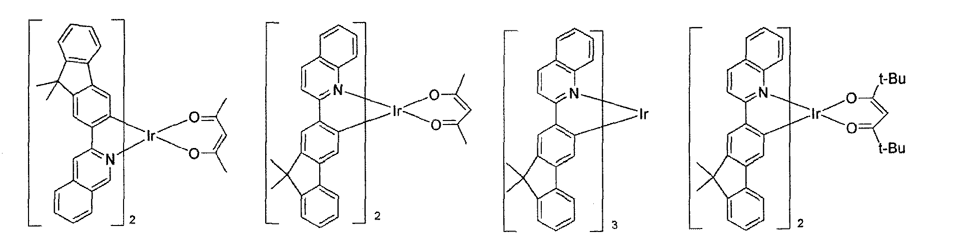

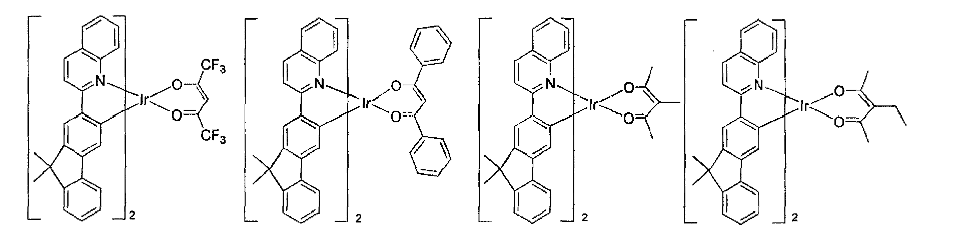

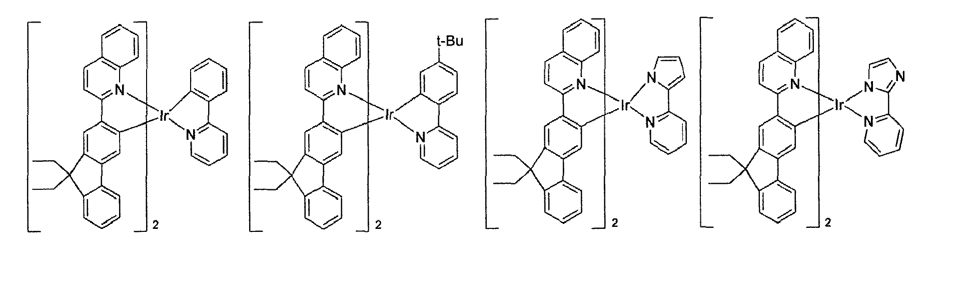

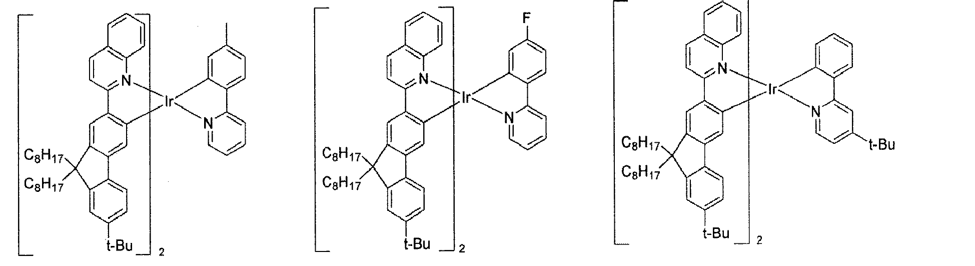

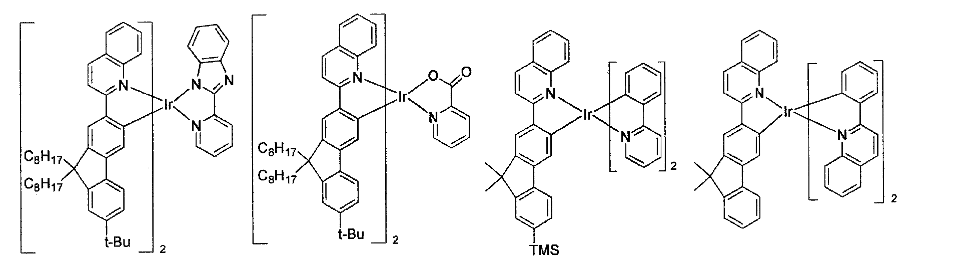

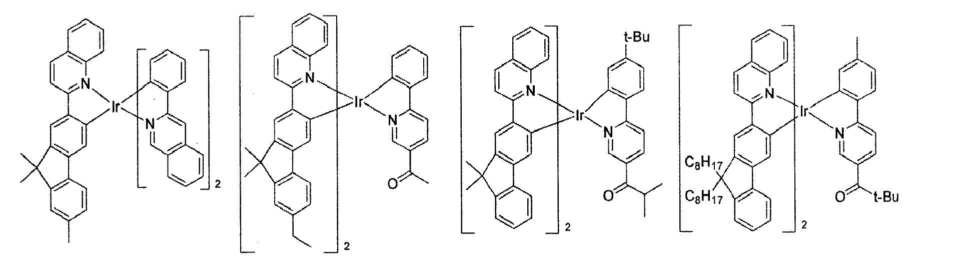

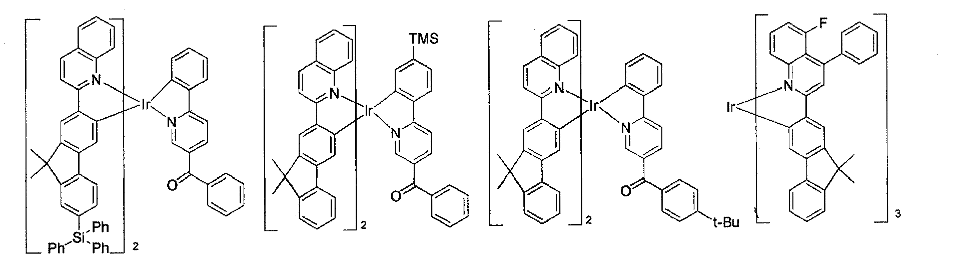

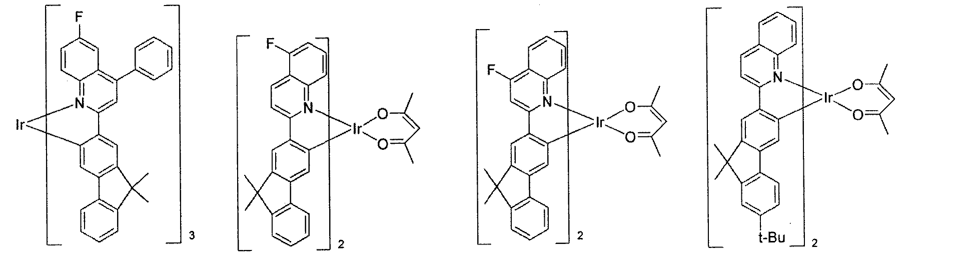

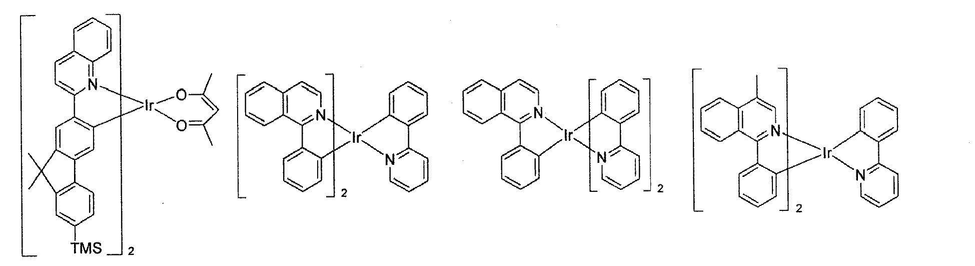

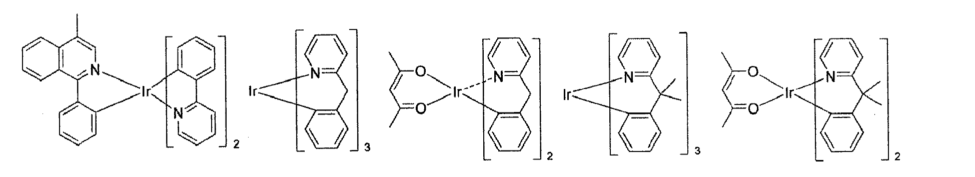

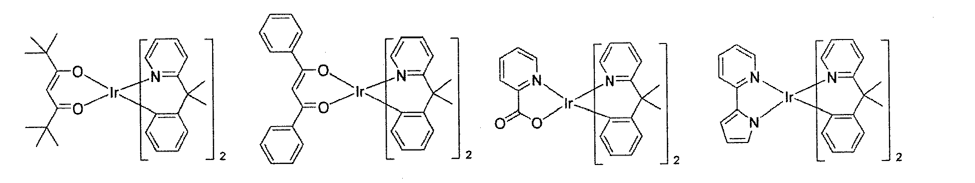

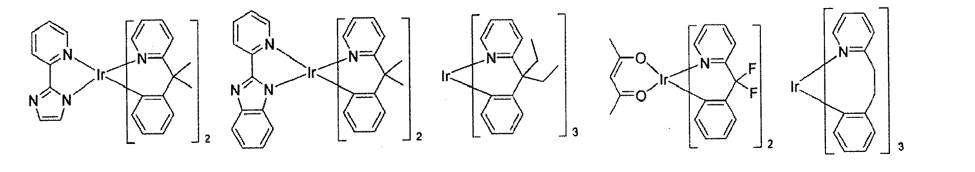

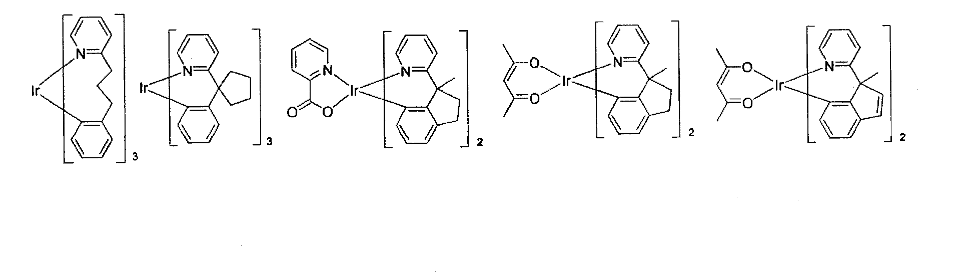

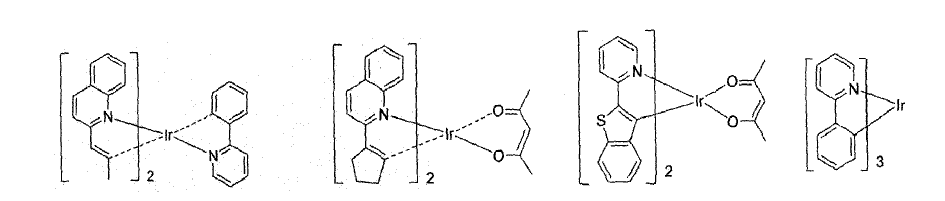

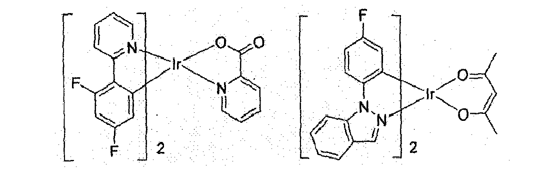

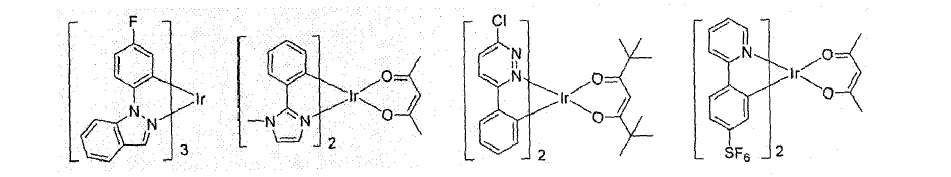

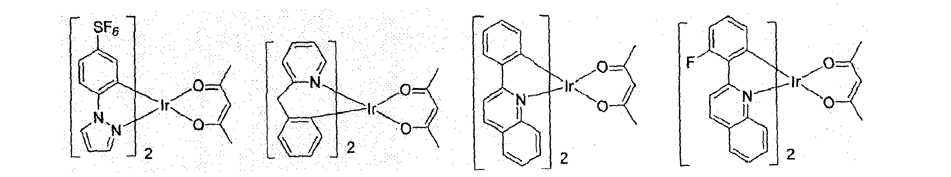

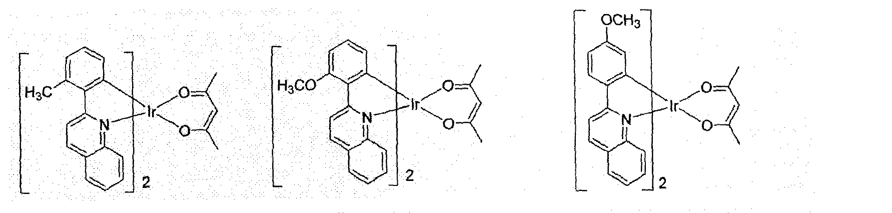

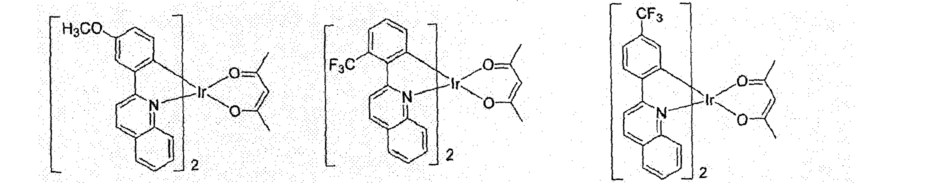

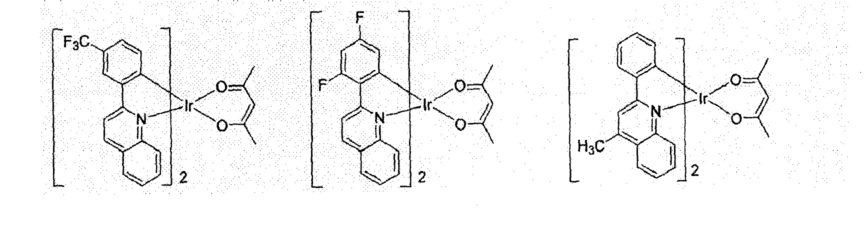

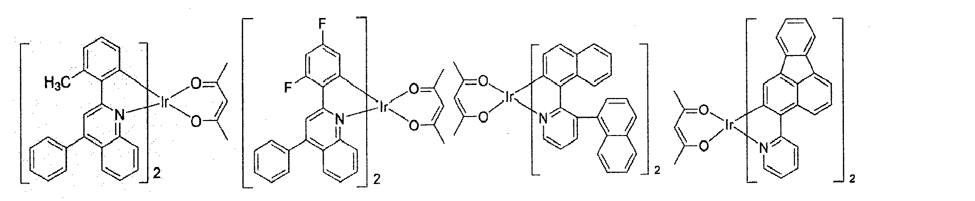

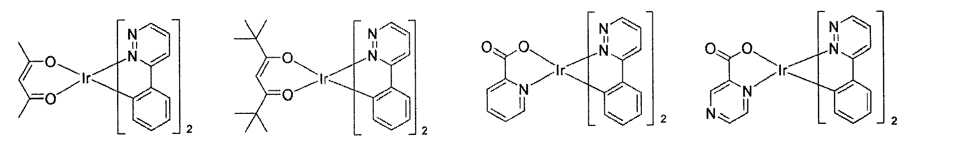

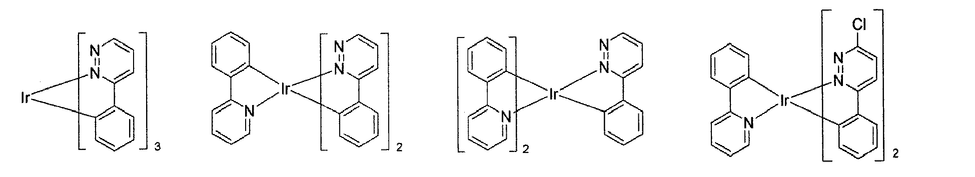

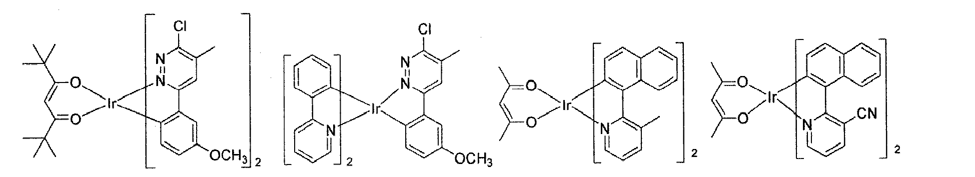

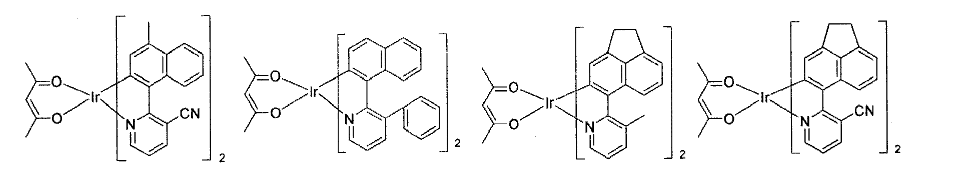

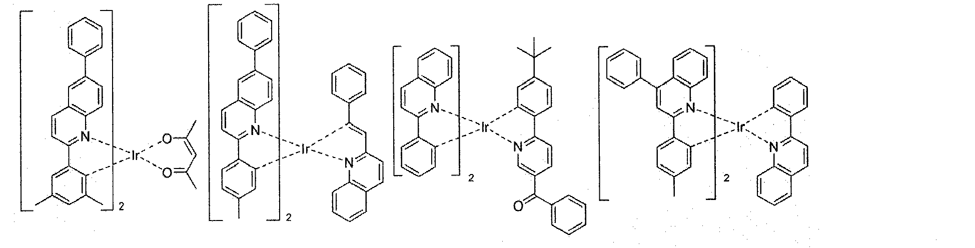

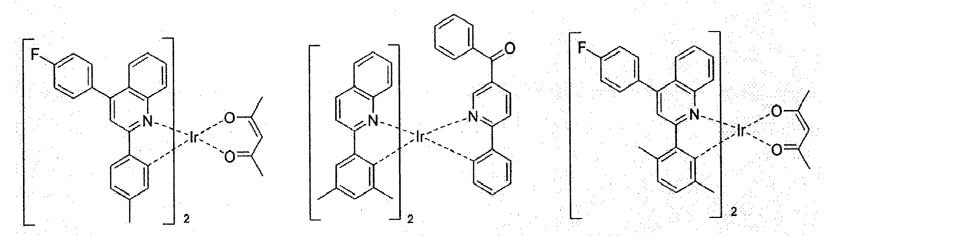

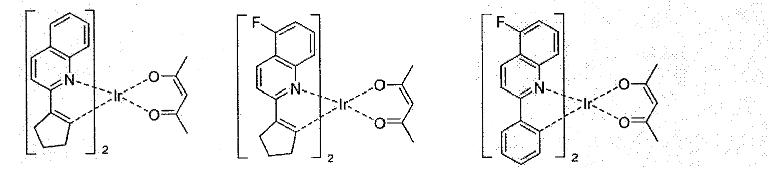

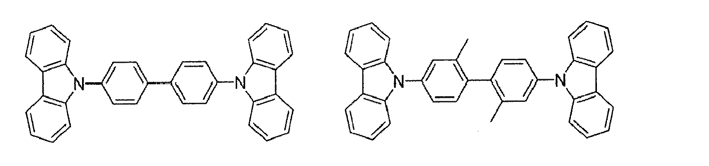

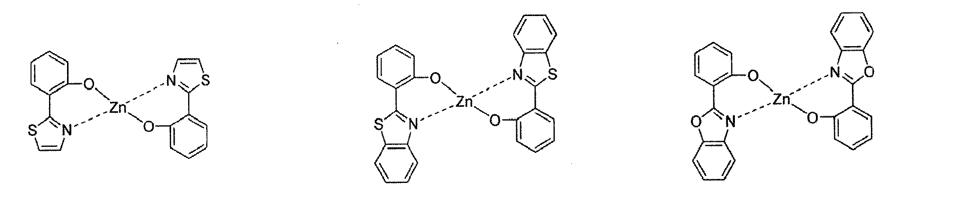

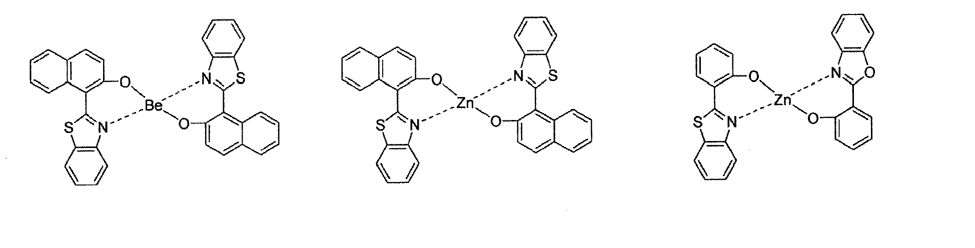

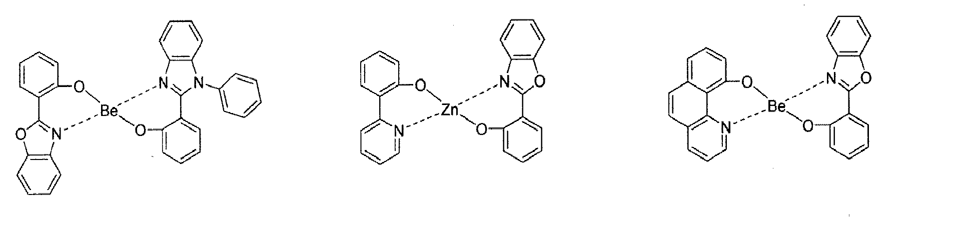

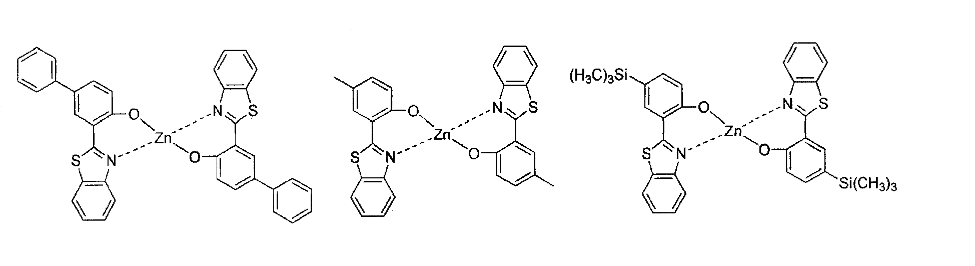

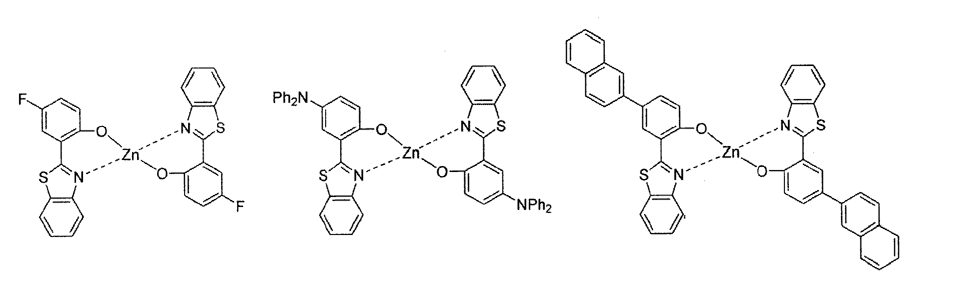

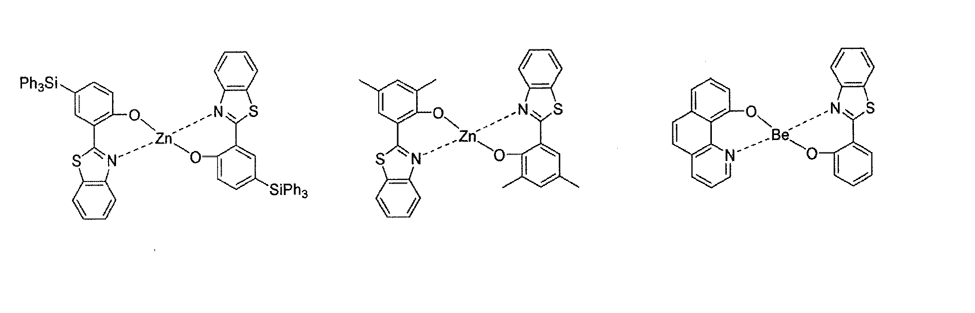

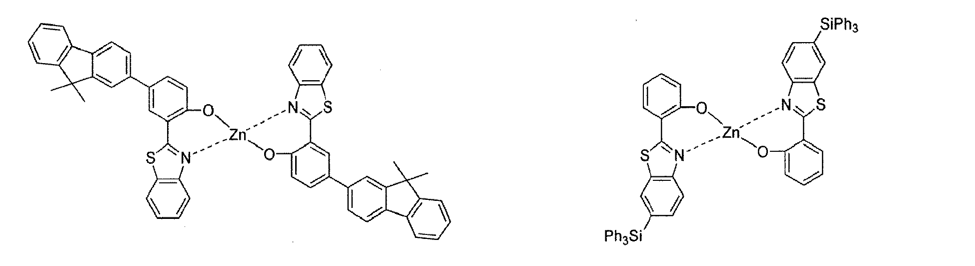

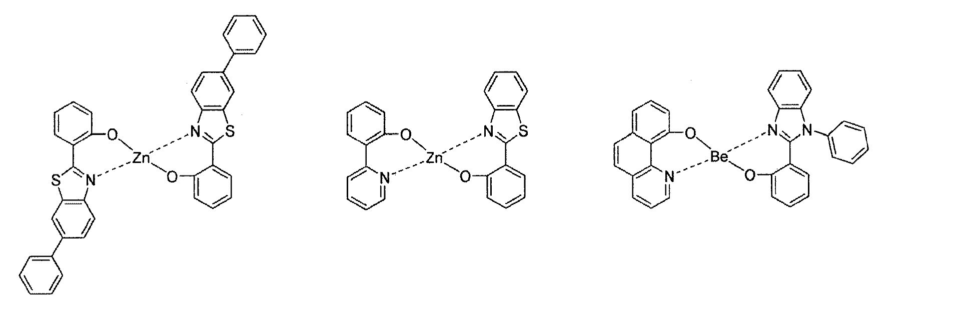

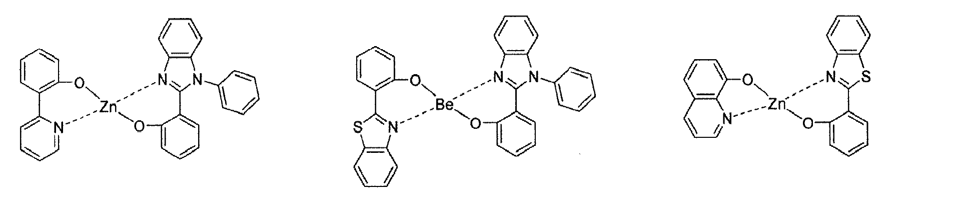

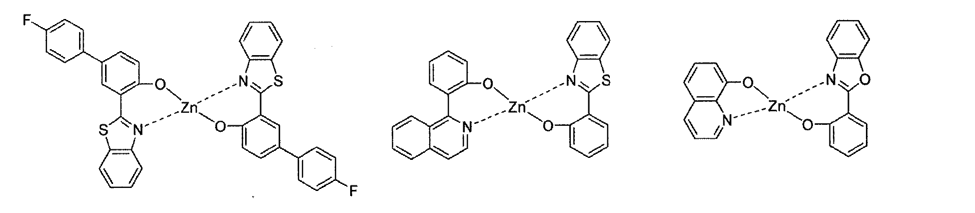

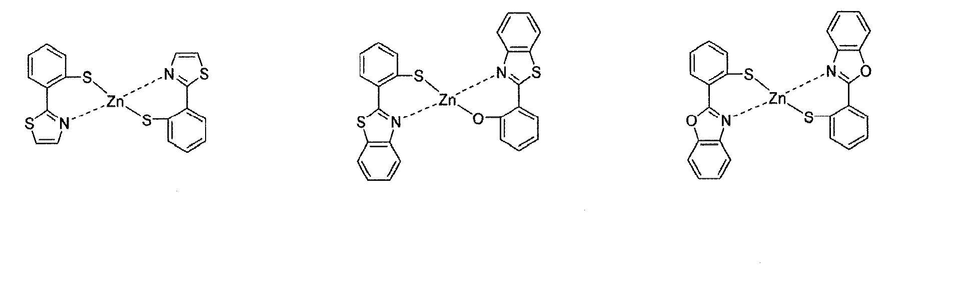

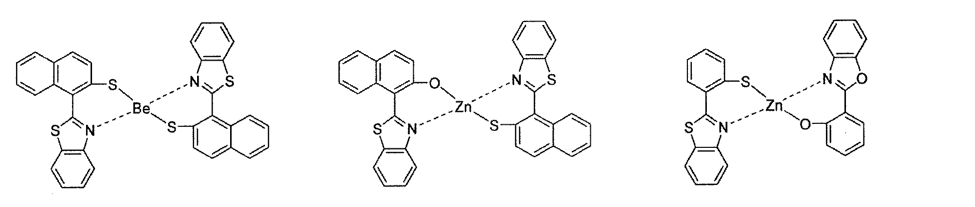

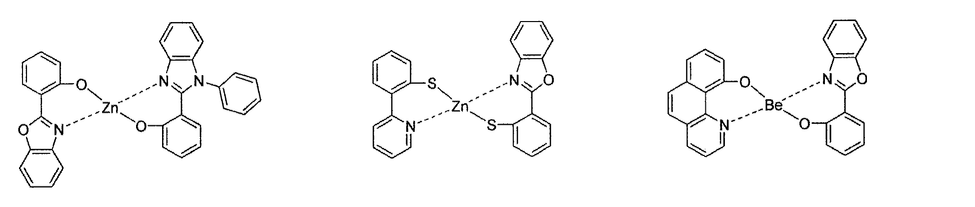

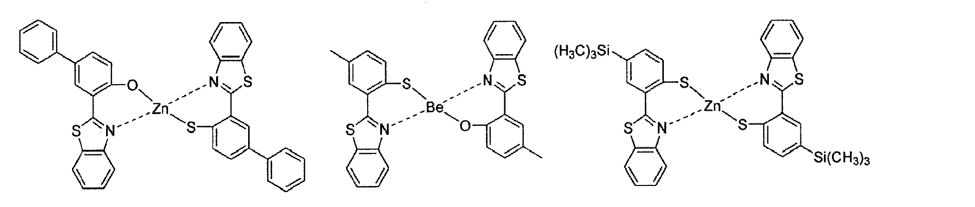

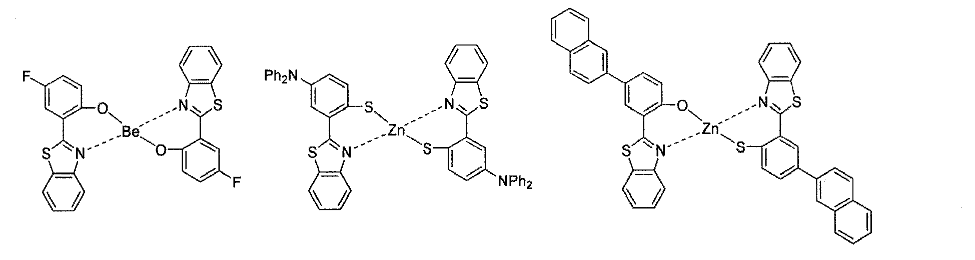

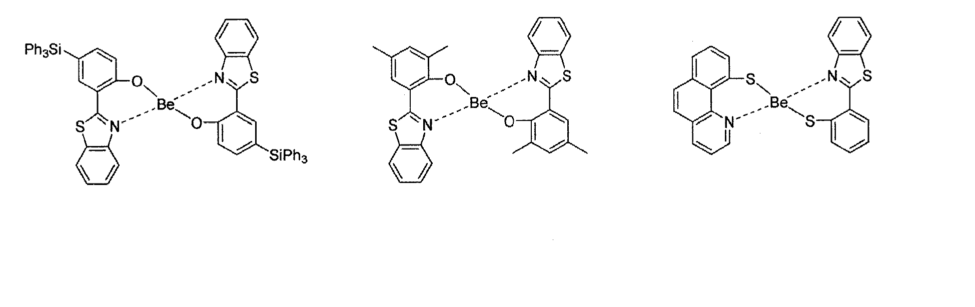

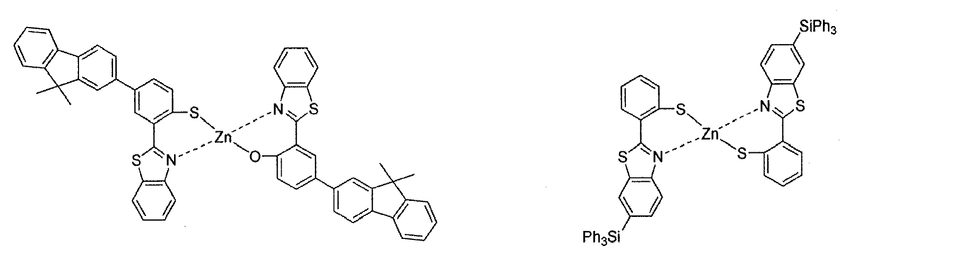

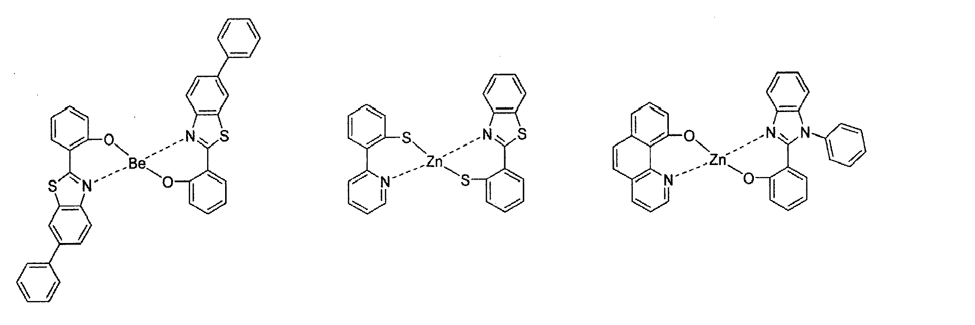

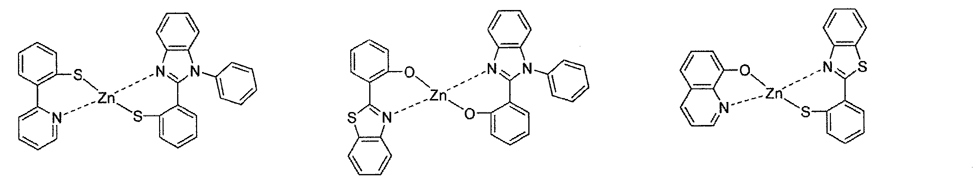

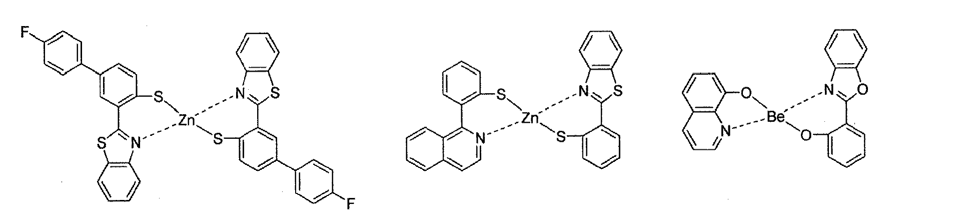

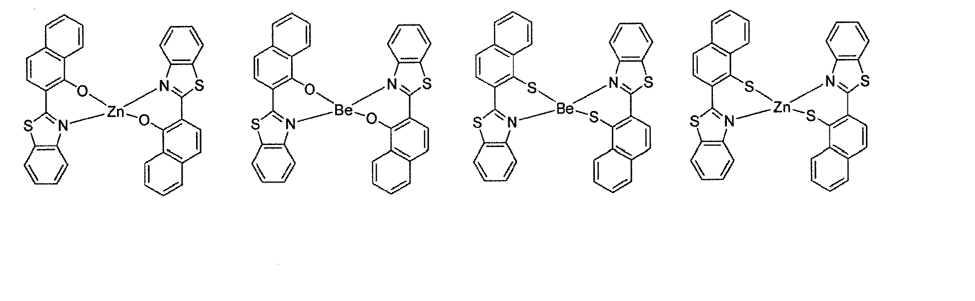

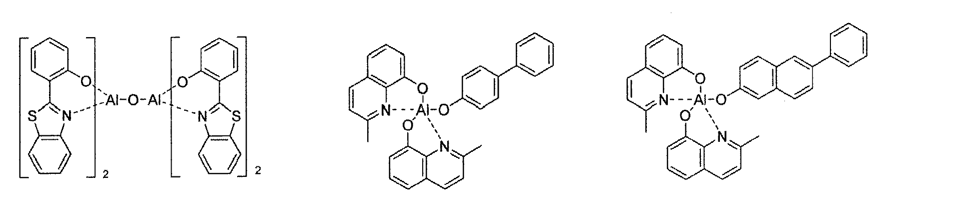

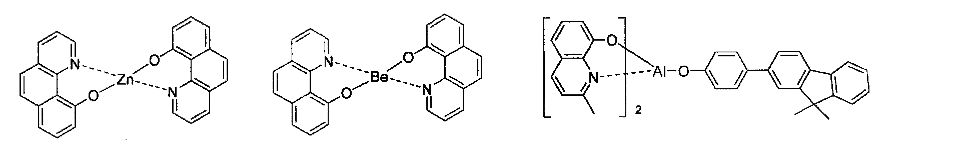

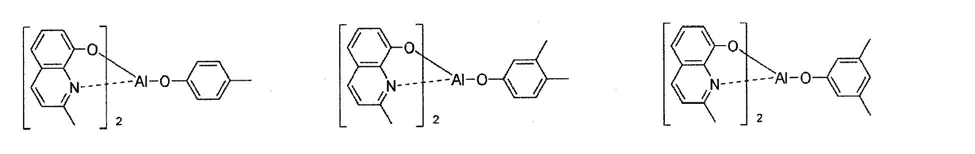

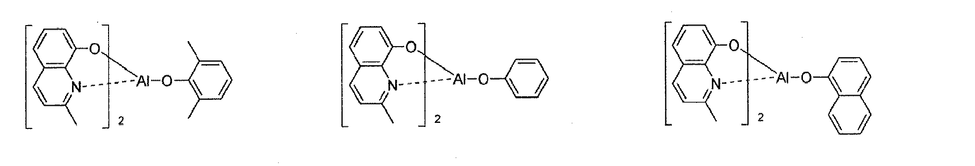

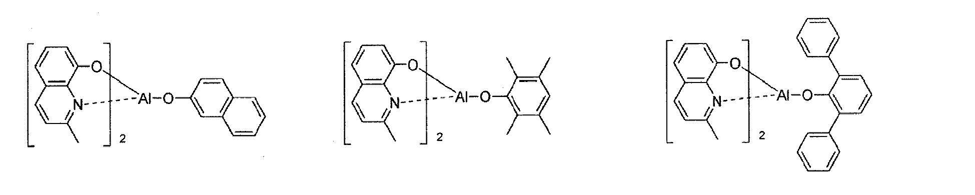

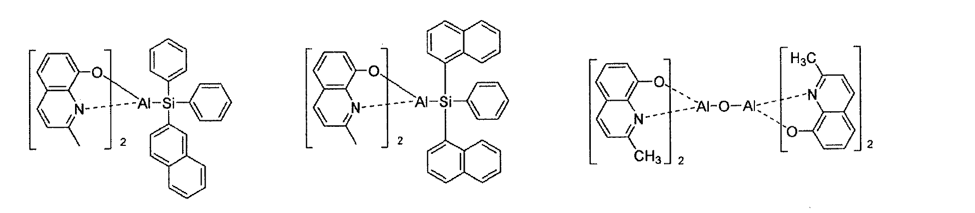

In an organic electroluminescent device according to the present invention, it is preferable to displace one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of lanthanides or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic electroluminescent compounds according to the invention exhibit high luminous efficiency and excellent color purity and life property as a material, so that an OLED having very good operation life can be prepared therefrom.

### Best Mode

The present invention is further described with respect to the compounds according to the invention, the processes for preparing the same, and electroluminescent properties of devices manufactured therefrom by referring to the representative compounds of the invention, which are provided for illustration of the embodiments only but are not intended to limit the scope of the invention by any means.

### Preparation Examples

### [Preparation Example 1] Preparation of Compound (2)

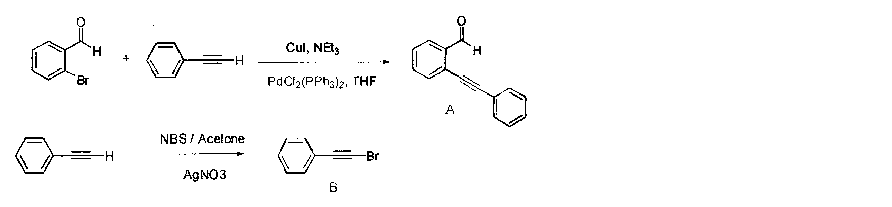

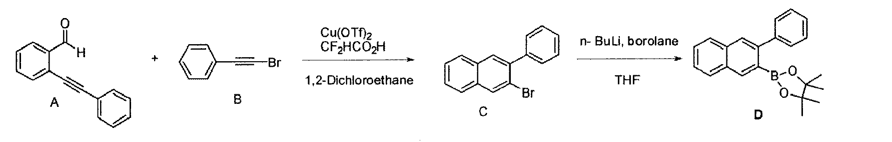

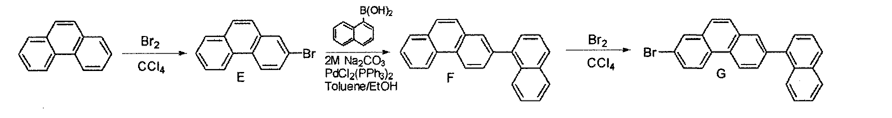

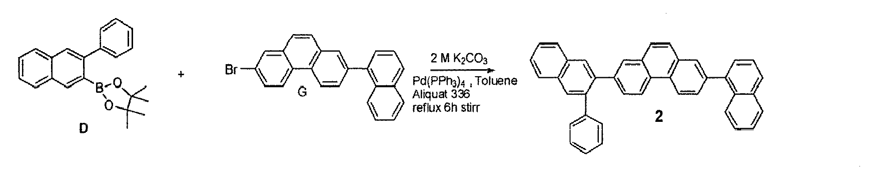

### Preparation of Compound (A)

To tetrahydrofuran (THF) (670 mL), added were 2-bromobenzaldehyde (25.0 g, 140 mmol), phenylacetylene (17.8 mL, 162 mmol), dichlorobis(triphenylphosphine)palladium (II) [PdCl₂(PPh₃)₂] (2.8 g, 4 mmol) and cuprous iodide [CuI] (1.3 g, 7 mmol) and triethylamine (38 mL, 270 mmol) under nitrogen atmosphere, and the mixture was stirred under reflux at 80°C for 3 hours. The reaction mixture was washed with distilled water and ethyl acetate, and purified via column chromatography to obtain Compound (A) (16.0 g, 78 mmol).

### Preparation of Compound (B)

Phenylacetylene (32.3 mL, 294 mmol), NBS (N-bromo succinimide) (58 g, 323 mmol) and silver nitrate [AgNO₃] (5.0 g, 30 mmol) were added to acetone under nitrogen atmosphere, and the mixture was stirred at 0°C. When the reaction was completed, n-hexane was added thereto, and the mixture was filtered. The solid obtained was washed four times with n-hexane to obtain Compound (B) (16.0 g, 93 mmmol).

### Preparation of Compound (C)

Compound (A) (16.0 g, 78 mmol), copper (II) trifluoromethanesulfonate [Cu(OSO₂CF₃)₂] (1.6 g, 1/10 of Compound A) and 1,2-dichloroethane (160 mL) were charged to a flask, and the mixture was maintained under nitrogen atmosphere. Compound (B) (16.0 g, 93 mmol), difluoroacetic acid (5 mL) and 1,2-dichloroethane (160 mL) were added to another flask while maintaining nitrogen atmosphere. The contents of two flasks were combined, and the resultant mixture was stirred at 100°C under reflux for 30 minutes. The compound obtained was washed with distilled water, and purified via column chromatography to obtain Compound (C) (7.3 g, 26 mmol).

### Preparation of Compound (D)

Compound (C) (7.3 g, 26 mmol) was dissolved in THF (130 mL), and n-butyllithium (13.5 mL, 33.8 mmol, 2.5 M in hexane) was added thereto, and the mixture was stirred at -78°C for 1 hour. After 1 hour, added was 2-isapropoxy-4,4,5,5-tetramethyl-1,3,2-dioxaborolane (7.9 mL) (39 mmol), and the resultant mixture was stirred at room temperature for 12 hours. When the reaction was completed, the reaction mixture was washed with distilled water and ethyl acetate. Purification via column chromatography gave Compound (D) (4.8 g, 14 mmol).

### Preparation of Compound (E)

Phenanthrene (10 g, 0.056 mmol), bromine (7.4 mL, 0.15 mol) and CCl₄ (280 mL) were charged to a reaction vessel, and the mixture was stirred under reflux at a temperature of 100°C or higher for 4 hours. When the reaction was completed, aqueous sodium thiosulfate (Na₂S₂O₃) solution was added, and the resultant mixture was stirred for 1 hour. The mixture was extracted with ethyl acetate, and the extract washed three times with distilled water. The organic layer thus obtained was evaporated by using a rotary evaporator, and purified via column chromatography to obtain Compound (E) (9 g, 48%).

### Preparation of Compound (F)

In a reaction vessel, Compound (E) (8.3 g, 0.025 mol) and 2-naphthylboronic acid (6.4 g, 0.037 mol) and tetrakis(triphenylphosphine)palladium (0) (Pd[P(CₛHₛ)₃]₄) (0.9 g, 0.001 mol) were kept under nitrogen atmosphere. Then, 2M Na₂CO₃ solution (50 mL), toluene (130 mL) and ethanol (65 mL) were added thereto, and the mixture was stirred under reflux at 80°C for 12 hours. When the reaction was completed, the reaction mixture was extracted with ethyl acetate, and the extract washed three times with distilled water. The organic layer thus obtained was evaporated by using a rotary evaporator and purified via column chromatography to obtain Compound (F) (8 g, 83.5%).

### Preparation of Compound (G)

Under nitrogen atmosphere, 2-bromophenanthrene (6.3 g, 0.021 mol) was added to bromine (1.6 mL, 0.31 mol) and CCl₄ (103 mL), and the mixture was stirred under reflux at a temperature of 100°C or higher for 4 hours. When the reaction was completed, aqueous sodium thiosulfate (Na₂S₂O₃) solution was added thereto, and the mixture was stirred for 1 hour. The resultant mixture was extracted with ethyl acetate, and the extract washed three times with distilled water. The organic layer thus obtained was evaporated by using a rotary evaporator, and purified via column chromatography to obtain Compound (G) (5 g, 62%).

### Preparation of Compound (2)

Compound (G) (4.6 g, 12 mmol), Compound (D) (4.8 g, 14 mmol), 2M potassium carbonate solution (18 mL) and tetrakis(triphenylphosphine)palladium [Pd(PPh₃)₄] (0.7 g, 0.6 mmol) were added to toluene (100 mL). Aliquat 336 (0.1 mL) was added thereto, and the mixture was stirred under reflux at 80°C for 12 hours. The compound thus obtained was washed with distilled water and ethyl acetate, and purified via column chromatography to obtain the target compound (Compound 2) (3.2 g, 5.5 mmol).

### [Preparation Example 2] Preparation of Compound (360)

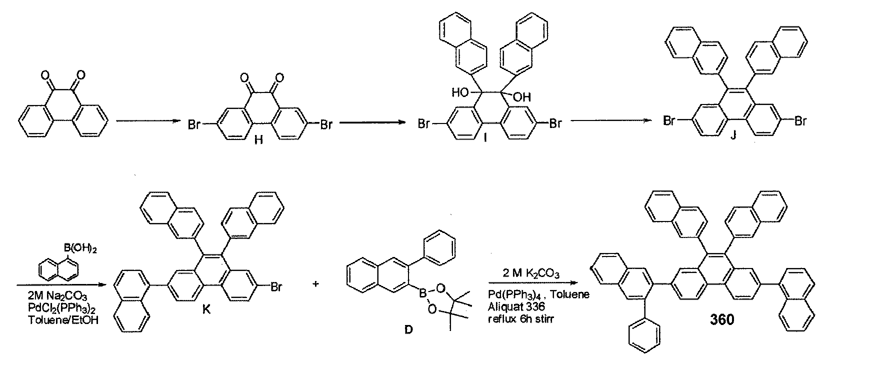

### Preparation of Compound (H)

Trifluoromethanesulfonic acid (29.5 mL, 0.33 mol) was slowly added to 9,10-phenanthrenequinone (7 g, 0.0336 mol) at 0°C. While maintaining the temperature at 0°C, NBS (13.2 g, 0.0742 mol) was slowly added thereto. Then the reaction mixture was warmed to ambient temperature, and stirred for 6 hours. Then the mixture was slowly poured into ice water, and filtered under reduced pressure. Washing with water and methanol gave Compound (H) (10 g, 81%).

### Preparation of Compound (I)

In THF, dissolved was 2-bromonaphthalene (16.9 g, 0.0819 mol), and the solution was chilled to -78°C. To the solution, slowly added was n-BuLi (2.5 M in hexane) (28 mL). After 30 minutes, the mixture was warmed to ambient temperature, and stirred for additional 30 minutes. Compound (H) (10 g, 0.0273 mol) was added at once, and the mixture was stirred at ambient temperature for 12 hours. After extracting with ethyl acetate/distilled water, the extract was dried over magnesium sulfate (MgSO₄) and filtered under reduced pressure. Purification via column chromatography gave Compound (I) (8 g, 47%) as solid.

### Preparation of Compound (J)

Compound (1) (8 g, 0.0129 mol) and acetic acid (100 mL) were heated under reflux. Zinc (12.3 g, 0.194 mol) and HCl (35%, 50 mL) were slowly added thereto. After 30 minutes, same amount of Zn and HCl were further added thereto. After 12 hours of heating under reflux, the solid produced was filtered, and neutralized by adding Na₂CO₃ (aq.). Purification via column chromatography gave Compound (J) (5 g, 65.8%) as solid.

### Preparation of Compound (K)

Compound (J) (5 g, 8.5 mmol), 1-naphthaleneboronic acid (1.5 g, 8.7 mmol), PdCl₂(PPh₃)₂ (0.3 g, 0.427 mmol), 2M K₂CO₃ solution (12.5 mL), toluene (40 mL) and ethanol (20 mL) were heated under reflux at 80°C for 3 hours. When the reaction was completed, the reaction mixture was extracted with ethyl acetate/ distilled water, and purified via adsorption column to obtain Compound (K) (3 g, 55.5%) as solid.

### Preparation of Compound (360)

Compound (K) (3 g, 4.72 mmol), Compound (D) (2.3 g, 9.27 mmol), Pd(PPh₃)₄ (0.5 g, 0.43 mmol), 2M K₂CO₃ (aq. 10 mL), Aliquat 336 (0.05 mL), toluene (30 mL) and ethanol (15 mL) were heated under reflux at 90°C for 6 hours. When the reaction was completed, the reaction mixture was extracted with ethyl acetate/ distilled water, and the extract was purified via adsorption column to obtain the target compound (Compound 360) (1.8 g, 50.2%) as solid.

### [Preparation Example 3] Preparation of Compound (794)

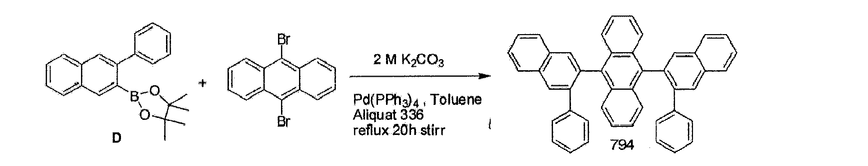

Under nitrogen atmosphere, 9,10-dibromoanthracene (4.6 g, 12 mmol), Compound (D) (4.8 g, 14 mmol), 2M potassium carbonate solution (18 mL) and tetrakis(triphenylphosphine)palladium [Pd(PPh₃)₄] (0.7 g, 0.6 mmol) were added to toluene (100 mL), and Aliquat 336 (0.1 mL) was added thereto. The resultant mixture was stirred under reflux at 80°C for 12 hours. The compound thus obtained was washed with distilled water and ethyl acetate, and purified via column chromatography to obtain Compound (794) (3.2 g, 5.5 mmol).

According to the same procedure as Preparation Examples 1 to 3, organic electroluminescent compounds (Compounds 1 to 939) were prepared, of which the ¹H NMR and MS/FAB data are shown in Table 1.

**Table 1**

| Comp. | ¹H NMR(CDCl₃, 200 MHz) | MS/FAB | |
|---|---|---|---|
| | | found | calculated |
| **1** | δ = 7.41(2H, m), 7.51∼7.52(6H, m), 7.59(2H, m), 7.71(2H, m), 7.79(2H, m), 8(2H, m), 8.1(2H, m)_{,} 8_{.}34(2H, m), 8.4(2H, m), 8.99(2H, m) | 456.58 | 456.19 |
| **2** | δ = 7.41(1H, m), 7.51∼7.61(7H, m), 7.71(2H, m), 7.79(2H, m), 8∼8.1(6H, m), 8.34(2H, m), 8.4-8.42(3H, m), 8.55(1H, m), 8.99(2H, m) | 506.63 | 506.20 |
| **4** | δ = 2.34(3H, s), 7.29∼7.33(4H, m), 7.41(1H, m), 7.51(2H, m), 7.59(2H, m), 7.71(2H, m), 7.79(2H, m), 8(2H, m), 8.1(2H, m), 8.34(2H, m), 8.4(2H, m), 8.99(2H, m) | 470.60 | 470.20 |
| **7** | δ = 7.41(1H, m), 7.51(2H, m), 7.59(2H, m), 7.71(2H, m), 7.79∼7.93(7H, m), 8(2H, m), 8.1-8.12(4H, m), 8.34(2H, m), 8.4(2H, m), 8.93(2H, m), 8.99(2H, m) | 556.69 | 556.22 |
| **13** | δ = 7.41(3H, m), 7.51∼7.52(10H, m), 7.59(2H, m), 7.66∼7.71(5H, m), 7.79(2H, m), 8(2H, m), 8.1(2H, m)_{,} 8.34(2H, m), 8.4(2H, m), 8.99(2H, m) | 608.77 | 608.25 |
| **15** | δ = 7.41(2H, m), 7.51(4H, m), 7.59(4H, m), 7.71(2H, m), 7.79(4H, m), 8(4H, m), 8.1(2H, m), 8.34(2H, m), 8.4(4H, m), 8.99(2H, m) | 582.73 | 582.23 |
| **23** | δ = 7.41(1H, m), 7.5∼7.59(7H, m), 7.71(2H, · m), 7.79∼7.82(3H, m), 7.94∼8(4H, m), 8.1(2H, m), 8.34(2H, m), 8.4∼8.45(3H, m), 8.99(2H, m) | 562.72 | 562.18 |
| **31** | δ = 7.41(1H, m), 7.51(2H, m), 7.58-7.59(3H, m), 7.71(2H, m), 7.79∼7.8(4H, m), 7.9∼8(4H, m), 8.1(4H, m), 8.34(2H, m), 8.4∼8.42(4H, m), 8.99(2H, m) | 580.71 | 580.22 |
| **33** | δ = 7.25∼7.33(3H, m), 7.41(1H, m), 7.5∼7.51(3H, m), 7.59∼7.63(3H, m), 7.7(2H, m), 7.79(2H, m), 7.9∼8(4H, m), 8.1-8.12(3H, m), 8.3(1H, m), 8.4(2H, m), 8.55(1H, m), 8.9(1H, m), 9(1H, m) | 545.67 | 545.21 |
| **39** | δ = 1.96(2H, m), 2.76(2H, m), 3.06(2H, m), 6.55(1H, m), 6.72(1H, m), 7.05∼7.07(3H, m), 7.29(1H, m), 7.41(1H, m), 7.51(2H, m), 7.59(2H, m), 7.71(2H, m), 7.79(2H, m), 8(2H, m), 8.1(1H, m), 8.34(1H, m), 8.4(2H, m), 8.71(1H, m), 8.99(1H, m) | 511.65 | 511.23 |
| **48** | δ = 6.97(2H, m), 7.08(1H, m), 7.16∼7.21(6H, m), 7.32(1H, m), 7.41(1H, m), 7.51(2H, m), 7.59(2H, m), 7.71(2H, m), 7.79(2H, m), 8(2H, m), 8.1(1H, m), 8.34(1H, m), 8.4(2H, m), 8.68(1H, m), 8.99(1H, m) | 577.74 | 577.19 |
| **52** | δ = 2.88(4H, m), 6.58(2H, m), 6.76(2H, m), 7.02∼7.08(5H, m), 7.32(1H, m), 7.41(1H, m), 7.51(2H, m), 7.59(2H, m), 7.71(2H, m), 7.79(2H, m), 8(2H, m), 8.1(1H, m), 8.34(1H, m), 8.4(2H, m), 8.68(1H, m), 8.99(1H, m) | 573.72 | 573.25 |
| **56** | δ = 7.36-7.42(4H, m), 7.48-7.51(3H, m), 7.59(2H, m), 7.71∼7.84(9H, m), 8∼8.12(7H, m), 8.34(2H, m), 8.4(2H, m), 8.99(2H, m) | 638.73 | 638.22 |
| **70** | δ = 7.41∼7.51(13H, m), 7.59(2H, m), 7.71-7.79(5H, m), 7.89(1H, m), 8(2H, m), .1(2H, m), 8.28(1H, m), 8.34(2H, m), 8.4(2H, m), 8.99(2H, m) | 660.80 | 660.26 |
| **72** | δ = 6.63(4H, m), 6.69(2H, m), 6.81(2H, m), 7.2(4H, m), 7.41(1H, m), 7.51∼7.59(6H, m), 7.71(2H, m), 7.79(2H, m), 8(2H, m), 8.1(2H, m), 8.34(2H, m), 8.4(2H, m), 8.99(2H, m) | 623.78 | 623.26 |
| **74** | δ = 6.63(2H, m), 6.81(1H, m), 7.08(1H, m), 7.2(2H, m), 7.32~7.41(3H, m), 7.49∼7.51(4H, m), 7.59(2H, m), 7.71∼7.88(8H, m), 8(2H, m), 8.1(1H, m), 8.34(1H, m), 8.4(2H, m), 8.68(1H, m), 8.99(1H, m) | 597.74 | 597.25 |
| **77** | δ = 1.72(6H, s), 6.58(1H, m), 6.75(1H, m), 7.08(1H, m), 7.28∼7.41(5H, m), 7.49∼7.62(8H, m), 7.71∼7.88(9H, m), 8(2H, m), 8.1(1H, m), 8.34(1H, m), 8.4(2H, m), 8.68(1H, m), 8.99(1H, m) | 713.90 | 713.31 |
| **79** | δ = 7.25(4H, m), 7.41(1H, m), 7.51-7.61(7H, m), 7.71(2H, m), 7.79(2H, m), 8-8.1(6H, m), 8.34(2H, m), 8.4∼8.42(3H, m), 8.55(1H, m), 8.99(2H, m) | 582.73 | 582.23 |
| **80** | δ = 7.25(4H, m), 7.41(1H, m), 7.51(2H, m), 7.58~7.59(5H, m), 7.71∼7.79(5H, m), 7.92(1H, m), 8(4H, m), 8.1(2H, m), 8.34(2H, m), 8.4(2H, m), 8.99(2H, m) | 582.73 | 582.23 |
| **82** | δ = 7.25(4H, m), 7.41(1H, m), 7.51(2H, m), 7.59(2H, m), 7.71(2H, m), 7.79∼7.93(7H, m), 8(2H, m), 8.1∼8.12(4H, m), 8.34(2H, m), 8.4(2H, m), 8.93(2H, m), 8.99(2H, m) | 632.79 | 632.25 |
| **85** | δ = 7.39∼7.41(5H, m), 7.51(2H, m), 7.59(2H, m), 7.71(2H, m), 7.79∼7.93(11H, m), 8(2H, m), 8.1∼8.12(4H, m), 8.34(2H, m), 8.4(2H, m), 8.93(2H, m), 8.99(2H, m) | 732.91 | 732.28 |
| **100** | δ = 7.41(2H, m), 7.51∼7.59(8H, m), 7.71(2H, m), 7.79(4H, m), 8∼8.01(4H, m), 8.1(2H, m), 8.34(2H, m), 8.4(2H, m), 8.55(2H, m), 8.99(2H, m) | 582.73 | 582.23 |
| **106** | δ = 1.72(6H, s), 7.41(2H, m), 7.51-7.52(6H, m), 7.59∼7.63(4H, m), 7.71∼7.79(6H, m), 7.93(2H, m), 8(2H, m), 8.1(2H, m), 8.34(2H, m), 8.4(2H, m), 8.99(2H, m) | 648.83 | 648.28 |
| **110** | δ = 7.41(1H, m), 7.48∼7.51(4H, m), 7.57∼7.59(3H, m), 7.7∼7.71(3H, m), 7.79∼7.93(7H, m), 8(2H, m), 8.1∼8.12(4H, m), 8.34(2H, m), 8.4(2H, m), 8.93(2H, m), 8.99(2H, m) | 632.79 | 632.25 |
| **113** | δ = 7.37∼7.59(22H, m), 7.71(2H, m), 7.79(2H, m), 7.89(2H, m), 8(2H, m), 8.1(2H, m), 8.34(2H, m), 8.4(2H, m), 8.99(2H, m) | 714.97 | 714.27 |
| **114** | δ = 7.39∼7.41(6H, m), 7.48∼7.59(11H, m), 7.7∼7.71(3H, m), 7.79(2H, m), 7.91(4H, m), 8(2H, m), 8.1(2H, m), 8.34(2H, m), 8.4(2H, m), 8.99(2H, m) | 708.89 | 708.28 |
| **115** | δ = 7.41(1H, m), 7.51∼7.64(7H, m), 7.71∼7.84(10H, m), 8(2H, m), 8.1(2H, m), 8.34(2H, m), 8.4(2H, m), 8.99(2H, m) | 560.68 | 560.21 |
| **117** | δ = 1.72(6H, s), 6.63(2H, m), 6.69(2H, m), 6.81(1H, m), 7.08(1H, m), 7.2(2H, m), 7.28∼7.41(4H, m), 7.51∼7.63(8H, m), 7.71∼7.79(5H, m), 7.87∼7.93(2H, m), 8(2H, m), 8.1(1H, m), 8.34(1H, m), 8.4(2H, m), 8.68(1H, m), 8.99(1H, m) | 739.94 | 739.32 |
| **121** | δ = 7.41(4H, m), 7.51∼7.52(12H, m), 7.59(2H, m), 7.66(3H, m), 7.79(4H, m), 7.93(1H, m), 8(2H, m), 8.1(2H, m), 8.34(2H, m), 8.4(2H, m), 8.99(2H, m) | 684.86 | 684.28 |
| **131** | δ = 7.41(2H, m), 7.51(4H, m), 7.59(2H, m), 7.79∼7.93(10H, m), 8(2H, m), 8.1∼8.12(4H, m), 8.34(2H, m), 8.4(2H, m), 8.93(2H, m), 8.99(2H, m) | 632.79 | 632.25 |
| **156** | δ = 7.41∼7.59(14H, m), 7.79(4H, m), 7.93(1H, m), 8(2H, m), 8.1(2H, m), 8.2(2H, m), 8.3∼8.34(6H, m), 8.4(2H, m), 8.99(2H, m) | 685.85 | 685.28 |
| **160** | δ = 7.41(2H, m), 7.51(4H, m), 7.59(2H, m), 7.79∼7.93(9H, m), 8∼8.04(3H, m), 8.1-8.12(4H, m), 8.18(1H, m), 8.34(2H, m), 8.4(2H, m), 8.93(2H, m), 8.99(2H, m), 9.15(1H, m) | 682.85 | 682.27 |
| **173** | δ = 7.25(4H, m), 7.41(2H, m), 7.51(4H, m), 7.59(2H, m), 7.79∼7.93(10H, m), 8(2H, m). 8.1∼8.12(4H, m), 8.34(2H, m), 8.4(2H, m), 8.93(2H, m), 8.99(2H, m) | 708.89 | 708.28 |
| **179** | δ = 1.72(6H, s), 7.28(1H, m), 7.38∼7.41(7H, m), 7.51∼7.63(8H, m), 7.77∼7.79(5H, m), 7.87∼7.93(7H, m), 8(2H, m), 8.1(2H, m), 8.34(2H, m), 8.4(2H, m), 8.99(2H, m) | 825.04 | 824.34 |
| **182** | δ = 7.41(2H, m), 7.48∼7.51(6H, m), 7.57∼7.59(6H, m), 7.7∼7.79(6H, m), 7.92∼7.93(2H, m), 8(4H, m), 8.1(2H, m), 8.34(2H, m), 8.4(2H, m), 8.99(2H, m) | 658.83 | 658.27 |
| **193** | δ = 7.41(3H, m), 7.51(6H, m), 7.59∼7.61(4H, m), 7.79(6H, m), 7.93(1H, m), 8(2H, m), 8.1(2H, m), 8.34(2H, m), 8.4∼8.42(4H, m), 8.51(2H, m), 8.99(2H, m) | 658.83 | 658.27 |
| **202** | δ = 6.63(4H, m), 6.81(2H, m), 7.08(1H, m), 7.2(4H, m), 7.32(1H, m), 7.41(2H, m), 7.51(4H, m), 7.59(2H, m), 7.79(4H, m), 7.93(1H, m), 8(2H, m), 8.1(1H, m), 8.34(1H, m), 8.4(2H, m), 8.68(1H, m), 8.99(1H, m) | 623.78 | 623.26 |
| **206** | δ = 7.25(4H, m), 7.39∼7.41(7H, m), 7.51∼7.52(8H, m), 7.59(2H, m), 7.79(4H, m), 7.91∼7.93(5H, m), 8(2H, m), 8.1(2H, m), 8.34(2H, m), 8.4(2H, m), 8.99(2H, m) | 784.98 | 784.31 |
| **208** | δ = 7.11(6H, m), 7.26~7.33(13H, m), 7.41(2H, m), 7.51(4H, m), 7.59(2H, m), 7.79(4H, m), 7.93(1H, m), 8(2H, m), 8.1(2H, m), 8.34(2H, m), 8.4(2H, m), 8.99(2H, m) | 774.99 | 774.33 |
| **214** | δ = 7.41(4H, m), 7.51∼7.52(12H, m), 7.59(4H, m), 7.79(4H, m), 8(4H, m), 8.1(2H, m), 8.34(2H, m), 8.4(4H, m), 8.99(2H, m) | 734.92 | 734.30 |
| **217** | δ = 7.41(3H, m), 7.51~7.61(15H, m), 7.79(2H, m), 8~8.1(6H, m), 8.34(2H, m), 8.4∼8.42(3H, m), 8.55(1H, m), 8.99(2H, m) | 658.83 | 658.27 |
| **230** | δ = 7.32∼7.44(6H, m), 7.51∼7.52(10H, m), 7.59-7.66(4H, m), 7.75-7.79(3H, m), 7.89(1H, m), 8(2H, m), 8.1(2H, m), 8.34(2H, m), 8.4(2H, m), 8.99(2H, m) | 698.85 | 698.26 |
| **248** | δ = 7.36∼7.42(6H, m), 7.48∼7.52(11H, m), 7.59(2H, m), 7.74∼7.84(7H, m), 8~8.12(7H, m), 8.34(2H, m), 8.4(2H, m), 8.99(2H, m) | 790.93 | 790.28 |
| **256** | δ = 1.72(6H, s), 6.58~6.63(5H, m), 6.75~6.81(3H, m), 7.2(4H, m), 7.41(3H, m), 7.511~7.52(10H, m), 7.59∼7.63(4H, m)_{,} 7.77∼7.79(3H, m), 7.93(1H, m), 8(2H, m), 8.1(2H, m), 8.34(2H, m), 8.4(2H, m), 8.99(2H, m) | 892.13 | 891.39 |
| **266** | δ = 7.39∼7.41(7H, m), 7.51∼7.61(15H, m), 7.79(2H, m), 7.91(4H, m), 8~8.1(6H, m), 8.34(2H, m), 8.4∼8.42(3H, m), 8.55(1H, m), 8.99(2H, m) | 835.04 | 834.33 |
| **273** | δ = 7.41(3H, m), 7.48∼7.61(18H, m), 7.7(1H, m), 7.79(2H, m), 8~8.1(6H, m), 8.34(2H, m), 8.4∼8.42(3H, m), 8.55(1H, m), 8.99(2H, m) | 734.92 | 734.30 |
| **281** | δ = 7.41(4H, m), 7.51∼7.59(16H, m), 7.79(4H, m), 8~8.01(4H, m), 8.1(2H, m), 8.34(2H, m), 8.4(2H, m), 8.55(2H, m), 8.99(2H, m) | 734.92 | 734.30 |
| **293** | δ = 7.11(6H, m), 7.26∼7.33(13H, m), 7.41(3H, m), 7.51∼7.52(10H, m), 7.59(2H, m), 7.79(2H, m), 8(2H, m), 8.1(2H, m), 8.34(2H, m), 8.4(2H, m), 8.99(2H, m) | 851.08 | 850.36 |
| **297** | δ = 7.25(4H, m), 7.39∼7.41(8H, m), 7.51∼7.52(14H, m), 7.59(2H, m), 7.79(2H, m), 7.91(4H, m), 8(2H, m), 8.1(2H, m), 8.34(2H, m), 8.4(2H, m), 8.99(2H, m) | 861.08 | 860.34 |
| **302** | δ = 7.41(1H, m), 7.51~7.61(10H, m), 7.73∼7.79(3H, m), 7.92~7.93(2H, m), 8∼8.1(8H, m), 8.34(2H, m), 8.4~8.42(3H, m), 8.55(1H, m), 8.99(2H, m) | 632.79 | 632.25 |
| **313** | δ = 7.25~7.33(3H, m), 7.41(1H, m), 7.5∼7.51(3H, m), 7.58~7.79(13H, m), 7.92∼8(7H, m), 8.1∼8.12(3H, m), 8.34(2H, m), 8.4(2H, m), 8.55(1H, m), 8.99(2H, m) | 747.92 | 747.29 |
| **320** | δ = 7.25(4H, m), 7.41(1H, m), 7.51∼7.61(10H, m), 7.73∼7.79(3H, m), 7.92∼7.93(2H, m), 8∼8.1(8H, m), 8.34(2H, m), 8.4~8.42(3H, m), 8.55(1H, m), 8.99(2H, m) | 708.89 | 708.28 |
| **339** | δ = 7.41(2H, m), 7.51(4H, m), 7.58∼7.61(7H, m), 7.73∼7.79(5H, m), 7.92∼7.93(2H, m), 8(4H, m), 8.1(2H, m), 8.34(2H, m), 8.4∼8.42(4H, m), 8.51(2H, m), 8.99(2H, m) | 708.89 | 708.28 |
| **351** | δ = 1.72(6H, s), 6.63(2H, m), 6.69(2H, m), 6.81(1H, m), 7.08(1H, m), 7.2(2H, m), 7.28∼7.41(4H, m), 7.51∼7.63(11H, m), 7.73∼7.79(4H, m), 7.87∼7.93(4H, m), 8(4H, m), 8.1(1H, m), 8.34(1H, m), 8.4(2H, m), 8.68(1H, m), 8.99(1H, m) | 866.10 | 865.37 |
| **355** | δ = 7.41(3H, m), 7.51~7.52(10H, m), 7.58∼7.59(8H, m), 7.66(3H, m), 7.73∼7.79(4H. m), 7.92(2H, m), 8(6H, m), 8.1(2H, m), 8.34(2H, m), 8.4(2H, m), 8.99(2H, m) | 861.08 | 860.34 |
| **358** | δ = 7.21(1H, m), 7.41(3H, m), 7.51(6H, m), 7.58∼7.59(8H, m), 7.73∼7.79(9H, m), 7.91~7.92(3H, m), 8(6H, m), 8.1(2H_{,} m), 8.34(2H, m), 8.4(2H, m), 8.99(2H, m) | 861.08 | 860.34 |
| **360** | δ = 7.41(1H, m), 7.51∼7.61(13H, m), 7.73∼7.79(4H, m), 7.92(2H, m), 8∼8.1(10H, m), 8.34(2H, m), 8.4~8.42(3H, m), 8.55(1H, m), 8.99(2H, m) | 758.94 | 758.30 |
| **372** | δ = 7.41(1H, m), 7.5~7.59(13H, m), 7.73∼7.82(5H, m), 7.92∼8(10H, m), 8.1(2H, m), 8.34(2H, m), 8.4∼8.45(3H, m), 8.99(2H, m) | 815.03 | 814.27 |
| **374** | δ = 7.41(1H, m), 7.5~7.52(4H, m), 7.58∼7.59(9H, m), 7.73∼7.79(4H, m), 7.92(2H, m), 7.98∼8(7H, m), 8.1(2H, m), 8.2(1H, m), 8.34(2H, m), 8.4∼8.45(4H, m), 8.99(2H, m) | 815.03 | 814.27 |
| **383** | δ = 2.34(3H, m), 6.51(2H, m), 6.98(2H, m), 7.08(1H, m), 7.32∼7.41(3H, m), 7.49∼7.51(4H, m), 7.58∼7.59(8H, m), 7.73~7.92(10H, m), 8(6H, m), 8.1(1H, m), 8.34(1H, m), 8.4(2H, m), 8.68(1H, m), 8.99(1H m) | 864.08 | 863.36 |
| **393** | δ = 7.37∼7.59(32H, m), 7.79(2H, m), 7.89(2H, m), 8(2H, m), 8.1(2H, m), 8.34(2H, m), 8.4(2H, m), 8.99(2H, m) | 867.16 | 866.34 |
| **398** | δ = 7.41(1H, m), 7.51∼7.64(13H, m), 7.73∼7.84(10H, m), 7.92(2H, m), 8(6H, m), 8.1(2H, m), 8.34(2H, m), 8.4(2H, m), 8.99(2H, m) | 812.99 | 812.31 |
| **403** | δ = 1.72(6H, s), 7.28(1H, m), 7.38∼7.41(3H, m), 7.51∼7.63(10H, m), 7.77∼7.79(5H, m), 7.87∼7.93(3H, m), 8(4H, m), 8.1(2H, m), 8.34(2H, m), 8.4(4H, m), 8.99(2H, m) | 774.99 | 774.33 |
| **407** | δ = 1.72(6H, s), 7.21(1H, m), 7.28(1H, m), 7.38∼7.41(4H, m), 7.51∼7.63(10H, m), 7.76∼7.79(8H, m), 7.87∼7.93(4H, m), 8(2H, m), 8.1(2H, m), 8.34(2H, m), 8.4(2H, m), 8.99(2H, m) | 801.02 | 800.34 |
| **413** | δ = 1.72(6H, s), 7.28(1H, m), 7.38∼7.41(2H, m), 7.51∼7.63(6H, m), 7.77∼7.93(11H, m), 8(2H, m), 8.1~8.12(4H, m), 8.34(2H, m), 8.4(2H, m), 8.93(2H, m), 8.99(2H, m) | 748.95 | 748.31 |
| **424** | δ = 1.72(12H, s), 7.28(2H, m), 7.38∼7.41(3H, m), 7.51∼7.63(11H, m), 7.73∼7.79(5H, m), 7.87~7.93(5H, m), 8(4H, m), 8.1(2H, m), 8.34(2H, m), 8.4(2H, m), 8.99(2H, m) | 891.15 | 890.39 |
| **431** | δ = 1.35(9H, s), 1.72(12H, s), 7.28(2H, m), 7.37∼7.41(7H, m), 7.51∼7.63(8H, m), 7.77∼7.79(4H, m), 7.87∼7.93(4H, m), 8(2H, m), 8.1(2H, m), 8.34(2H, m), 8.4(2H, m), 8.99(2H, m) | 897.19 | 896.44 |
| **440** | δ = 7.41(1H, m), 7.51~7.61(10H, m), 7.73∼7.79(3H, m), 7.92∼7.93(2H, m), 8∼8.1(8H, m), 8.34(2H, m), 8.4∼8.42(3H, m), 8.55(1H, m), 8.99(2H, m) | 632.79 | 632.25 |
| **452** | δ = 1.72(6H, s), 7.28(1H, m), 7.38∼7.41(2H, m), 7.51∼7.63(12H, m), 7.77∼7.79(3H, m), 7.87∼7.93(2H, m), 8∼8.1(8H, m), 8.34(2H, m), 8.4∼8.42(4H, m), 8.55(2H, m), 8.99(2H, m) | 835.04 | 824.34 |
| **456** | δ = 2.34(6H, s), 7.31(1H, m), 7.41(1H, m), 7.51∼7.61(12H, m), 7.79(2H, m), 8∼8.1(8H, m), 8.34(2H, m), 8.4∼8.42(4H, m), 8.55(2H, m), 8.99(2H, m) | 736.94 | 736.31 |
| **464** | δ = 7.41(1H, m), 7.51-7.61(7H, m), 7.79∼7.93(8H, m), 8∼8.12(6H, m), 8.4∼8.42(3H, m), 8.55(1H, m) | 530.66 | 530.20 |
| **465** | δ = 7.41(1H, m), 7.51(2H, m), 7.59(2H, m), 7.79∼7.93(13H, m), 8(2H, m), 8.12(4H, m), 8.4(2H, m), 8.93(2H, m) | 580.71 | 580.22 |
| **478** | δ = 7.25∼7.33(3H, m), 7.41(1H, m), 7.5∼7.51(3H, m), 7.59∼7.63(3H, m), 7.7(1H, m), 7.79∼7.8(4H, m), 7.9∼8(6H, m), 8.1∼8.12(3H, m), 8.4(2H, m), 8.55(1H, m) | 569.69 | 569.21 |
| **491** | δ = 7.39∼7.41(6H, m), 7.51(4H, m), 7.59~7.61(4H, m), 7.79(4H, m), 7.91∼8(6H, m), 8.4(2H, m) | 530.66 | 530.20 |
| **497** | δ = 2.34(6H, s), 7.31(1H, m), 7.39∼7.41(5H, m), 7.51(2H, m), 7.59∼7.61(6H, m), 7.79(2H, m), 7.91∼8(6H, m), 8.4(2H, m) | 558.71 | 558.23 |
| **506** | δ = 7.41(2H, m), 7.51∼7.52(6H, m), 7.59(2H, m), 7.79∼7.81(3H, m), 8(2H, m), 8.06(1H, m), 8.22(2H, m), 8.4(2H, m), 8.57(2H, m) | 458.55 | 458.18 |
| **514** | δ = 2.34(6H, s), 7.31(1H, m), 7.41(1H, m), 7.51(2H, m), 7.59∼7.6(4H, m), 7.79∼7.81(3H, m), 8(2H, m), 8.06(1H m), 8.22(2H, m), 8.4(2H, m), 8.57(2H, m) | 486.61 | 486.21 |
| **518** | δ = 7.41(3H, m), 7.51∼7.52(10H, m), 7.59(2H, m), 7·66(3H, m), 7.79∼7.81(3H, m), 8(2H, m), 8.06(1H, m), 8.22(2H, m), 8.4(2H, m), 8.57(2H, m) | 610.74 | 610.24 |
| **523** | δ = 1.72(6H, s), 7.41(1H, m), 7.51∼7.63(9H, m), 7.77∼7.79(4H, m), 7.93(2H, m), 8∼8.08(4H, m), 8.4∼8.42(3H, m), 8.55(1H, m) | 522.68 | 522.23 |
| **533** | δ = 1.72(12H, s), 7.41(2H, m), 7.51∼7.52(6H, m), 7.59∼7.63(3H, m), 7.69(2H, s), 7.69(0H, m), 7.77(2H, s), 7.77∼7.83(3H, m), 7.93(1H, m), 8(2H, m), 8.15(1H, m), 8.4(2H, m) | 588.78 | 588.28 |
| **536** | δ =1.72(12H, s), 7.41(1H, m), 7.51∼7.63(8H, m), 7.69(2H, s), 7.69(0H, m), 7.77(2H, s), 7.77∼7.83(3H, m), 7.93(1H, m), 8∼8.08(4H, m), 8.15(1H, m), 8.4∼8.42(3H, m), 8.55(1H, m) | 638.84 | 638.30 |
| **553** | δ = 2.34(6H, s), 7.19(1H, m), 7.31(1H, m), 508.65 7.41(1H, m), 7.48~7.51(3H, m), 7.58∼7.63(6H, m), 7.79∼7.8(4H, m), 7.9∼8(4H, m), 8.4(2H, m) | 508.65 | 508.22 |
| **560** | δ = 7.19∼7.25(5H, m), 7.41∼7.52(11H, m), 7.58∼7.63(4H, m), 7.79∼8(10H, m), 8.4(2H, m) | 632.79 | 632.25 |
| **564** | δ = 7.41(1H, m), 7.51∼7.61(7H, m), 7.79∼7.88(4H, m), 8∼8.12(7H, m), 8.18(1H, m), 8.34(1H, m), 8.4-8.42(3H, m), 8.55(1H, m), 8.93(1H m), 8.99(1H, m), 9.15(1H, m) | 556.59 | 556.22 |
| **571** | δ = 7.25(4H, m), 7.41(1H, m), 7.51∼7.61(7H, m), 7.79∼7.88(4H, m), 8∼8.12(7H, m), 8.18(1H, m), 8.34(1H, m), 8.4∼8.42(3H, m), 8.55(1H, m), 8.93(1H, m), 8.99(1H, m), 9.15(1H, m) | 632.79 | 632.25 |
| **573** | δ = 7.41(1H, m), 7.51(2H, m), 7.58∼7.59(5H, m), 7.73∼7.92(8H, m), 7.93(1H, s), 8(4H, m), 8.12(2H, m), 8.4(2H, m), 8.93(2H, m), 9.15 (1H, s) | 556.69 | 556.22 |
| **576** | δ = 7.41(3H, m), 7.51∼7.52(10H, m), 7.59(2H, m), 7.66(3H, m), 7.79∼7.88(6H, m), 7.93(1H, s), 8(2H, m), 8.12(2H, m), 8.4(2H, m), 8.93(2H, m), 9.15(1H, s) | 658.83 | 658.27 |
| **591** | δ = 7.39~7.41(5H, m), 7.51(2H, m), 7.59(2H, m), 7.79~7.93(11H, m), 8(2H, m), 8.12(2H, m), 8.31(2H, m), 8.4(2H, m), 8.93(2H, m) | 606.75 | 606.23 |
| **598** | δ = 7.39~7.41(6H, m), 7.51(4H, m), 7.59(4H, m), 7.79(4H, m), 7.91(4H, m), 8(4H, m), 8.31(2H, m), 8.4(4H, m) | 632.79 | 632.25 |
| **608** | δ = 7.39∼7.41(4H, m), 7.51(2H, m), 7.58∼7.59(5H, m), 7.73∼7.92(9H, m), 8(4H, m), 8.12(1H, m), 8.4(2H, m), 8.93(1H, m) | 556.69 | 556.22 |
| **621** | δ = 7.41(1H, m), 7.51(2H, m), 7.58∼7.59(7H, m), 7.73∼7.92(10H, m), 8(4H, m), 8.4(2H, m) | 530.66 | 530.20 |
| **630** | 5 = 7.21(1H, m), 7.41(3H, m), 7.51(6H, m), 7.58∼7.59(4H, m), 7.76∼7.91(14H, m), 8(2H, m), 8.4(2H, m) | 632.79 | 632.25 |
| **637** | δ = 7.39∼7.41(3H, m), 7.51(2H, m), 7.58∼7.59(5H, m), 7.73∼7.92(10H, m), 8(4H, m), 8.12(2H, m), 8.4(2H, m), 8.93(2H, m) | 606.75 | 606.23 |
| **638** | δ = 7.39~7.41(3H, m), 7.51(2H, m), 7.59(2H, m), 7.79∼7.93(13H, m), 8(2H, m), 8.12(4H, m), 8.4(2H, m), 8.93(4H, m) | 656.81 | 656.25 |
| **653** | 5 = 7.41~7.51(6H, m), 7.58∼7.59(7H, m), 7.69∼7.79(6H, m), 7.87∼7.92(2H, m), 8(5H, m), 8.18(1H, m), 8.4(2H, m) | 571.71 | 571.23 |
| **658** | δ = 7.41(1H, m), 7.51(2H, m), 7.58∼7.59(5H, m), 7.73∼7.79(3H, m), 7.86∼7.92(3H, m), 8(8H, m), 8.4(2H, m) | 512.66 | 512.16 |
| **660** | δ = 7.36∼7.41(4H, m), 7.48∼7.52(8H, m), 7.59(2H, m), 7.78∼7.79(5H, m), 8∼8.08(6H, m), 8.4(2H, m) | 538.62 | 538.19 |
| **667** | 5 = 7.41(1H, m), 7.51(2H, m), 7.59(2H, m), 7.71∼7.72(4H, m), 7.79∼7.93(9H, m), 8(2H, m), 8.12(2H, m), 8.4(2H, m), 8.93(2H, m) | 546.66 | 546.20 |
| **670** | δ = 1.3(4H, m), 1.45(4H, m), 7.41(1H, m), 7.51(2H, m), 7.58∼7.59(7H, m), 7.73∼7.85(7H, m), 7.92(1H, m), 8(4H, m), 8.4(2H, m) | 564.79 | 564.23 |
| **672** | δ = 7.39∼7.41(4H, m), 7.51∼7.52(6H, m), 7.58∼7.59(8H, m), 7.73∼7.79(4H, m), 7.91∼7.92(4H, m), 8(6H, m), 8.1(2H, m), 8.34(2H, m), 8.4(2H, m), 8.99(2H, m) | 809.00 | 808.31 |
| **686** | δ = 2.88(4H, m), 6.63(2H, m), 6.76∼6.84(5H, m), 7.1(2H, m), 7.2(2H, m), 7.41(1H, m), 599.76 7.51(2H, m), 7.58∼7.59(5H, m), 7.73∼7.79(3H, m), 7.92(1H, m), 8(4H, m), 8.4(2H, m) | 599.76 | 599.26 |
| **693** | δ = 7.41(4H, m), 7.51(8H, m), 7.59(8H, m), 7.71(4H, m), 7.79(8H, m), 8(8H, m), 8.26(2H, s), 8.4(8H, m) | 1011.25 | 1010.39 |
| **694** | δ = 6.63(2H, s), 6.81(1H, m), 6.89(2H, m), 6.99∼7(4H, m), 7.2(2H, m), 7.31(2H, m), 7.41(1H, m), 7.51(2H, m), 7.58∼7.59(5H, m), 7.73∼7.79(3H, m), 7.92(1H, m), 8(4H, m), 8.4(2H, m) | 597.75 | 597.25 |
| **695** | δ = 7.41(2H, m), 7.51∼7.52(6H, m), 7.59(2H, m), 7.71(4H, m), 7.79(2H, m), 8(2H, m), 8.1(4H, m), 8.34(4H, m), 8.4(2H, m), 8.99(4H, m) | 632.79 | 632.25 |
| **699** | 5 = 7.25(4H, m), 7.41(1H, m), 7.51(2H, m), 7.58∼7.59(7H, m), 7.71∼7.79(7H, m), 7.92(3H, m), 8(4H, m), 8.1(2H, m), 8.34(2H, m), 8.4(2H, m), 8.99(2H, m) | 708.89 | 708.28 |
| **701** | δ = 7.41(1H, m), 7.51(2H, m), 7.59(2H, m), 7.71(8H, m), 7.79∼7.88(4H, m), 8(2H, m), 8.1∼8.12(8H, m), 8.34(7H, m), 8.4(2H, m), 8.93(1H, m), 8.99(7H, m) | 909.12 | 908.34 |
| **702** | δ = 7.4(6H,m), 7.5(6H,m), 7.6(2H,m), 7.8(2H,m), 7.9(4H,m), 8.0(2H,m), 8.4(2H,m) | 456.58 | 456.19 |
| **703** | δ = 7.4(5H,m), 7.5(2H,m), 7.6(5H,m), 7.7(1H,m), 7.8(2H,m), 7.9(5H,m), 8.0(4H,m), 8.4(2H,m) | 506.63 | 506.20 |
| **704** | δ = 1.7(6H,s), 7.3(1H,m), 7.4(6H,m), 7.5(2H,m), 7.6(4H,m), 7.8(3H,m), 7.9(6H,m), 8.0(2H,m), 8.4(2H,m) | 572.74 | 572.25 |
| **705** | δ = 7.3(4H,m), 7.4(6H,m), 7.5(8H,m), 7.6(2H,m), 7.8(2H,m), 7.9(6H,m), 8.0(2H,m), 8.4(2H,m) | 608.77 | 608.25 |
| **706** | δ = 7.3(4H,m), 7.4(6H,m), 7.5(6H,m), 7.6(2H,m), 7.8(2H,m), 7.9(4H,m), 8.0(2H,m), 8.4 (2H,m) | 532.67 | 532.22 |
| **707** | δ = 7.4(5H,m), 7.5(2H,m), 7.6(2H,m), 7.8(4H,m), 7.9(7H,m), 8.0(2H,m), 8.1(2H,m), 8.4(2H,m), 8.9(2H,m) | 566.69 | 566.22 |
| **708** | δ = 2.3(6H,m), 7.3(1H,m), 7.4(5H,m), 7.5(2H,m), 7.6(4H,m), 7.8(2H,m), 7.9(4H,m), 8.0(2H,m), 8.4(2H,m) | 484.63 | 484.22 |
| **709** | δ =7.4(7H,m), 7.5(10H,m), 7.6(2H,m), 7.7(3H,m), 7.8(2H,m), 7.9(4H,m), 8.0(2H,m), 8.4(2H,m) | 608.77 | 608.25 |
| **710** | δ = 1.4(9H,s), 7.4(9H,m), 7.5(2H,m), 7.6(2H,m), 7.8(2H,m), 7.9(4H,m), 8.0(2H,m), 8.4 (2H,m) | 512.68 | 512.25 |
| **711** | δ = 7.4(6H,m), 7.5(8H,m), 7.6(3H,m), 7.7(1H,m), 7.8(2H,m), 7.9(4H,m), 8.0(2H,m), 8.4(2H,m) | 532.67 | 532.22 |
| **712** | δ = 7.4(6H,m), 7.5(6H,m), 7.6(2H,m), 7.8(4H,m), 7.9(6H,m), 8.0(2H,m), 8.4(2H,m) | 532.67 | 532.22 |
| **713** | δ = 7.4(6H,m), 7.5(2H,m), 7.6(3H,m), 7.8(3H,m), 7.9(4H,m), 8.0(2H,m), 8.1(2H,m), 8.4(3H,m), 8.8(1H,m) | 558.67 | 558.21 |
| **714** | δ = 7.4(6H,m), 7.5(2H,m), 7.6(3H,m), 7.8(3H,m), 7.9(4H,m), 8.0(3H,m), 8.1(2H,m), 8.4(2H,m) | 507.62 | 507.20 |
| **715** | δ= 7.4(5H,m), 7.5(2H,m), 7.6(3H,m), 7.7(1H,m 7.8(2H,m), 7.9(4H,m), 8.0(3H,m), 8.2(1H,m), 8.4(3H,m), 8.8(1H,m) | 507.62 | 507.20 |
| **716** | δ = 7.3(3H,m), 7.4(5H,m), 7.5(3H,m), 7.6(3H,m), 7.8(2H,m), 7.9(5H,m), 8.0(2H,m), 8.1(1H,m), 8.4(2H,m), 8.6(1H,m) | 545.67 | 545.21 |
| **721** | δ = 2.5(3H,s), 7.3(5H,m), 7.4(4H,m), 7.5(6H,m), 7.6(2H,m), 7.7(1H,m 7.8(2H,m), 7.9(3H,m), 8.0(2H,m), 8.4(2H,m) | 546.70 | 546.23 |
| **727** | δ = 2.5(3H,s), 7.3(1H,m), 7.4(4H,m), 7.5(6H,m), 7.6(2H,m), 7.7(1H,m), 7.8(4H,m), 7.9(5H,m), 8.0(2H,m), 8.4(2H,m) | 546.70 | 546.23 |
| **739** | δ = 2.5(6H,s), 7.3(2H,m), 7.4(3H,m), 7.5(10H,m), 7.6(2H,m), 7.7(5H,m), 7.8(2H,m), 7.9(2H,m), 8.0(2H,m), 8.4(2H,m) | 636.82 | 636.28 |
| **740** | δ = 1.4(9H,s), 2.5(6H,s), 7.3(2H,m), 7.4(5H,m), 7.5(2H,m), 7.6(2H,m), 7.7(2H,m), 7.8(2H,m), 7.9(2H,m), 8.0(2H,m), 8.4(2H,m) | 540.74 | 540.28 |
| **758** | δ = 2.5(6H,s), 7.4(4H,m), 7.5(2H,m), 7.6(5H,m), 7.8(3H,m), 7.9(2H,m), 8.0(2H,m), 8.1(2H,m), 8.4(3H,m), 8.8(1H,m) | 586.72 | 586.24 |
| **759** | δ = 2.5(6H,s), 7.4(4H,m), 7.5(2H,m), 7.6(5H,m), 7.8(3H,m), 7.9(2H,m), 8.0(3H,m), 8.1(2H,m), 8.4(2H,m) | 535.68 | 535.23 |
| **775** | δ = 2.5(9H,s), 7.3(1H,m), 7.4(1H,m), 7.5(2H,m), 7.6(5H,m), 7.7(2H,m), 7.8(2H,m), 7.9(1H,m), 8.0(3H,m), 8.2(1H,m), 8.4(3H,m), 8.8(1H,m) | 549.70 | 549.25 |
| **776** | δ = 2.5(9H,s), 7.3(4H,m), 7.4(1H,m) 7.5(3H,m), 7.6(5H,m), 7.7(1H,m), 7.8(3H,m), 7.9(1H,m), 8.0(2H,m), 8.1(1H,m), 8.4(2H,m), 8.6(1H,m) | 587.75 | 587.26 |
| **777** | δ= 2.5(12H,s), 7.4(2H,m), 7.5(6H,m), 7.59(2H,m), 7.64(4H,s), 7.8(2H,m), 8.0(2H,m), 8.4(2H,m) | 512.68 | 512.25 |
| **778** | δ = 2.5(12H,s), 7.4(1H,m) 7.5(2H,m), 7.6(5H,m), 7.64(4H,s), 7.7(1H,m), 7.8(2H,m), 7.9(1H,m), 8.0(4H,m), 8.4(2H,m) | 562.74 | 562.27 |
| **792** | δ = 7.4(5H,m), 7.5(2H,m), 7.6(5H,m), 7.8(2H,m), 7.9(4H,m), 8.0(3H,m), 8.1(1H,m), 8.4(3H,m), 8.6(1H,m) | 506.63 | 506.20 |
| **793** | δ = 7.3(2H,m), 7.4(5H,m), 7.5(4H,m), 7.6(2H,m), 7.8(2H,m), 7.9(6H,m), 8.0(3H,m), 8.2(1H,m), 8.4(2H,m) | 589.75 | 589.19 |
| **794** | δ = 7.4(6H,m), 7.5(4H,m), 7.6(4H,m), 7.8(4H,m), 7.9(4H,m), 8.0(4H,m), 8.4(4H,m) | 582.73 | 582.23 |
| **795** | δ = 7.2(2H,m), 7.3(2H,m), 7.4(5H,m), 7.5(5H,m), 7.6(5H,m), 7.8(2H,m), 7.9(6H,m), 8.0(2H,m), 8.4(2H,m), 8.6(1H,m) | 648.79 | 648.26 |
| **796** | δ = 7.4(6H,m), 7.5(4H,m), 7.6(4H,m), 7.8(4H,m), 7.9(4H,m), 8.0(3H,m), 8.1(2H,m), 8.4(2H,m), 8.6(1H,m) | 582.73 | 582.23 |
| **800** | δ = 7.4(9H,m), 7.5(2H,m), 7.6(5H,m), 7.8(2H,m), 7.9(8H,m), 8.0(3H,m), 8.1(1H,m), 8.4(3H,m), 8.6(1H,m) | 682.85 | 682.27 |
| **801** | δ = 1.7(6H,s), 7.3(1H,m), 7.4(10H,m), 7.5(2H,m), 7.6(4H,m), 7.8(3H,m), 7.9(10H,m), 8.0(2H,m), 8.4(2H,m) | 748.95 | 748.31 |
| **802** | δ = 7.3(4H,m), 7.4(10H,m), 7.5(8H,m), 7.6(2H,m), 7.8(2H,m), 7.9(10H,m), 8.0(2H,m), 8.4(2H,m) | 784.98 | 784.31 |
| **808** | δ = 7.4(10H,m), 7.5(6H,m), 7.6(2H,m), 7.8(4H,m), 7.9(10H,m), 8.0(2H,m), 8.4(2H,m) | 708.89 | 708.28 |
| **818** | δ = 7.4(14H,m), 7.5(6H,m), 7.6(2H,m), 809.00 7.8(2H,m), 7.9(12H,m), 8.0(2H,m), 8.4(2H,m) | 809.00 | 808.31 |
| **819** | δ = 7.4(13H,m), 7.5(2H,m), 7.6(5H,m), 7.7(1H,m), 7.8(2H,m), 7.9(13H,m), 8.0(4H,m), 8.4 (2H,m) | 859.06 | 858.33 |
| **826** | δ = 7.4(18H,m), 7.5(6H,m), 7.6(2H,m). 7.8(2H,m), 7.9(16H,m), 8.0(2H,m), 8.4(2H,m) | 985.22 | 984.38 |
| **827** | δ = 7.3(4H,m), 7.4(5H,m), 7.5(2H,m), 7.6(5H,m), 7.7(1H,m), 7.8(2H,m), 7.9(5H,m), 8.0(4H,m), 8.4(2H,m) | 582.73 | 582.23 |
| **829** | δ = 1.7(6H,s), 7.3(5H,m), 7.4(6H,m), 7.5(2H,m), 7.6(4H,m), 7.8(3H,m), 7.9(6H,m), 8.0(2H,m), 8.4(2H,m) | 648.83 | 648.28 |
| **831** | δ = 7.3(4H,m), 7.4(5H,m), 7.5(2H,m), 7.6(5H,m), 7.8(2H,m), 7.9(4H,m), 8.0(3H,m), 8.1(1H,m), 8.4(3H,m), 8.6(1H,m) | 582.73 | 582.23 |
| **833** | δ = 7.4(6H,m), 7.5(4H,m), 7.6(4H,m), 7.8(4H,m), 7.9(4H,m), 8.0(4H,m), 8.4(2H,m), 8.6(2H,m) | 582.73 | 582.23 |
| **835** | δ = 7.4(6H,m), 7.5(6H,m), 7.6(4H,m), 7.7(2H,m), 7.8(2H,m), 7.9(6H,m), 8.0(2H,m), 8.4(2H,m) | 582.73 | 582.23 |
| **837** | δ = 7.4(5H,m), 7.5(2H,m), 7.6(7H,m), 7.7(2H,m), 7.8(2H,m), 7.9(6H,m), 8.0(3H,m), 8.1(1H,m), 8.4(3H,m), 8.6(1H,m) | 632.79 | 632.25 |
| **838** | δ = 7.3(4H,m), 7.4(6H,m), 7.5(4H,m), 7.6(4H,m), 7.8(4H,m), 7.9(4H,m), 8.0(4H,m), 8.4(2H,m), 8.6(2H,m) | 658.83 | 658.27 |
| **844** | δ = 1.7(6H,s), 7.3(1H,m), 7.4(6H,m), 7.5(4H,m), 7.6(5H,m), 7.7(1H,m), 7.8(3H,m), 7.9(6H,m), 8.0(2H,m), 8.4(2H,m) | 648.83 | 648.28 |
| **849** | δ = 1.7(6H,s), 7.3(1H,m), 7.4(6H,m), 7.5(4H,m), 7.6(5H,m), 7.7(1H,m), 7.8(3H,m), 7.9(6H,m), 8.0(2H,m), 8.4(2H,m) | 648.83 | 648.28 |
| **852** | δ = 1.7(6H,s), 7.3(1H,m), 7.4(6H,m), 7.5(2H,m), 7.6(4H,m), 7.8(3H,m), 7.9(6H,m), 8.0(2H,m), 8.0(2H,m) | 572.74 | 572.25 |
| **866** | δ = 7.4(4H,m), 7.5(6H,m), 7.6(6H,m), 7.7(1H,m), 7.8(2H,m), 7.9(3H,m), 8.0(5H,m), 8.1(1H,m), 8.4(2H,m) | 582.73 | 582.23 |
| **887** | δ = 7.4(5H,m), 7.5(10H,m), 7.6(6H,m), 7.7(4H,m), 7.8(2H,m), 7.9(3H,m), 8.0(5H,m), 8.1(1H,m), 8.4(2H,m) | 734.92 | 734.30 |
| **894** | δ = 7.3(2H,m), 7.4(5H,m), 7.5(4H,m), 7.6(2H,m), 7.7(1H,m), 7.8(3H,m), 7.9(6H,m), 8.0(3H,m), 8.4(2H,m) | 588.76 | 588.19 |
| **897** | 5 = 6.8(1H,m), 6.9(1H,m) 7.3(3H,m), 7.4(5H,m), 7.5(5H,m), 7.6(4H,m), 7.8(2H,m), 7.9(6H,m), 8.0(2H,m), 8.3(2H,m), 8.4(2H,m) | 647.80 | 647.26 |
| **900** | 7.4(4H,m), 7.5(14H,m), 7.6(4H,m), 7.8(2H,m), 8.0(4H,m), 8.1(2H,m), 8.4(2H,m) | 608.77 | 608.25 |
| **901** | δ = 7.25(8H,m), 7.39(8H,m), 7.41(2H,m), 7.51(4H,m), 7.52(4H,m), 7.59(4H,m), 7.91(8H,m), 8(4H,m), 8.4(4H,m) | 910.36 | 910.14 |
| **904** | δ = 7.3(4H,m), 7.4(9H,m), 7.5(4H,m), 7.6(5H,m), 7.7(1H,m), 7.9(9H,m), 8.0(4H,m), 8.4(2H,m) | 758.94 | 758.30 |
| **907** | δ = 7.3(4H,m), 7.4(10H,m), 7.5(6H,m), 7.6(2H,m), 7.8(2H,m), 7.9(8H,m), 8.0(2H,m), 8.4(2H,m) | 708.89 | 708.28 |
| **908** | δ = 7.3(4H,m), 7.4(9H,m), 7.5(2H,m), 7.6(5H,m), 7.7(1H,m), 7.8(2H,m), 7.9(9H,m), 8.0(4H,m), 8.4(2H,m) | 758.94 | 758.30 |
| **909** | δ = 1.7(6H,s), 7.3(5H,m), 7.4(10H,m), 7.5(2H,m), 7.6(4H,m), 7.8(3H,m), 7.9(10H,m), 8.0(2H,m), 8.4(2H,m) | 824.34 | 824.04 |
| **911** | δ = 1.7(6H,s), 7.3(4H,m), 7.4(10H,m), 7.5(6H,m), 7.6(4H,m), 7.8(4H,m), 7.9(10H,m), 8.0(2H,m), 8.4(2H,m) | 901.14 | 900.38 |
| **912** | δ = 1.7(6H,s), 7.4(10H,m), 7.5(6H,m), 7.6(4H,m), 7.8(4H,m), 7.9(10H,m), 8.0(2H,m), 8.4(2H,m) | 825.04 | 824.34 |
| **913** | δ = 1.7(6H,s), 7.4(9H,m), 7.5(2H,m), 7.6(7H,m), 7.8(4H,m), 7.9(10H,m), 8.0(3H,m), 8.1(1H,m), 8.4(3H,m), 8.6(1H,m) | 875.10 | 874.36 |
| **914** | δ = 7.4(10H,m), 7.5(8H,m), 7.6(3H,m), 7.7(1H,m), 7.8(2H,m), 7.9(8H,m), 8.0(2H,m), 8.4(2H,m) | 708.89 | 708.28 |
| **916** | δ = 1.7(6H,s), 7.3(8H,m), 7.4(10H,m), 7.5(6H,m), 7.6(4H,m), 7.8(4H,m), 7.9(10H,m), 8.0(2H,m), 8.4(2H,m) | 977.24 | 976.41 |
| **917** | δ = 7.4(3H,m), 7.5(10H,m), 7.6(7H,m), 7.7(1H,m) 7.8(2H,m), 7.9(1H,m) 8.0(6H,m), 8.1(2H,m), 8.4(2H,m) | 658.83 | 658.27 |
| **919** | δ = 7.25(4H, m), 7.41(4H, m), 7.51-7.52(14H, m), 7.59∼7.61(4H, m), 7.79(2H, m), 7.97∼8(4H, m), 8.13(2H, m), 8.4(2H, m) | 684.86 | 684.28 |
| **922** | 5 = 2.18(3H, s), 2.34(6H, s), 7.39(4H, m), 7.48(2H, m), 7.58∼7.59(5H, m), 7.73(1H m), 7.91∼7.92(5H, m), 8(4H, m), 8.4(2H, m) | 548.71 | 548.25 |
| **928** | δ = 2.34(6H, s), 7.31(1H, m), 7.39(4H, m), 7.58∼7.6(7H, m), 7.73(1H, m), 7.91∼7.92(5H,m), 8(4H, m), 8.4(2H, m) | 534.69 | 534.23 |
| **934** | δ = 7.25(2H, m), 7.39∼7.41(5H, m), 7.51∼7.59(6H, m), 7.79∼7.85(4H, m), 7.91(4H,m), 8∼8.01(3H, m), 8.18(1H, m), 8.4(2H, m) | 589.75 | 589.19 |
| **939** | δ = 2.88(4H, m), 6.58(2H, m), 6.69(2H, m), 6.76(2H, m), 7.02∼7.04(4H, m), 7.39∼7.41(5H, m), 7.51∼7.59(6H, m), 7.79(2H, m), 7.91(4H, m), 8(2H, m), 8.4(2H, m) | 649.82 | 649.28 |

### [Example 1] Manufacture of an OLED (1)

An OLED device was manufactured by using an organic electroluminescent compound according to the invention.

First, a transparent electrode ITO thin film (15 Ω/□) (2) prepared from glass for OLED (produced by Samsung Corning) (1) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injection layer (3) having 60 nm of thickness on the ITO substrate.

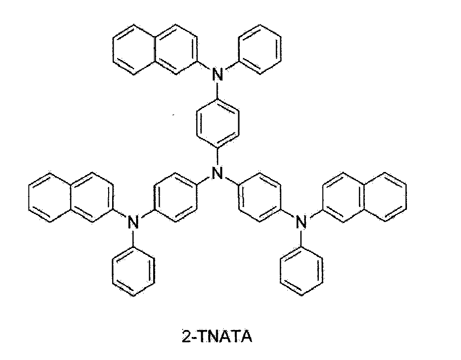

Then, to another cell of the vacuum vapor-deposit device, charged was N,N'-bis(α-naphthyl)-N,N'-diphenyl-4,4'-diamine (NPB), and electric current was applied to the cell to evaporate NPB, thereby providing vapor-deposit of a hole transport layer (4) of 20 nm of thickness on the hole injection layer.

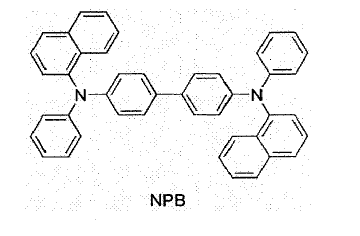

After forming a hole injection layer and hole transport layer, an electroluminescent layer was vapor-deposited thereon as follows.

To one cell of a vacuum vapor-deposit device, charged was a compound according to the present invention (for example, Compound 2) as an electroluminescent material, and DSA-Ph (of which the structure is shown below) was charged to another cell. The two cells were simultaneously heated to vapor-deposit an electroluminescent layer (5) having 30 nm of thickness on the hole transport layer with the vapor-deposition rate of DSA-Ph of 2 to 5% by weight.

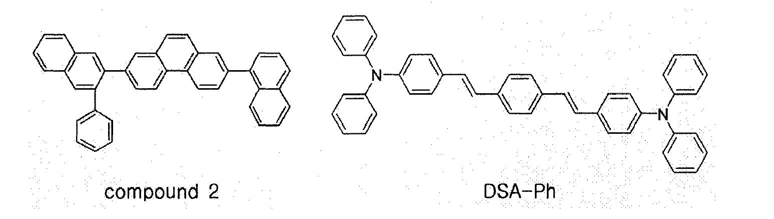

Tris(8-hydroxyquinoline)aluminum (III) (Alq) was then vapor-deposited as an electron transport layer (6) with a thickness of 20 nm. Thereafter, lithium quinolate (Liq) was vapor-deposited as an electron injection layer (7) with a thickness of 1 to 2 nm. An Al cathode (8) was vapor-deposited thereon with a thickness of 150 nm by using another vacuum vapor-deposit device to manufacture an OLED.

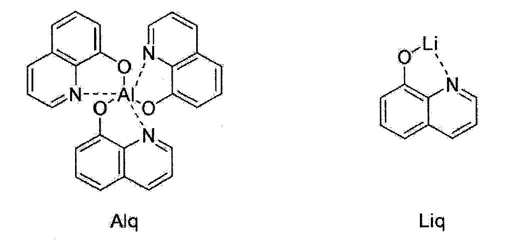

Each material was purified via vacuum sublimation at 10⁻⁶ torr before being used as electroluminescent material for an OLED.

### [Example 2] Manufacture of an OLED by using a compound according to the invention

After forming a hole injection layer and a hole transport layer according to the same procedure as Example 1, a compound according to the present invention (for example, Compound 705) was charged to one cell of said vacuum vapor-deposition device, while Compound (E) (of which the structure is shown below) was charged to another cell. The two materials were evaporated at different rates to give doping at a concentration of 2 to 5% by weight on the basis of the host, thereby vapor-depositing an electroluminescent layer with a thickness of 30 nm on the hole transport layer.

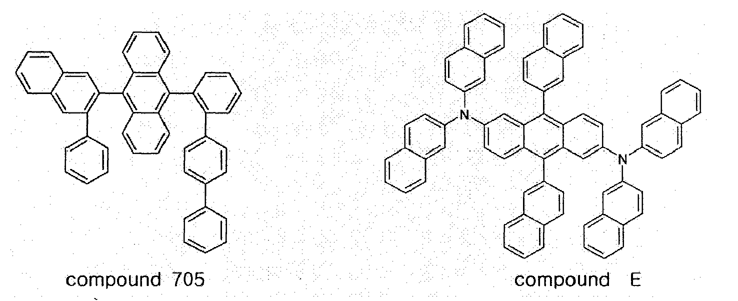

Then, an electron transport layer (6) and an electron injection layer (7) were vapor-deposited according to the same procedure of Example 1, and Al cathode (8) was vapor-deposited by using another vacuum vapor-deposit device with a thickness of 150 nm, to manufacture an OLED.

### [Example 3] Manufacture of an OLED by using a compound according to the invention

After forming a hole injection layer and a hole transport layer according to the same procedure as Example 1, a compound according to the present invention (for example, Compound 218) was charged to one cell of said vacuum vapor-deposition device, while Compound (A) (of which the structure is shown below) was charged to another cell. The two materials were evaporated at different rates to give doping at a concentration of 2 to 5% by weight on the basis of the host, thereby vapor-depositing an electroluminescent layer with a thickness of 30 nm on the hole transport layer.

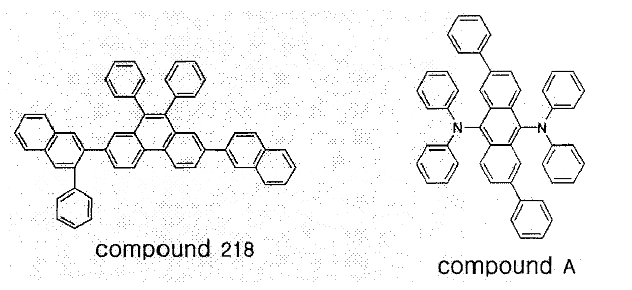

Then, an electron transport layer (6) and an electron injection layer (7) were vapor-deposited according to the same procedure of Example 1, and Al cathode (8) was vapor-deposited by using another vacuum vapor-deposit device with a thickness of 150 nm, to manufacture an OLED.

### [Comparative Example 1] Manufacture of an OLED by using conventional electroluminescent material

After forming a hole injection layer (3) and hole transport layer (4) according to the same procedure described in Example 1, dinaphthylanthracene (DNA) was charged to one cell of said vacuum vapor-deposit device as an electroluminescent material, and DSA-Ph was charged to another cell, as in Example 1. Then an electroluminescent layer (5) having 30 nm of thickness was vapor-deposited on the hole transport layer at the vapor-deposition rate of 100:3.

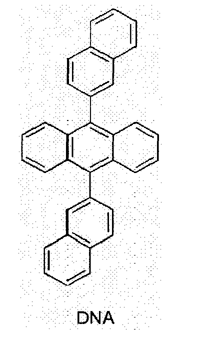

Then, an electron transport layer (6) and an electron injection layer (7) were vapor-deposited according to the same procedure of Example 1, and Al cathode (8) was vapor-deposited by using another vacuum vapor-deposit device with a thickness of 150 nm, to manufacture an OLED.

### [Comparative Example 2] Manufacture of an OLED by using conventional electroluminescent material

After forming a hole injection layer and hole transport layer according to the same procedure described in Example 2, Alq was charged to another cell of said vacuum vapor-deposit device as an electroluminescent host material, while Coumarin 545T (C545T) was charged to still another cell. The two materials were evaporated at different rates to give doping, thereby vapor-depositing an electroluminescent layer with a thickness of 30 nm on the hole transport layer. The doping concentration preferably is from 1 to 3% by weight on the basis of Alq.

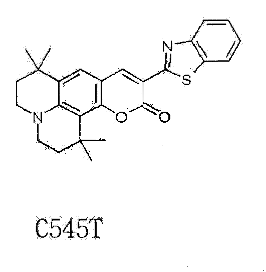

Then, an electron transport layer and an electron injection layer were vapor-deposited according to the same procedure of Example 1, and Al cathode was vapor-deposited by using another vacuum vapor-deposit device with a thickness of 150 nm, to manufacture an OLED.

The luminous efficiencies of an OLED's of Examples 1 to 3 comprising the organic electroluminescent compound according to the invention, and OLED's prepared from Comparative Examples 1 and 2 comprising a conventional electroluminescent compound were measured at 5,000 cd/m², and the results are shown in Table 2.

**Table 2**

| No. | | Host | Dopant | Doping Conc. (wt%) | Luminous efficiency (cd/A) | Color |
|---|---|---|---|---|---|---|
| | | | | | @5000cd/m² | |
| Ex. 1 | 1 | **2** | DSA-Ph | 3 | 8.2 | Blue |
| | 2 | **32** | DSA-Ph | 3 | 7.9 | Blue |
| | 3 | **79** | DSA-Ph | 3 | 7.3 | Blue |
| | 4 | **106** | DSA-Ph | 3 | 7.1 | Blue |
| | 5 | **121** | DSA-Ph | 3 | 7.3 | Blue |
| | 6 | **182** | DSA-Ph | 3 | 7.5 | Blue |
| | 7 | **234** | DSA-Ph | 3 | 7.7 | Blue |
| | 8 | **262** | DSA-Ph | 3 | 7.2 | Blue |
| | 9 | **337** | DSA-Ph | 3 | 7.5 | Blue |
| | 10 | **358** | DSA-Ph | 3 | 7.6 | Blue |
| | 11 | **449** | DSA-Ph | 3 | 7.9 | Blue |
| | 12 | **487** | DSA-Ph | 3 | 7.7 | Blue |
| | 13 | **705** | DSA-Ph | 3 | 7.6 | Blue |
| | 14 | **722** | DSA-Ph | 3 | 7.7 | Blue |
| | 15 | **792** | DSA-Ph | 3 | 7.6 | Blue |
| | 16 | **796** | DSA-Ph | 3 | 7.1 | Blue |
| | 17 | **800** | DSA-Ph | 3 | 7.0 | Blue |
| | 18 | **831** | DSA-Ph | 3 | 7.1 | Blue |
| | 19 | **865** | DSA-Ph | 3 | 6.2 | Blue |
| | 20 | **909** | DSA-Ph | 3 | 6.8 | Blue |
| | 21 | **914** | DSA-Ph | 3 | 7.5 | Blue |
| Ex. 2 | 1 | **6** | Compound E | 3 | 19.5 | Green |
| | 2 | **59** | Compound E | 3 | 19.8 | Green |
| | 3 | **100** | Compound E | 3 | 21.5 | Green |
| | 4 | **142** | Compound E | 3 | 20.2 | Green |
| | 5 | **218** | Compound E | 3 | 19.8 | Green |
| | 6 | **281** | Compound E | 3 | 20.6 | Green |
| | 7 | **346** | Compound E | 3 | 19.9 | Green |
| | 8 | **421** | Compound E | 3 | 20.0 | Green |
| | 9 | **525** | Compound E | 3 | 21.1 | Green |
| | 10 | **610** | Compound E | 3 | 22.0 | Green |
| | 11 | **705** | Compound E | 3 | 22.0 | Green |
| | 12 | **849** | Compound E | 3 | 21.5 | Green |
| | 13 | **897** | Compound E | 3 | 19.8 | Green |
| | 14 | **885** | Compound E | 3 | 20.2 | Green |
| | 15 | **900** | Compound E | 3 | 21.5 | Green |
| Ex. 3 | 1 | **13** | Compound A | 3 | 18.2 | Green |
| | 2 | **21** | Compound A | 3 | 18.6 | Green |
| | 3 | **127** | Compound A | 3 | 18.2 | Green |
| | 4 | **181** | Compound A | 3 | 18.9 | Green |
| | 5 | **218** | Compound A | 3 | 19.3 | Green |
| | 6 | **270** | Compound A | 3 | 19.3 | Green |
| | 7 | **307** | Compound A | 3 | 19.8 | Green |
| | 8 | **354** | Compound A | 3 | 20.1 | Green |
| | 9 | **443** | Compound A | 3 | 19.5 | Green |
| | 10 | **477** | Compound A | 3 | 19.6 | Green |
| | 11 | **525** | Compound A | 3 | 19.2 | Green |
| | 12 | **566** | Compound A | 3 | 19.3 | Green |
| | 13 | **634** | Compound A | 3 | 20.1 | Green |
| | 14 | **798** | Compound A | 3 | 19.9 | Green |
| | 15 | **867** | Compound A | 3 | 19.8 | Green |
| Comp. Ex. 1 | | DNA | DSA-Ph | 3 | 7.3 | Jade green |
| Comp. Ex. 2 | | Alq | Compound C545T | 1 | 10.3 | Green |

As can be seen from Table 2, when the electroluminescent material according to the invention, being doped by the same type of DSA-Ph, was applied to a blue electroluminescent device, color purity was noticeably enhanced while maintaining at least comparable luminous efficiency as compared to DNA as conventional EL material employed in Comparative Example 1.

When the electroluminescent material according the present invention, with being doped by Compound (E) or Compound (A) with 3.0% of doping concentration, was applied to a green electroluminescent device, the luminous efficiency was noticeably improved while maintaining at least comparable color purity as compared to conventional compound, Alq:C545T (Comparative Example 2).

As described above, the organic electroluminescent compounds according to the invention can be used as blue or green electroluminescent material of high efficiency, and the electroluminescent device, to which the host material according to the invention is applied, exhibited noticeable improvement in terms of color purity. The results of improvement in both color purity and luminous efficiency verify excellent features of the material according to the invention.

## Claims

1. An organic electroluminescent compound represented by Chemical Formula (1): 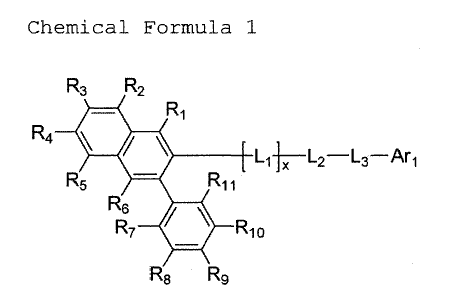 wherein, L₁ represents (C6-C60)arylene or (C3-C60)heteroarylene containing one or more heteroatom(s) selected from N, O and S, or a bivalent group selected from the following structures: 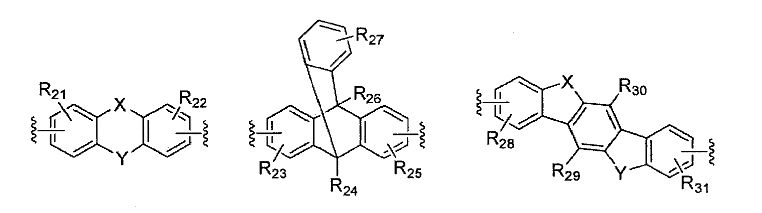 L₂ and L₃ independently represent a chemical bond, or (C1-C60)alkyleneoxy, (C1-C60)alkylenethio, (C6-C60)aryleneoxy, (C6-C60)arylenethio, (C6-C60)arylene or (C3-C60)heteroarylene containing one or more heteroatom(s) selected from N, O and S;
Ar₁ represents NR₄₁R₄₂, (C6-C60)aryl, (C3-C60)heteroaryl containing one or more heteroatom(s) selected from N, O and S, a 5-or 6-membered heterocycloalkyl containing one or more heteroatom(s) selected from N, O and S, (C3-C60)cycloalkyl, adamantyl, (C7-C60)bicycloalkyl, or a substituent selected from the following structures: 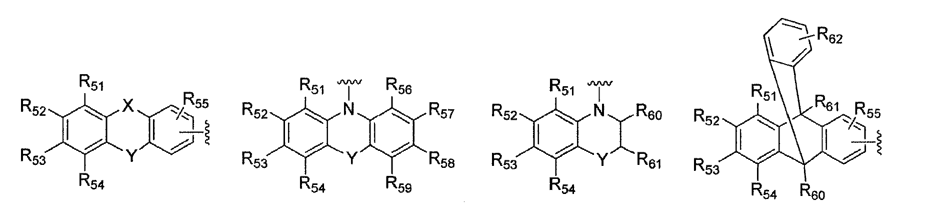 R₁ through R₁₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁ through R₁₁ may be linked to an adjacent substituent via (C3-C60)alkylene or (C3-C60)alkenylene with or without a fused ring to form an alicyclic ring, or a monocyclic or polycyclic aromatic ring;
R₂₁ through R₃₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₂₁ through R₃₁ may be linked to an adjacent substituent via (C3-C60)alkylene or (C3-C60)alkenylene with or without a fused ring to form an alicyclic ring, or a monocyclic or polycyclic aromatic ring;
R₄₁ and R₄₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₄₁ and R₄₂ may be linked via (C3-C60)alkylene or (C3-C60)alkenylene with or without a fused ring to form an alicyclic ring, or a monocyclic or polycyclic aromatic ring;
R₅₁ through R₆₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₅₁ through R₆₂ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or -(CR₇₁R₇₂)m-, -N(R₇₃)-, -S-, -O-, -Si(R₇₄)(R₇₅)-, -P(R₇₆), -C(=O)-, -B(R₇₇)-, -In(R₇₈)-, -Se-, -Ge(R₇₉) (R₈₀)-, -Sn(R₈₁) (R₈₂)-, -Ga(R₈₃)- or - (R₈₄)C=C(R85)-;
R₇₁ through R₈₅ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₇₁ and R₇₂, R₇₄ and R₇₅, R₇₉ and R₈₀, R₈₁ and R₈₂, or R₈₄ and R₈₅ may be linked via (C3-C60)alkylene or (C3-C60)alkenylene with or without a fused ring to form an alicyclic ring, or a monocyclic or polycyclic aromatic ring;
the arylene or heteroarylene of L₁ through L₃, the aryl or heteroaryl of Ar₁, or the alkyl, aryl, heteroaryl, heterocycloalkyl, cycloalkyl, trialkylsilyl, dialkylarylsilyl, triarylsilyl, alkenyl, alkynyl, alkylamino or arylamino of R₁ through R₁₁, R₂₁ through R₃₁, R₄₁, R₄₂, R₅₁ through R₆₂, and R₇₁ through R₈₅ may be further substituted by one or more substituent(s) selected from deuterium, halogen, (C1-C60)alkyl, halo(C1-C60)alkyl, (C6-C60)aryl, (C3-C60)heteroaryl containing one or more heteroatom(s) selected from N, O and S, with or without (C6-C60)aryl substituent(s), morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 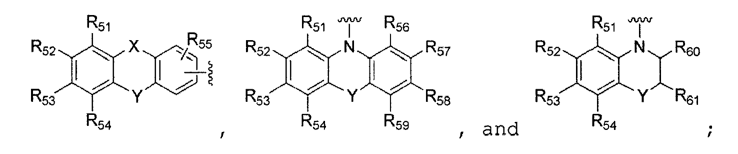 m is an integer from 1 to 4; and
x is an integer from 1 to 4.

2. The organic electroluminescent compound according to claim 1,
wherein L₁ is selected from the following structure: 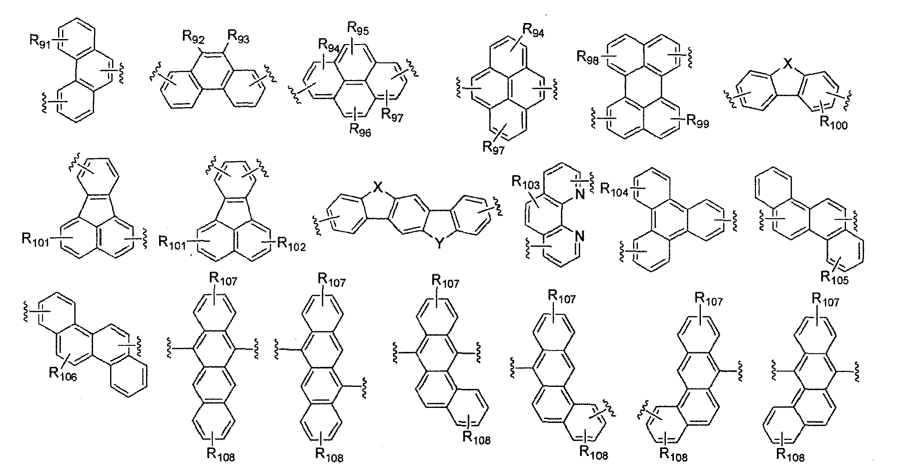 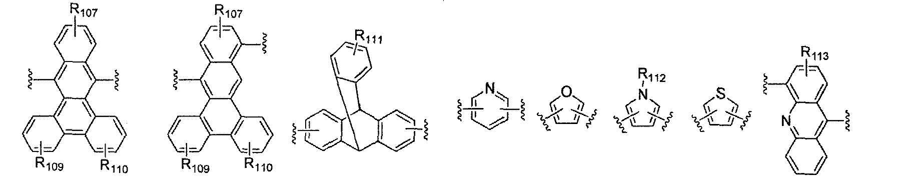 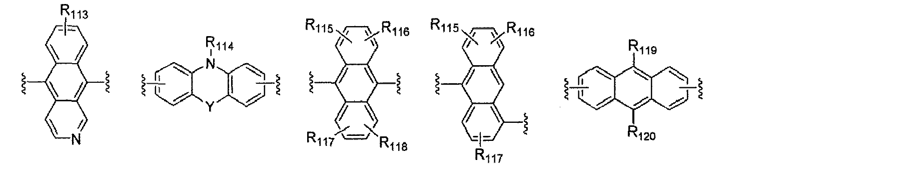 wherein, X and Y independently represent - (CR₇₁R₇₂)ₘ-, -N(R₇₃)-, -S-, -O-, -Si(R₇₄)(R₇₅)-, -P(R₇₆)- or -(R₈₄)C=C(R₈₅)-;
R₇₁ through R₇₆, R₈₄, R₈₅ and m are defined as in claim 1;
R₉₁ through R₁₂₀ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsil tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₉₁ through R₁₂₀ may be linked via (C3-C60)alkylene or (C3-C60)alkenylene with or without a fused ring to form an alicyclic ring, or a monocyclic or polycyclic aromatic ring.

3. The organic electroluminescent compound according to claim 2,
wherein L₁ is selected from the following structure: 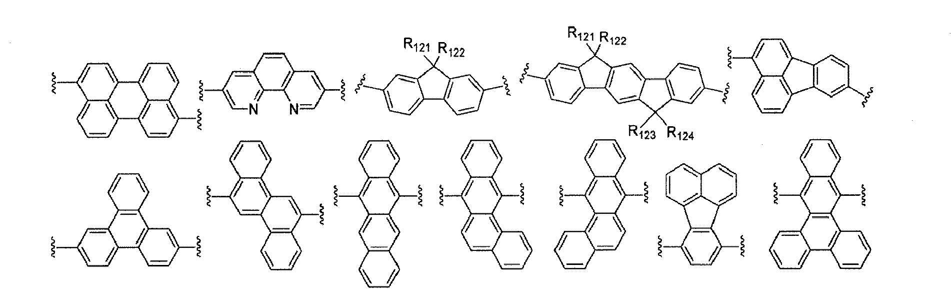 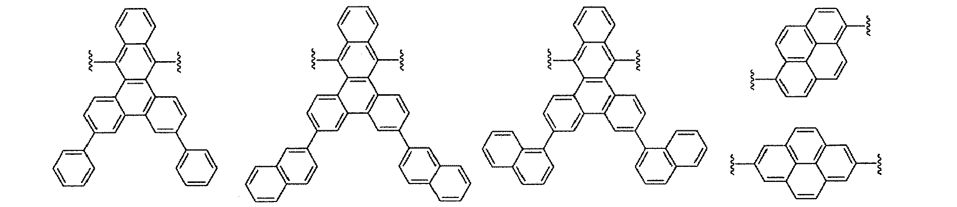 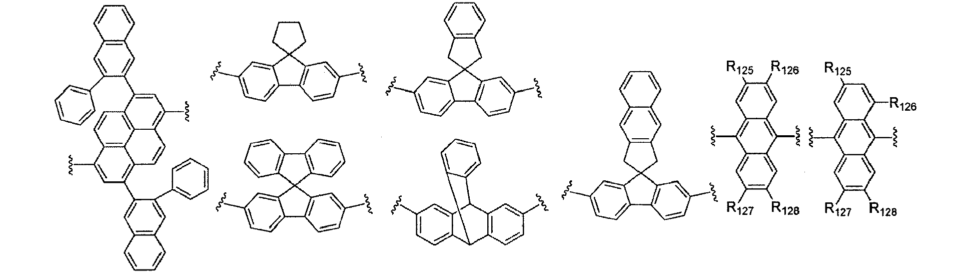 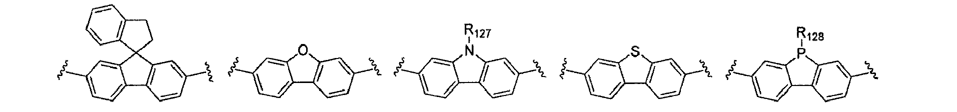 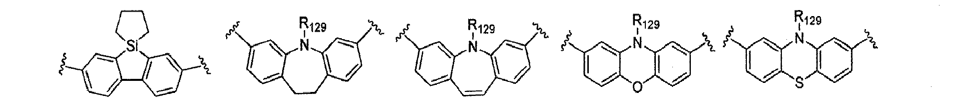 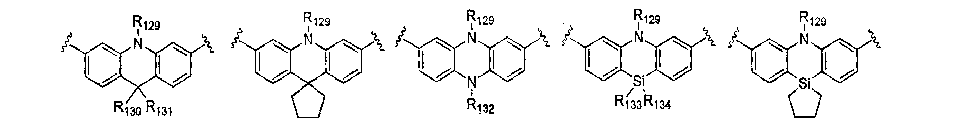 wherein, R₁₂₁ through R₁₃₄ independently represent hydrogen, deuterium, (C1-C60)alkyl or (C6-C60)aryl; the alkyl or aryl of R₁₂₁ through R₁₃₄ may be further substituted by one or more substituent(s) selected from deuterium, halogen, (C1-C60)alkyl, halo(C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl.

4. The organic electroluminescent compound according to claim 1,
wherein 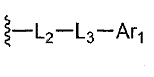 is selected from the following structure: 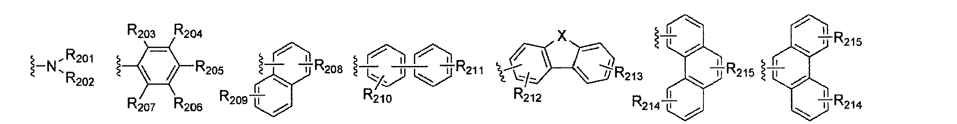 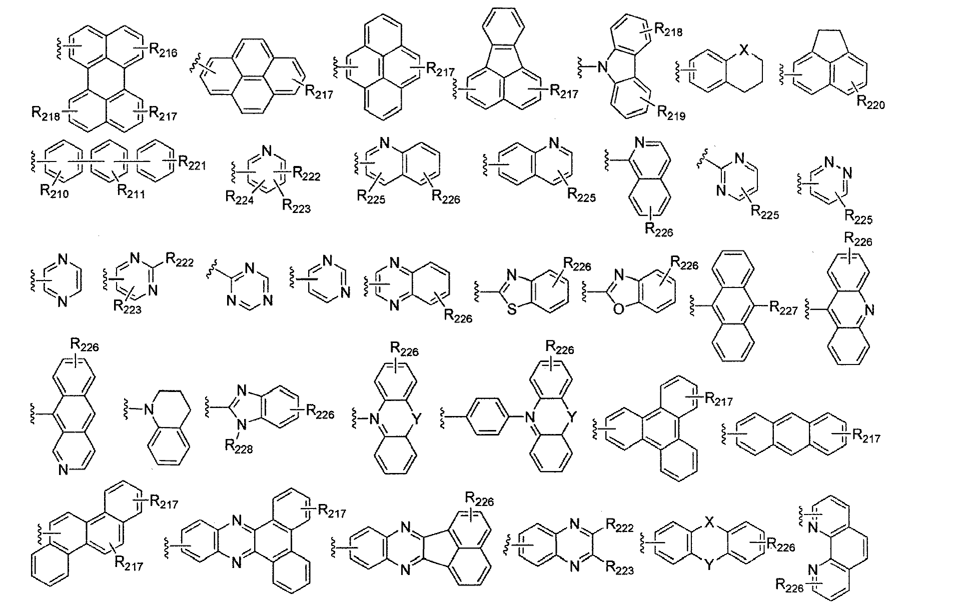 wherein, X and Y independently represent -(CR₇₁R₇₂)m,-, -N(R₇₃)-, -S-, -O-, -Si(R₇₄)(R₇₅)-, -P(R₇₆)- or -(R₈₄)C=C(R₈₅)-;
R₇₁ through R₇₆, R₈₄, R₈₅ and m are defined as in claim 1;
R₂₀₁ and R₂₀₂ independently represent hydrogen, deuterium, (C1-C60)alkyl, (C3-C60)cycloalkyl, (C6-C60)aryl or (C3-C60)heteroaryl containing one or more heteroatom(s) selected from N, O and S;
R₂₀₃ through R₂₂₈ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl;
the alkyl, aryl, heteroaryl, heterocycloalkyl, cycloalkyl, trialkylsilyl, dialkylarylsilyl, triarylsilyl, alkenyl, alkynyl, alkylamino or arylamino of R₂₀₁, R₂₀₂ and R₂₀₃ through R₂₂₈ may be further substituted by one or more substituent(s) selected from deuterium, halogen, (C1-C60)alkyl, hala(C1-C60)alkyl, (C6-C60)aryl, (C3-C60)heteroaryl containing one or more heteroatom(s) selected from N, O and S, with or without (C6-C60)aryl substituent(s), morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl.

5. The organic electroluminescent compound according to claim 1,
wherein 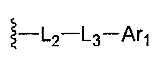 is selected from the following structure:

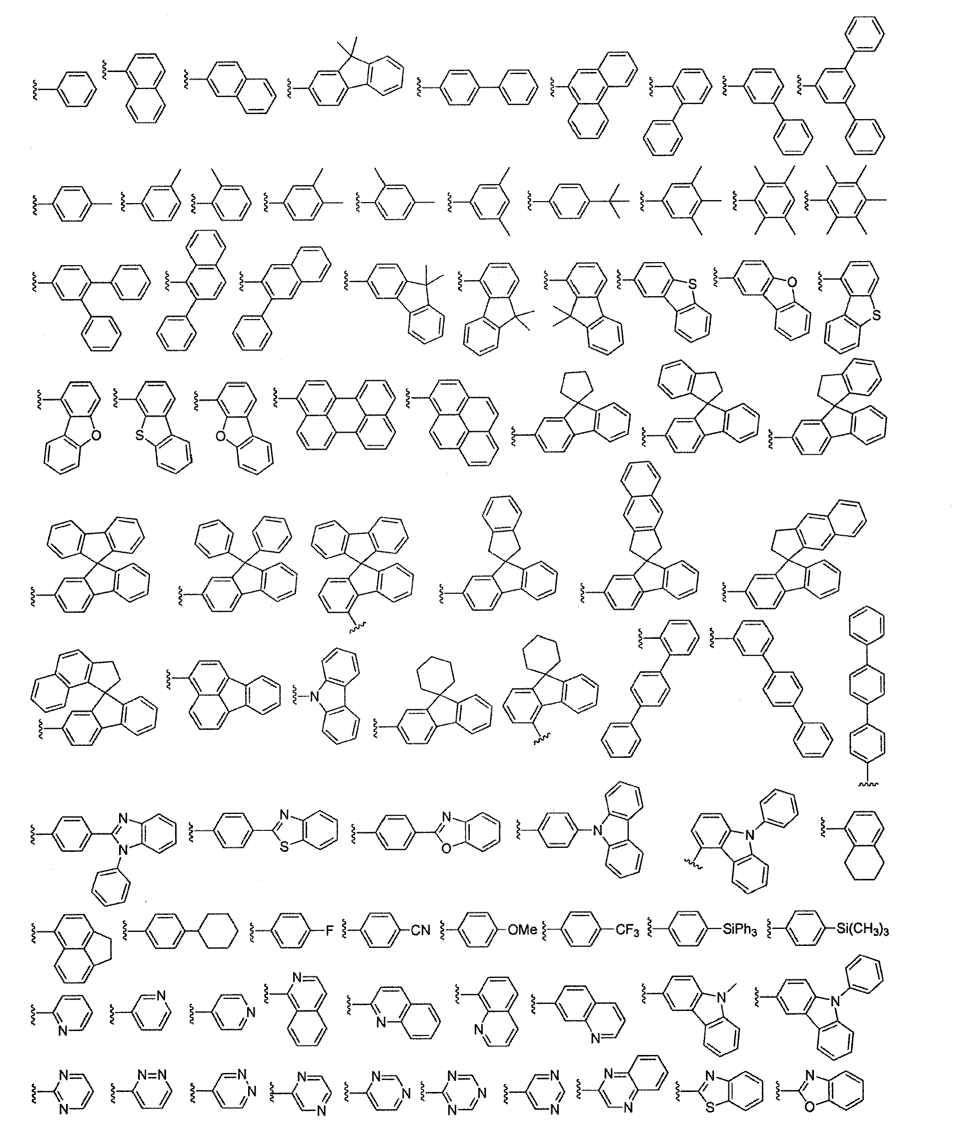

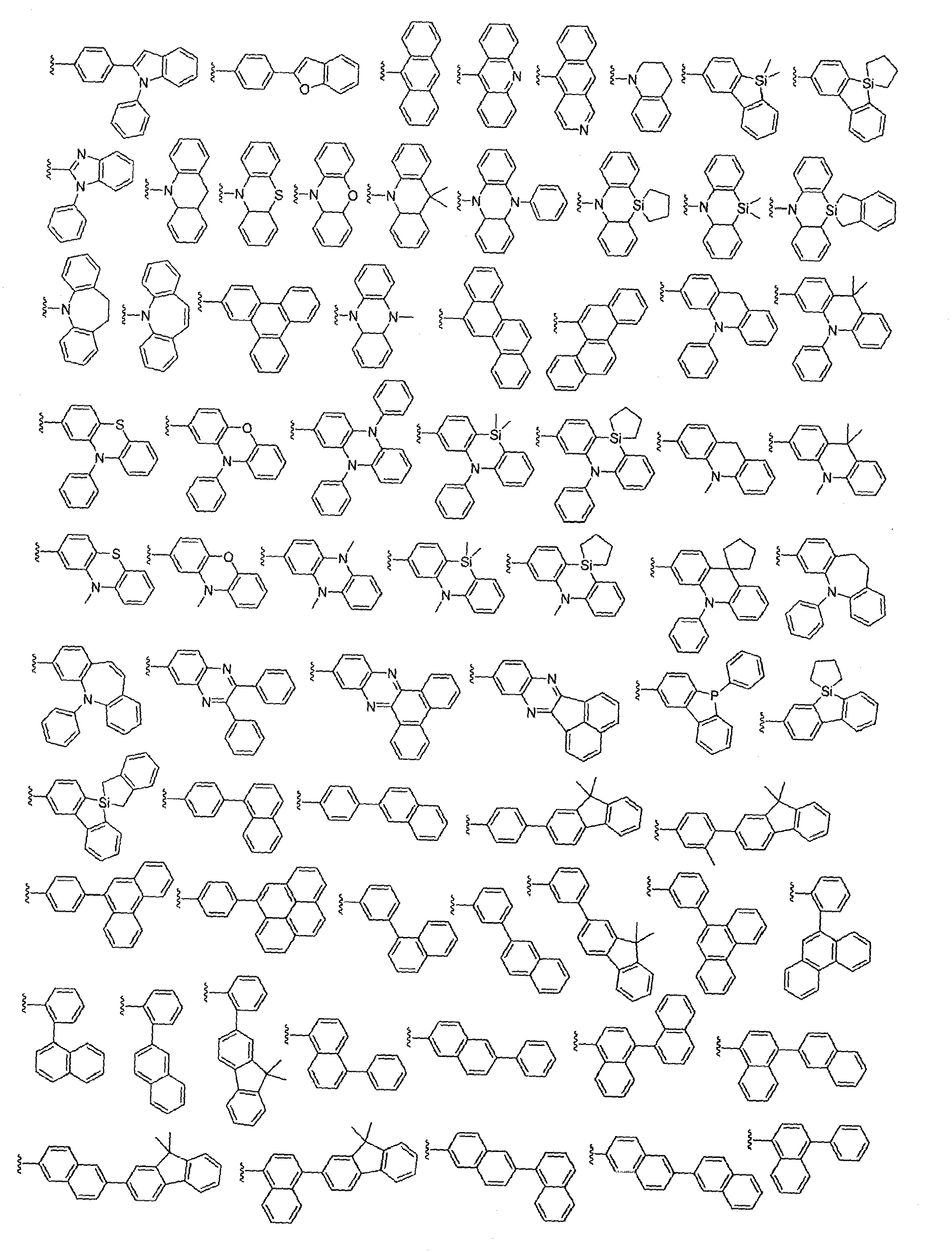

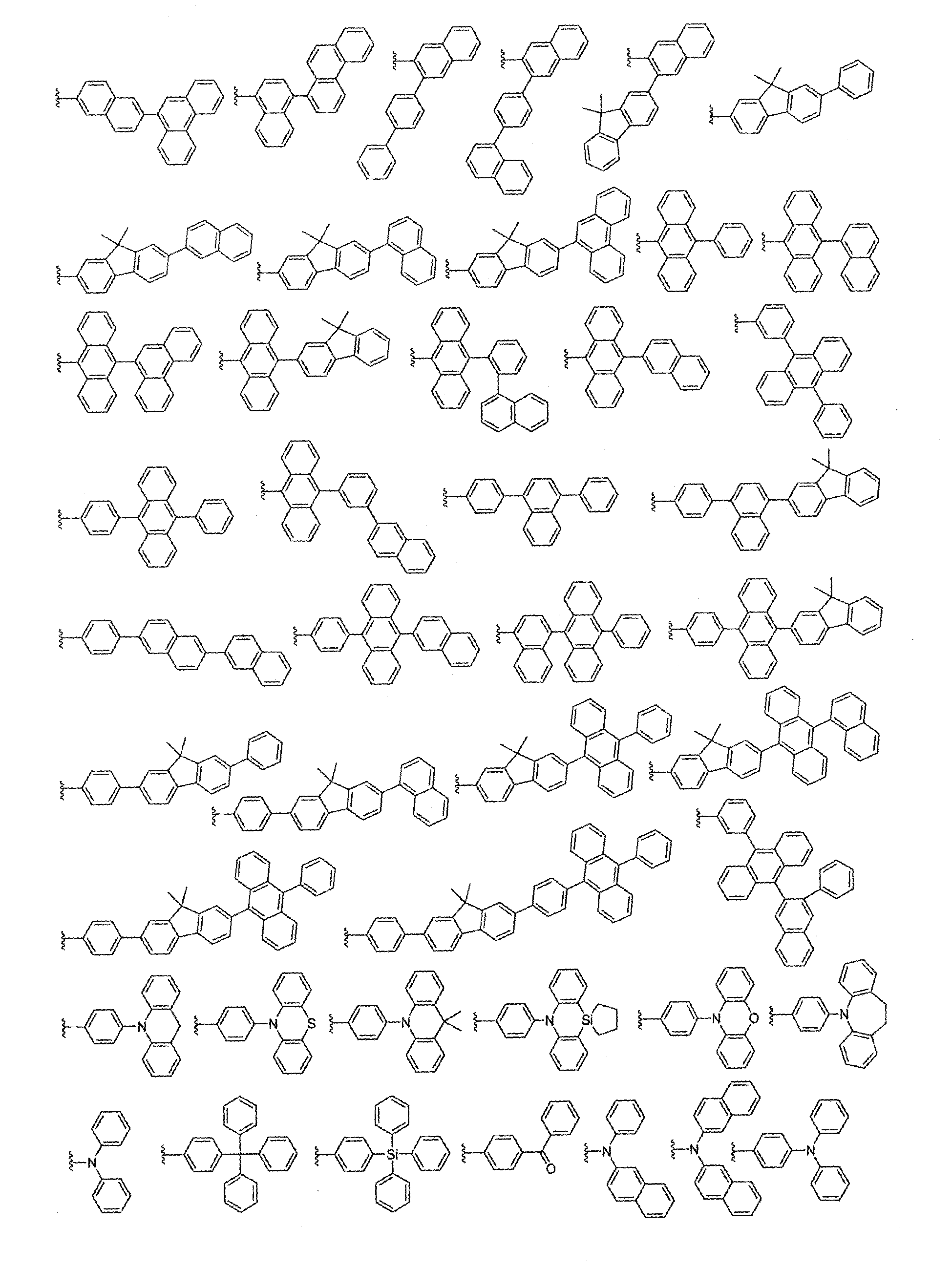

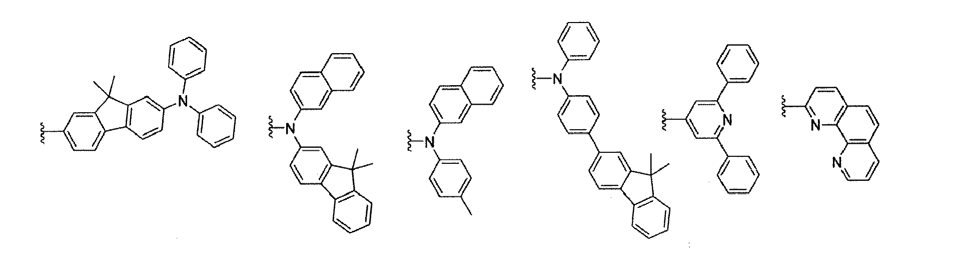

6. The organic electroluminescent compound according to claim 1,
wherein R₁ though R₁₁ independently represent hydrogen, deuterium, fluoro, chloro, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, benzyl, trifluoromethyl, perfluoroethyl, trifluoroethyl, perfluoropropyl, perfluorobutyl, methoxy, ethoxy, n-propoxy, i-propoxy, n-butoxy, i-butoxy, t-butoxy, n-pentoxy, i-pentoxy, n-hexyloxy, n-heptyloxy, cyclopropyl, cyclobutyl, cyclopentyl, cyclohexyl, cycloheptyl, cyclooctyl, cyclononyl, cyclodecyl, morpholino, thiomorpholino, morpholinyl, thiomorpholinyl, trimethylsilyl, triethylsilyl, tripropylsilyl, tri(t-butyl)silyl, t-butyldimethylsilyl, dimethylphenylsilyl, triphenylsilyl, bicycle[2.2.1]heptyl, bicycle[2.2.2]octyl, bicycle[5.2.0]nonyl, bicycle[4.2.2]decyl4-pentylbicycle[2.2.2]octyl, ethenyl, phenylethenyl, ethynyl, phenylethynyl, cyano, methylthio, phenyloxy, phenylthio, methoxycarbonyl, ethoxycarbonyl, t-butoxycarbonyl, methylcarbonyl, ethylcarbonyl, benzylcarbonyl, phenylcarbonyl, carboxyl, nitro or hydroxyl.

7. An organic light-emitting diode comprising an organic electroluminescent compound represented by Chemical Formula (1): 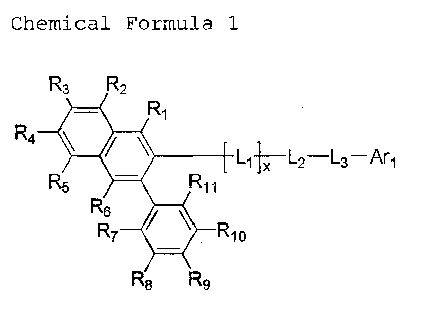 wherein, L₁ represents (C6-C60)arylene or (C3-C60)heteroarylene containing one or more heteroatom(s) selected from N, O and S, or a bivalent group selected from the following structures: 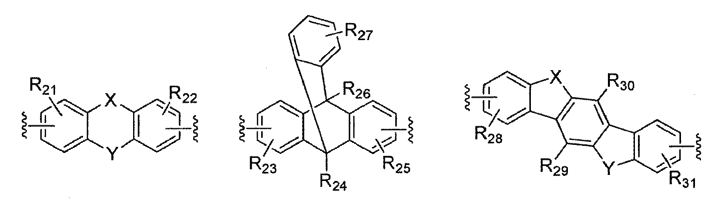 L₂ and L₃ independently represent a chemical bond, or (C1-C60)alkyleneoxy, (C1-C60)alkylenethio, (C6-C60)aryleneoxy, (C6-C60)arylenethio, (C6-C60)arylene or (C3-C60)heteroarylene containing one or more heteroatom(s) selected from N, O and S;
Ar₁ represents NR₄₁R₄₂, (C6-C60)aryl, (C3-C60) heteroaryl containing one or more heteroatom(s) selected from N, O and S, a 5-or 6-membered heterocycloalkyl containing one or more heteroatom(s) selected from N, O and S, (C3-C60)cycloalkyl, adamantyl, (C7-C60)bicycloalkyl, or a substituent selected from the following structures: 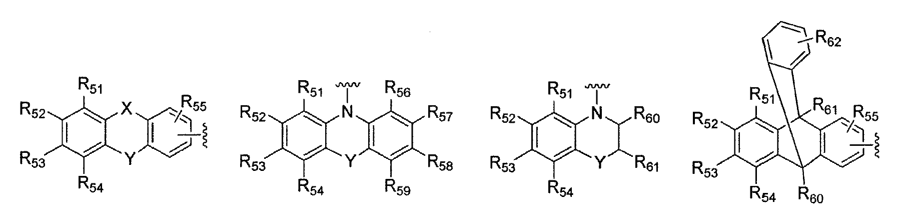 R₁ through R₁₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁ through R₁₁ may be linked to an adjacent substituent via (C3-C60)alkylene or (C3-C60)alkenylene with or without a fused ring to form an alicyclic ring, or a monocyclic or polycyclic aromatic ring;
R₂₁ through R₃₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₂₁ through R₃₁ may be linked to an adjacent substituent via (C3-C60)alkylene or (C3-C60)alkenylene with or without a fused ring to form an alicyclic ring, or a monocyclic or polycyclic aromatic ring;
R₄₁ and R₄₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₄₁ and R₄₂ may be linked via (C3-C60)alkylene or (C3-C60)alkenylene with or without a fused ring to form an alicyclic ring, or a monocyclic or polycyclic aromatic ring;
R₅₁ through R₆₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₅₁ through R₆₂ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or -(CR₇₁R₇₂)m-, -N(R₇₃)-, -S-, -O-, -Si(R₇₄) (R₇₅)-, -P(R₇₆)-, -C(=O)-, -B(R₇₇)-, -In(R₇₈)-, -Se-, -Ge(R₇₉) (R₈₀)-, -Sn(R₈₁) (R₈₂)-, -Ga(R₈₃) - or -(R₈₄)C=C(R₈₅)-;
R₇₁ through R₈₅ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₇₁ and R₇₂, R₇₄ and R₇₅, R₇₉ and R₈₀, R₈₁ and R₈₂, or R₈₄ and R₈₅ may be linked via (C3-C60)alkylene or (C3-C60)alkenylene with or without a fused ring to form an alicyclic ring, or a monocyclic or polycyclic aromatic ring; the arylene or heteroarylene of L₁ through L₃, the aryl or heteroaryl of Ar₁, or the alkyl, aryl, heteroaryl, heterocycloalkyl, cycloalkyl, trialkylsilyl, dialkylarylsilyl, triarylsilyl, alkenyl, alkynyl, alkylamino or arylamino of R₁ through R₁₁, R₂₁ through R₃₁, R₄₁, R₄₂, R₅₁ through R₆₂, and R₇₁ through R₈₅ may be further substituted by one or more substituent(s) selected from deuterium, halogen, (C1-C60)alkyl, halo(C1-C60)alkyl, (C6-C60)aryl, (C3-C60)heteroaryl containing one or more heteroatom(s) selected from N, O and S, with or without (C6-C60)aryl substituent(s), morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 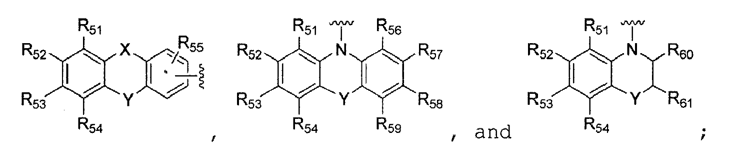 m is an integer from 1 to 4; and
x is an integer from 1 to 4,
wherein the organic light-emitting diode comprises a first electrode; a second electrode; and at least one organic layer(s) interposed between the first electrode and the second electrode;
wherein the organic layer comprises one or more organic electroluminescent compound(s), and one or more dopant(s) selected from the compounds represented by one of Chemical Formulas (2) to (4): 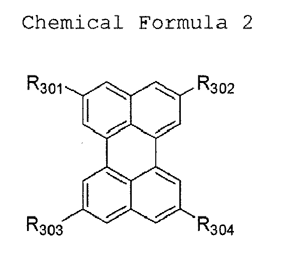 wherein, R₃₀₁ through R₃₀₄ independently represent hydrogen, deuterium, halogen, (C1-C60)alkyl, (C6-C60)aryl, (C4-C60)heteroaryl containing one or more heteroatom(s) selected from N, O and S, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C1-C60)alkylthio, (C6-C60)arylthio, (C1-C60)alkoxycarbonyl, (C1-C60)alkylcarbonyl, (C6-C60)arylcarbonyl, carboxyl, nitro or hydroxyl, or R₃₀₁ through R₃₀₄ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkyloxy, aryloxy, arylthio, alkylamino, arylamino of R₃₀₁ through R₃₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C1-C60)alkylthio, (C6-C60)arylthio, (C1-C60)alkoxycarbonyl, (C1-C60)alkylcarbonyl, (C6-C60)arylcarbonyl, carboxyl, nitro and hydroxyl; 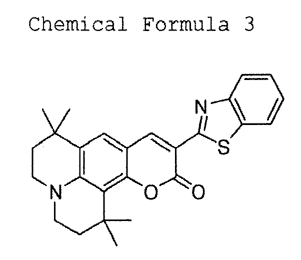 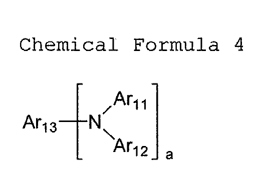 wherein, Ar₁₁ and Ar₁₂ independently represent (C1-C60)alkyl, (C6-C60)aryl, (C4-C60)heteroaryl containing one or more heteroatom(s) selected from N, O and S, (C6-C60)arylamino, (C1-C60)alkylamino, a 5- or 6-membered heterocycloalkyl containing one or more heteroatom(s) selected from N, O and S, or (C3-C60)cycloalkyl, or Ar₁₁ and Ar₁₂ may be linked via (C3-C60)alkylene or (C3-C60)alkenylene with or without a fused ring to form an alicyclic ring or a monocyclic or polycyclic aromatic ring;
when a is 1, Ar₁₃ represents (C6-C60)aryl, (C4-C60)heteroaryl, or a substituent selected from the following structures: 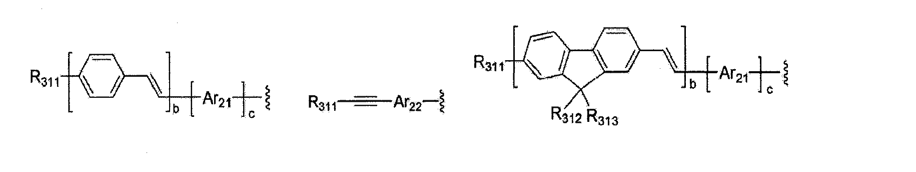 when a is 2, Ar₁₃ represents (C6-C60)arylene, (C4-C60)heteroarylene containing one or more heteroatom(s) selected from N, O and S, or a substituent selected from the following structures: 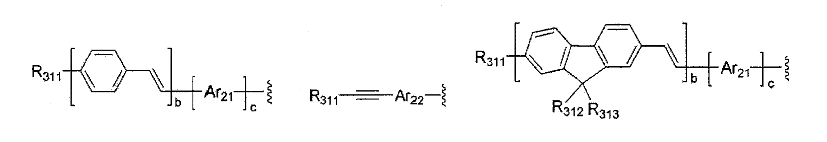 wherein, Ar₂₁ and Ar₂₂ independently represent (C6-C60)arylene or (C4-C60)heteroarylene containing one or more heteroatom(s) selected from N, O and S;
R₃₁₁ through R₃₁₅ independently represent hydrogen, deuterium, (C1-C60)alkyl or (C6-C60)aryl;
b is an integer from 1 to 4, c is an integer of 0 or 1, d is an integer of 0 or 1; and the alkyl, aryl, heteroaryl, arylamino, alkylamino, cycloalkyl or heterocycloalkyl of Ar₁₁ and Ar₁₂, the aryl, heteroaryl, arylene or heteroarylene of Ar₁₃, the arylene or heteroarylene of Ar₂₁ and Ar₂₂, or the alkyl or aryl of R₃₁₁ through R₃₁₅ may be further substituted by one or more substituent(s) selected from deuterium, halogen, (C1-C60)alkyl, (C6-C60)aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C1-C60)arylthio, (C6-C60)alkylthio, (C1-C60)alkoxycarbonyl, (C1-C60)alkylcarbonyl, (C6-C60)arylcarbonyl, carboxyl, nitro and hydroxyl.

8. The organic light-emitting diode according to claim 7, wherein the organic layer comprised one or more compound(s) selected from a group consisting of arylamine compounds and styrylarylamine compounds.

9. The organic light-emitting diode according to claim 7, wherein the organic layer comprised one or more metal(s) selected from a group consisting of organic metals of Group 1, Group 2, 4^{th} period and 5^{th} period transition metals, lanthanide metals and d-transition elements.

10. The organic light-emitting diode according to claim 7, which is an organic display comprising the organic electroluminescent compound(s) and a compound having an electroluminescent peak of the wavelength of not less than 560 nm, at the same time.

11. The organic light-emitting diode according to claim 7, wherein the organic layer comprises both an electroluminescent layer and a charge generating layer.

12. The organic light-emitting diode according to claim 7, wherein a mixed region of reductive dopant and organic substance, or a mixed region of oxidative dopant and organic substance is placed on the inner surface of one or both electrode(s) among the pair of electrodes.

13. An organic solar cell which comprises an organic electroluminescent compound represented by Chemical Formula (1): 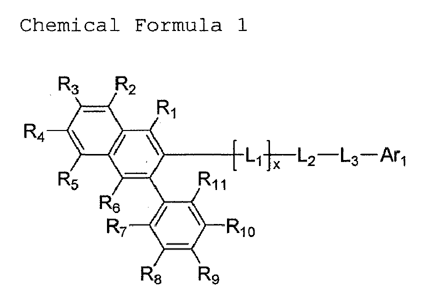 wherein, L₁ represents (C6-C60)arylene or (C3-C60)heteroarylene containing one or more heteroatom(s) selected from N, O and S, or a bivalent group selected from the following structures: 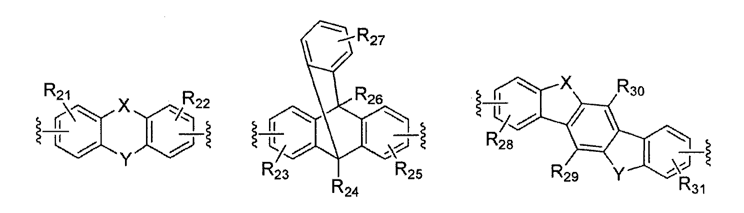 L₂ and L₃ independently represent a chemical bond, or (C1-C60)alkyleneoxy, (C1-C60)alkylenethio, (C6-C60)aryleneoxy, (C6-C60)arylenethio, (C6-C60)arylene or (C3-C60)heteroarylene containing one or more heteroatom(s) selected from N, O and S;
Ar₁ represents NR₄₁R₄₂, (C6-C60)aryl, (C3-C60)heteroaryl containing one or more heteroatom(s) selected from N, O and S, a 5-or 6-membered heterocycloalkyl containing one or more heteroatom(s) selected from N, O and S, (C3-C60)cycloalkyl, adamantyl, (C7-C60)bicycloalkyl, or a substituent selected from the following structures: 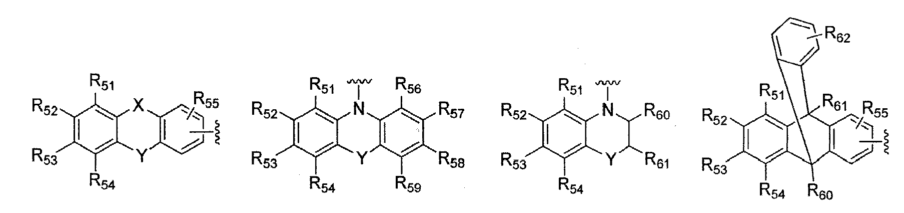 R₁ through R₁₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁ through R₁₁ may be linked to an adjacent substituent via (C3-C60)alkylene or (C3-C60)alkenylene with or without a fused ring to form an alicyclic ring, or a monocyclic or polycyclic aromatic ring;
R₂₁ through R₃₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₂₁ through R₃₁ may be linked to an adjacent substituent via (C3-C60)alkylene or (C3-C60)alkenylene with or without a fused ring to form an alicyclic ring, or a monocyclic or polycyclic aromatic ring;
R₄₁ and R₄₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₄₁ and R₄₂ may be linked via (C3-C60)alkylene or (C3-C60)alkenylene with or without a fused ring to form an alicyclic ring, or a monocyclic or polycyclic aromatic ring;
R₅₁ through R₆₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₅₁ through R₆₂ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or -(CR₇₁R₇₂)m-, -N(R₇₃)-, -S-, -O-, -Si(R₇₄) (R₇₅)-, -P(R₇₆)-, -C(=O)-, -B(R₇₇)-, - In(R₇₈)-, -Se-, -Ge(R₇₉) (R₈₀)-, -Sn(R₈₁) (R₈₂)-, -Ga(R₈₃)- or - (R₈₄)C=C(R₈₅)-;
R₇₁ through R₈₅ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₇₁ and R₇₂, R₇₄ and R₇₅, R₇₉ and R₈₀, R₈₁ and R₈₂, or R₈₄ and R₈₅ may be linked via (C3-C60)alkylene or (C3-C60)alkenylene with or without a fused ring to form an alicyclic ring, or a monocyclic or polycyclic aromatic ring; the arylene or heteroarylene of L₁ through L₃, the aryl or heteroaryl of Ar₁, or the alkyl, aryl, heteroaryl, heterocycloalkyl, cycloalkyl, trialkylsilyl, dialkylarylsilyl, triarylsilyl, alkenyl, alkynyl, alkylamino or arylamino of R₁ through R₁₁, R₂₁ through R₃₁, R₄₁, R₄₂, R₅₁ through R₆₂, and R₇₁ through R₈₅ may be further substituted by one or more substituent(s) selected from deuterium, halogen, (C1-C60)alkyl, halo(C1-C60)alkyl, (C6-C60)aryl, (C3-C60)heteroaryl containing one or more heteroatom(s) selected from N, O and S, with or without (C6-C60)aryl substituent(s), morpholino, thiomorpholino, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 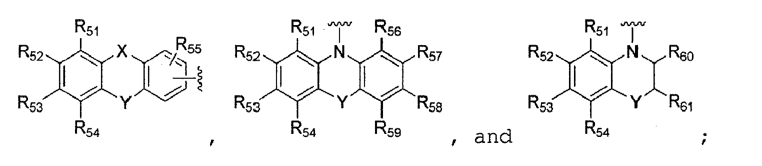 m is an integer from 1 to 4; and
x is an integer from 1 to 4.
